# EUROPEAN PATENT APPLICATION

(11) **EP 4 810 061 A1**
(43) Date of publication of application: **23.09.2026**
(21) Application number: 24890549.9
(22) Date of filing: 06.11.2024
(51) Int. Cl.: A61K 47/68, C07D 491/22, A61P 35/00, C07B 59/00

(54) **DEUTERATED CAMPTOTHECIN COMPOUND, AND PREPARATION AND USE THEREOF**

(30) Priority: 16.11.2023 CN 202311545719; 01.11.2024 CN 202411561506
(71) Applicant: Shanghai Qilu Pharmaceutical Research and Development Centre Ltd., Shanghai 201203 (CN)
(72) Inventor: LV, Wei, Shanghai 201203 (CN); JIN, Jiyu, Shanghai 201203 (CN); JIN, Chen, Shanghai 201203 (CN); YANG, Yang, Shanghai 201203 (CN); HUANG, Ying, Shanghai 201203 (CN); TAO, Weikang, Shanghai 201203 (CN)
(74) Representative: Dai, Simin
(86) International application number: PCT/CN2024/130277
(87) International publication number: WO 2025/103192

(57) **Abstract**

A compound represented by formula I or a pharmaceutically acceptable salt thereof or a stereoisomer thereof, a preparation method therefor, a pharmaceutical composition containing the compound represented by formula I or a pharmaceutically acceptable salt of the composition or a stereoisomer of the composition, and a use thereof in treating tumor-related diseases.

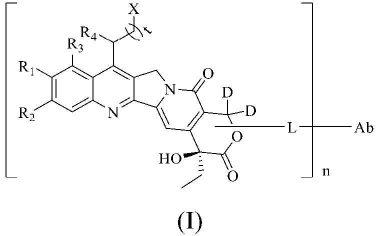

## Description

### CROSS-REFERENCE TO RELATED APPLICATIONS

The present application claims the benefits and priorities of Chinese Patent Application No. 202311545719.9 filed with the China National Intellectual Property Administration on November 16, 2023 and Chinese Patent Application No. 202411561506.X filed with the China National Intellectual Property Administration on November 1, 2024, the entire contents of which are hereby incorporated by reference herein in their entirety.

### TECHNICAL FIELD

The present disclosure belongs to the field of medicinal chemistry, and specifically relates to a class of deuterated camptothecin compounds, pharmaceutically acceptable salts thereof, preparation methods therefor, pharmaceutical compositions containing the deuterated camptothecin compounds or pharmaceutically acceptable salts thereof, and the use of the deuterated camptothecin compounds, pharmaceutically acceptable salts thereof, or the pharmaceutical compositions in the treatment of tumor-related diseases.

### BACKGROUND

Camptothecin (CPT) is a pyrroloquinoline cytotoxic alkaloid and one of the most extensively studied natural antitumor drugs besides paclitaxel. Camptothecin is primarily found in the fruits and root bark of *Camptotheca acuminata,* a plant in the Nyssaceae family that is unique to China. In 1985, Hsiang *et al.* discovered that camptothecin and its derivatives exert their anticancer effects by targeting topoisomerase I (Topo I) to inhibit DNA synthesis. This revelation reignited widespread interest, leading to the emergence of numerous derivatives and establishing them as a new focal point in anticancer research. Subsequently, a new generation of camptothecin-based drugs, such as 10-hydroxycamptothecin (HCPT), irinotecan, topotecan, SN-38, and belotecan, were successively approved for marketing. These drugs are used to treat cancers such as colorectal cancer, small cell lung cancer, and ovarian cancer, with promising advancements in both their indications and formulations.

With the development of drug delivery systems, a series of camptothecin drugs that were previously non-drugable or had significant side effects have regained application. However, the issue of *in vivo* toxicity associated with camptothecin drugs has not been entirely avoided. How to further reduce the toxicity of this class of drugs remains an issue that needs attention at present.

### SUMMARY

In one aspect, the present disclosure provides a compound of Formula I, a pharmaceutically acceptable salt thereof, or a stereoisomer thereof: 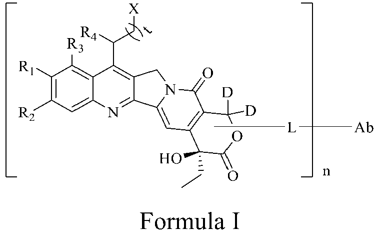
wherein Ab represents an antibody or an antigen-binding fragment; L represents a linker connecting Ab and a payload drug molecule; n is selected from the group consisting of 1-12;
R₁ is H, halogen, OH, SH, NH₂, C₁₋₄ alkyl, C₁₋₄ haloalkyl, or C₁₋₄ alkoxy;
R₂ is H, halogen, C₁₋₄ alkyl, or C₁₋₄ alkoxy;
or R₁ and R₂ are cyclized to form -O-(CH₂)ₘ-O-, wherein m is 1, 2, or 3;
R₃ and R₄ are each independently H, C₁₋₄ alkyl, C₁₋₄ alkoxy, C₁₋₄ haloalkyl, or C₁₋₄ haloalkoxy;
or R₃ and R₄ are cyclized to form -(CH₂)ₖ-, wherein k is 1, 2, 3, or 4;
X is H, OH, HO-CH(R₅)-(CH₂)ₚ-CO-NH-, or -N(R₆)(R₇), wherein p is 0, 1, or 2;
R₅ is H, C₁₋₄ alkyl, C₁₋₄ haloalkyl, 3- to 6-membered cycloalkyl, or 3- to 6-membered heterocycloalkyl;
R₆ is H, C₁₋₄ alkyl, or C₁₋₄ haloalkyl;
R₇ is H or R₈-S(O)₂-;
R₈ is C₁₋₄ alkyl; and
t is 0, 1, 2, 3, 4, or 5.

In the present disclosure, the expression "R₁ and R₂ are cyclized to form -O-(CH₂)ₘ-O-" means that R₁ and R₂ are connected to form -O-(CH₂)ₘ-O-. The expression "R₃ and R₄ are cyclized to form -(CH₂)ₖ-" means that R₃ and R₄ are connected to form -(CH₂)ₖ-.

In any one embodiment of the compound of Formula I of the present disclosure, R₁ is C₁₋₄ alkyl, and R₂ is halogen.

In any one embodiment of the compound of Formula I of the present disclosure, R₁ is methyl, and R₂ is F.

In any one embodiment of the compound of Formula I of the present disclosure, R₁ is H, and R₂ is H.

In any one embodiment of the compound of Formula I of the present disclosure, R₁ and R₂ are cyclized to form -O-CH₂-O-.

In any one embodiment of the compound of Formula I of the present disclosure, R₁ is NH₂, and R₂ is H or halogen.

In any one embodiment of the compound of Formula I of the present disclosure, R₁ is NH₂, and R₂ is H or F.

In any one embodiment of the compound of Formula I of the present disclosure, R₃ is H, and R₄ is H.

In any one embodiment of the compound of Formula I of the present disclosure, R₃ is H, and R₄ is C₁₋₄ alkyl.

In any one embodiment of the compound of Formula I of the present disclosure, R₃ is H, and R₄ is methyl.

In any one embodiment of the compound of Formula I of the present disclosure, R₃ and R₄ are cyclized to form -CH₂-CH₂-.

In any one embodiment of the compound of Formula I of the present disclosure, X is HO-CH(R₅)-(CH₂)ₚ-CO-NH-, wherein p is 0, 1, or 2.

In any one embodiment of the compound of Formula I of the present disclosure, X is OH or NH₂.

In any one embodiment of the compound of Formula I of the present disclosure, X is H, and t is 0.

In any one embodiment of the compound of Formula I of the present disclosure, X is - N(R₆)(R₇).

In any one embodiment of the compound of Formula I of the present disclosure, n is 4-9, 6-8, 7-8, 7.4-8.0, or 7.5-8.5; or n is 6, 7, or 8. In any one embodiment of the compound of Formula I of the present disclosure, n is 7-8, 7.3-7.9, 7.4-7.8, 7.5-7.7, 7.5-7.6, 7.5-7.8, or 7.4-7.7.

In one embodiment of the compound of Formula I of the present disclosure, the compound of Formula I is a compound of Formula Ia, a pharmaceutically acceptable salt thereof, or a stereoisomer thereof; 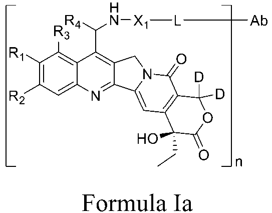 wherein R₁, R₂, R₃, and R₄ are as defined in any one embodiment of the compound of Formula I; X₁ is a chemical bond or -O-CH(R₅)-(CH₂)ₚ-CO-, wherein the -CO- end is connected to -NH-; p is 0, 1, or 2; R₅ is H, C₁₋₄ alkyl, 3- to 6-membered cycloalkyl, or 3- to 6-membered heterocycloalkyl.

In any one embodiment of the compound of Formula Ia of the present disclosure, R₁ is methyl or methoxy; or R₁ is methyl.

In any one embodiment of the compound of Formula Ia of the present disclosure, R₂ is F or Cl; or R₂ is F.

In any one embodiment of the compound of Formula Ia of the present disclosure, R₁ is methyl, and R₂ is F.

In any one embodiment of the compound of Formula Ia of the present disclosure, R₃ and R₄ are cyclized to form -(CH₂)ₖ-, wherein k is 2.

In any one embodiment of the compound of Formula Ia of the present disclosure, R₃ is H; and R₄ is H.

In any one embodiment of the compound of Formula Ia of the present disclosure, X₁ is -O-CH(R₅)-(CH₂)ₚ-CO-, wherein the -CO- end is connected to -NH-; wherein p is 0 or 1, and R₅ is H, C₁₋₄ alkyl, or 3- to 6-membered cycloalkyl.

In any one embodiment of the compound of Formula Ia of the present disclosure, X₁ is -O-CH(R₅)-(CH₂)ₚ-CO-, wherein the -CO- end is connected to -NH-; wherein p is 0 or 1, and R₅ is H, methyl, or cyclopropyl.

In any one embodiment of the compound of Formula Ia of the present disclosure, X₁ is -O-CH(R₅)-CO-, wherein the -CO- end is connected to -NH-; and R₅ is H or 3- to 6-membered cycloalkyl.

In any one embodiment of the compound of Formula Ia of the present disclosure, X₁ is -O-CH(R₅)-CO-, wherein the -CO- end is connected to -NH-; and R₅ is H or cyclopropyl.

In any one embodiment of the compound of Formula Ia of the present disclosure, X₁ is -O-CH(R₅)-CH₂-CO-, wherein the -CO- end is connected to -NH-; and R₅ is C₁₋₄ alkyl.

In any one embodiment of the compound of Formula Ia of the present disclosure, X₁ is -O-CH(R₅)-CH₂-CO-, wherein the -CO- end is connected to -NH-; and R₅ is methyl.

In any one embodiment of the compound of Formula Ia of the present disclosure, X₁ is the following group: 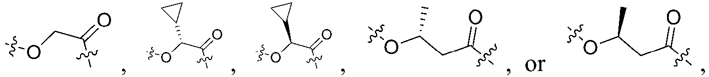 wherein the -CO- end is connected to -NH.

In some embodiments of the compound of Formula Ia of the present disclosure, the compound of Formula Ia is the following compound of Formula Ia-1 or Formula Ia-2, a pharmaceutically acceptable salt thereof, or a stereoisomer thereof; 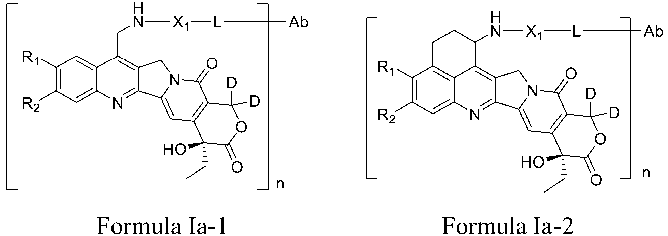 wherein Ab, L, R₁, R₂, X₁, and n are as defined in any one embodiment of the compound of Formula Ia.

In any one embodiment of the compound of Formula Ia of the present disclosure, 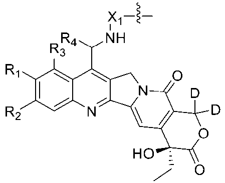 is the following compound moiety: 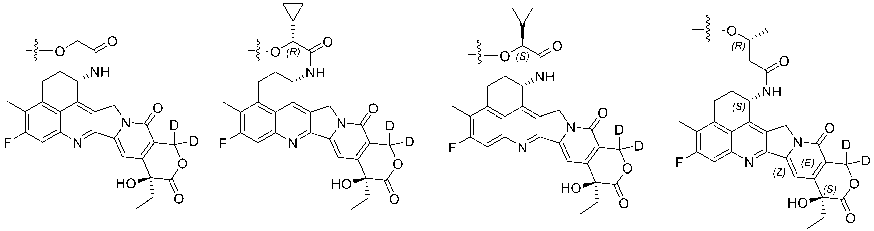 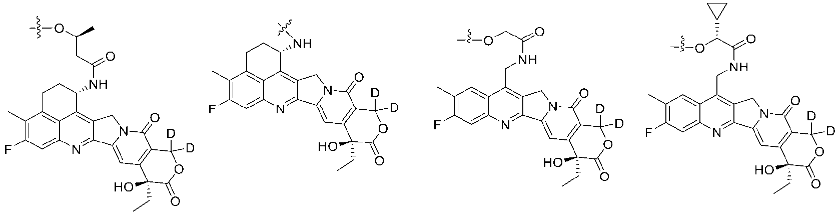 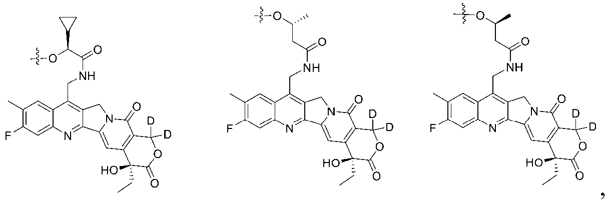 wherein " 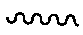 " indicates that this position is connected to the linker L via a chemical bond.

In one embodiment of the compound of Formula I of the present disclosure, the compound of Formula I is a compound of Formula Ib, a pharmaceutically acceptable salt thereof, or a stereoisomer thereof; 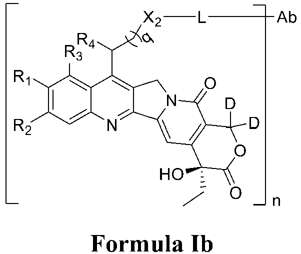
wherein Ab, L, and n are as defined in any one embodiment of the compound of Formula I;
R₁ is H, OH, halogen, NH₂, C₁₋₄ alkyl, C₁₋₄ haloalkyl, or C₁₋₄ alkoxy;
R₂ is H, halogen, C₁₋₄ alkyl, or C₁₋₄ alkoxy;
or R₁ and R₂ are cyclized to form -O-(CH₂)ₘ-O-, wherein m is 1, 2, or 3;
R₃ and R₄ are each independently H, C₁₋₄ alkyl, or C₁₋₄ alkoxy;
or R₃ and R₄ are cyclized to form -(CH₂)ₖ-, wherein k is 1, 2, or 3;
X₂ is O or -N(R₆)-;
R₆ is H or C₁₋₄ alkyl;
q is 0, 1, 2, 3, or 4.

In any one embodiment of the compound of Formula Ib of the present disclosure, R₁ is methyl, and R₂ is H, Cl, or F.

In any one embodiment of the compound of Formula Ib of the present disclosure, R₁ and R₂ are cyclized to form -O-(CH₂)ₘ-O-, wherein m is 1 or 2.

In any one embodiment of the compound of Formula Ib of the present disclosure, R₃ and R₄ are both H.

In any one embodiment of the compound of Formula Ib of the present disclosure, R₃ is H, and R₄ is methyl.

In any one embodiment of the compound of Formula Ib of the present disclosure, X₂ is -O- or -NH-.

In any one embodiment of the compound of Formula Ib of the present disclosure, X₂ is -O- or -NH-, and q is 0, 1, 2, 3, or 4.

In any one embodiment of the compound of Formula Ib of the present disclosure, X₂ is -O- or -NH-, q is 1, 2, 3, or 4; R₃ and R₄ are both H; R₁ is methyl, and R₂ is H, Cl, or F, or R₁ and R₂ are cyclized to form -O-(CH₂)ₘ-O-, wherein m is 1 or 2.

In some embodiments of the compound of Formula Ib of the present disclosure, the compound of Formula Ib is a compound of Formula Ib-1.

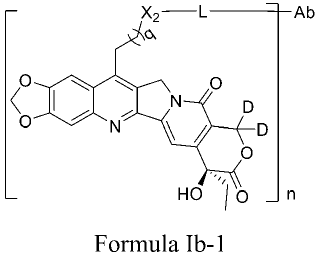

In the compound of Formula Ib-1, Ab, L, n, X₂, and q are as defined in any one embodiment of the compound of Formula Ib.

In any one embodiment of Formula Ib, 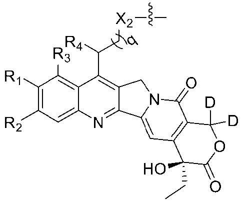 is the following compound moiety; 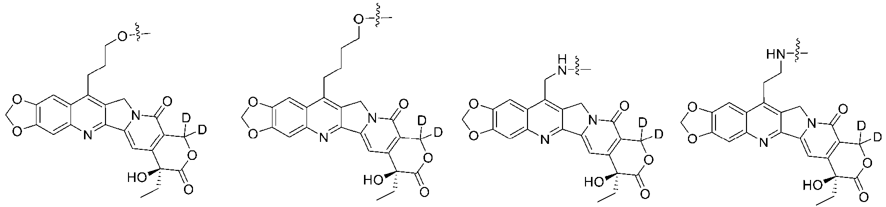 or 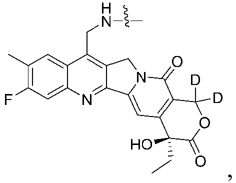 wherein " 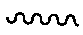 " indicates that this position is connected to the linker L via a chemical bond.

In one embodiment of the compound of Formula I of the present disclosure, the compound of Formula I is a compound of Formula Ic or a pharmaceutically acceptable salt thereof: 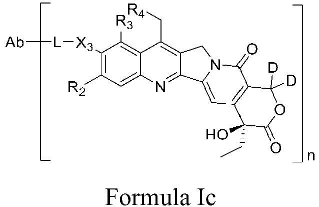 wherein X₃ is NH, O, or S; Ab, L, n, R₂, R₃, and R₄ are as defined in any one embodiment of the compound of Formula I of the present disclosure.

In any one embodiment of the compound of Formula Ic of the present disclosure, R₂ is H or halogen, or R₂ is H or F.

In any one embodiment of the compound of Formula Ic of the present disclosure, R₃ is H, and R₄ is C₁₋₄ alkyl; or R₃ is H, and R₄ is methyl.

In any one embodiment of the compound of Formula Ic of the present disclosure, R₃ and R₄ are cyclized to form -(CH₂)ₖ-, wherein k is 1, 2, or 3.

In any one embodiment of the compound of Formula Ic of the present disclosure, R₃ and R₄ are cyclized to form -(CH₂)₂-.

In any one embodiment of the compound of Formula Ic of the present disclosure, X₃ is NH or O; or X₃ is NH, or X₃ is O;

In any one embodiment of the compound of Formula Ic of the present disclosure, 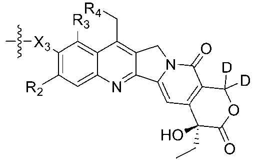 is the following compound moiety:

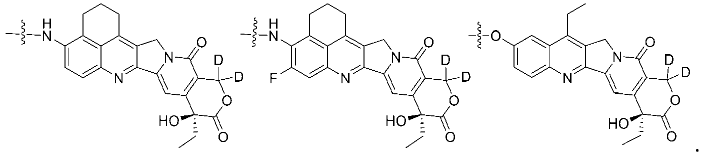

In one embodiment of the compound of Formula I of the present disclosure, the compound of Formula I is a compound of Formula Id or a pharmaceutically acceptable salt thereof: 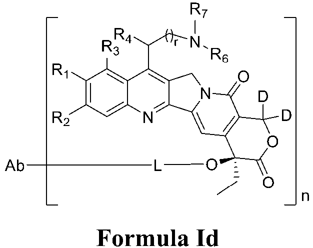 wherein R₁, R₂, R₃, R₄, R₆, and R₇ are as defined in any one embodiment of the compound of Formula I of the present disclosure, and Ab, L, and n are as defined in any one embodiment of the compound of Formula I of the present disclosure; r is 0, 1, 2, or 3.

In any one embodiment of the compound of Formula Id of the present disclosure, R₁ and R₂ are both H.

In any one embodiment of the compound of Formula Id of the present disclosure, R₃ and R₄ are both H.

In any one embodiment of the compound of Formula Id of the present disclosure, r is 1.

In any one embodiment of the compound of Formula Id of the present disclosure, R₆ is methyl, ethyl, or isopropyl; or R₆ is isopropyl.

In any one embodiment of the compound of Formula Id of the present disclosure, R₇ is H.

In any one embodiment of the compound of Formula Id of the present disclosure, R₇ is R₈-S(O)₂-, wherein R₈ is methyl or ethyl; or R₈ is methyl.

In any one embodiment of the compound of Formula Id of the present disclosure, 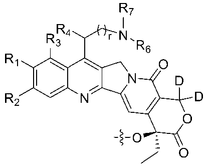 is the following compound moiety:

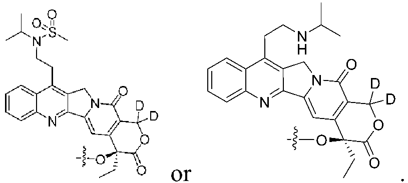

In one embodiment of the compound of Formula I, Formula Ia, Formula Ia-1, Formula Ia-2, Formula Ib, Formula Ib-1, Formula Ic, or Formula Id of the present disclosure, Ab is an anti-Her2 antibody, an anti-Trop2 antibody, or an anti-Claudin18.2 antibody.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having HCDR1, HCDR2, and HCDR3 as set forth in SEQ ID NO: 1, SEQ ID NO: 2, and SEQ ID NO: 3, respectively.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having LCDR1, LCDR2, and LCDR3 as set forth in SEQ ID NO: 6, SEQ ID NO: 7, and SEQ ID NO: 8, respectively.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having HCDR1, HCDR2, and HCDR3 as set forth in SEQ ID NO: 1, SEQ ID NO: 2, and SEQ ID NO: 3, respectively, and LCDR1, LCDR2, and LCDR3 as set forth in SEQ ID NO: 6, SEQ ID NO: 7, and SEQ ID NO: 8, respectively.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having a heavy chain variable region as set forth in SEQ ID NO: 4.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having a light chain variable region as set forth in SEQ ID NO: 9.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having a heavy chain variable region as set forth in SEQ ID NO: 4 and a light chain variable region as set forth in SEQ ID NO: 9.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having a heavy chain as set forth in SEQ ID NO: 5.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having a light chain as set forth in SEQ ID NO: 10.

In any one embodiment of the compound of Formula I, Formula Ia, Formula Ia-1, Formula Ia-2, Formula Ib, Formula Ib-1, Formula Ic, or Formula Id of the present disclosure, the Ab is an anti-Her2 antibody, having a heavy chain as set forth in SEQ ID NO: 5 and a light chain as set forth in SEQ ID NO: 10.

In any one embodiment of the compound of Formula I, Formula Ia, Formula Ia-1, Formula Ia-2, Formula Ib, Formula Ib-1, Formula Ic, or Formula Id of the present disclosure, the Ab is Trastuzumab.

In one embodiment of the compound of Formula I, Formula Ia, Formula Ia-1, Formula Ia-2, Formula Ib, Formula Ib-1, Formula Ic, or Formula Id of the present disclosure, Ab is Pertuzumab, Sacituzumab, or zolbetuximab.

In one embodiment of the compound of Formula I, Formula Ia, Formula Ia-1, Formula Ia-2, Formula Ib, Formula Ib-1, Formula Ic, or Formula Id of the present disclosure, L is L₁-L₂-L₃-L₄, wherein one end of L₁ is connected to Ab, and one end of L₄ is connected to the payload drug molecule D;
L₁ is the following group, and the end of L₁ marked with an asterisk * is connected to Ab: 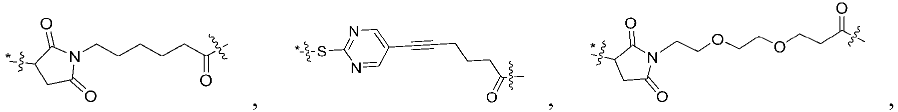 or 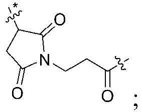
L₂ is a chemical bond, -N(R₁₀)-CH₂-CO-, or 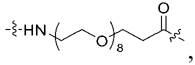 and the CO end of L₂ is connected to L₃, while the other end is connected to L₁; R₁₀ is C₁₋₄ alkyl or C₁₋₄ haloalkyl;
L₃ is a polypeptide residue composed of 2-6 amino acids, and the C-terminus of the polypeptide residue is connected to L₄;
L₄ is a chemical bond, -NH-CH-, or 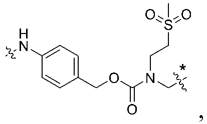 wherein the CH end is connected to the payload drug molecule, and the -NH- end is connected to L₃.

In any one embodiment of the compound of Formula I, Formula Ia, Formula Ia-1, Formula Ia-2, Formula Ib, Formula Ib-1, Formula Ic, or Formula Id of the present disclosure, L is L₁-L₂-L₃-L₄, and L₂ is a chemical bond.

In any one embodiment of the compound of Formula I, Formula Ia, Formula Ia-1, Formula Ia-2, Formula Ib, Formula Ib-1, Formula Ic, or Formula Id of the present disclosure, L is L₁-L₂-L₃-L₄, and L₂ is -N(CH₃)-CH₂-CO-.

In any one embodiment of the compound of Formula I, Formula Ia, Formula Ia-1, Formula Ia-2, Formula Ib, Formula Ib-1, Formula Ic, or Formula Id of the present disclosure, L is L₁-L₂-L₃-L₄, and L₃ is the following peptide residue: GFG, GGFG, GGGFG, GGVA, V-Cit, 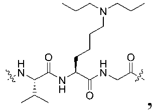 or VA.

In any one embodiment of the compound of Formula I, Formula Ia, Formula Ia-1, Formula Ia-2, Formula Ib, Formula Ib-1, Formula Ic, or Formula Id of the present disclosure, L is L₁-L₂-L₃-L₄, L₁ is 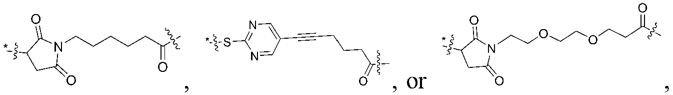 L₂ is a chemical bond or -N(CH₃)-CH₂-CO-, L₃ is GFG, GGFG, or GGGFG, and L₄ is -NH-CH-.

In any one embodiment of the compound of Formula I, Formula Ia, Formula Ia-1, Formula Ia-2, Formula Ib, Formula Ib-1, Formula Ic, or Formula Id of the present disclosure, L is the following moiety: 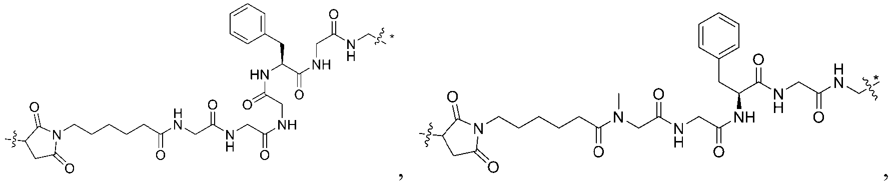 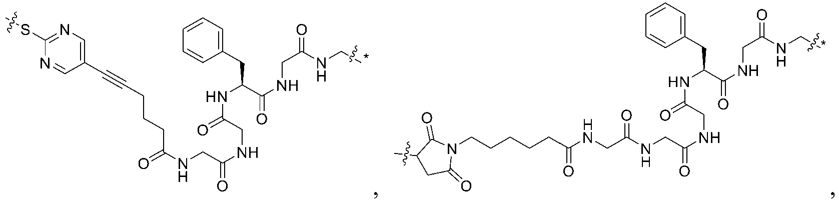 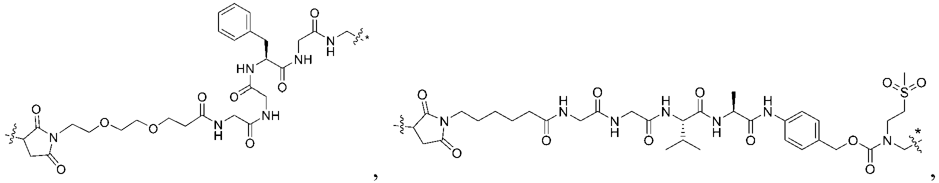 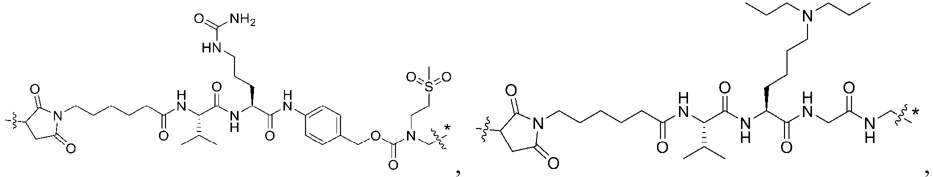 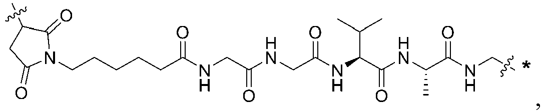 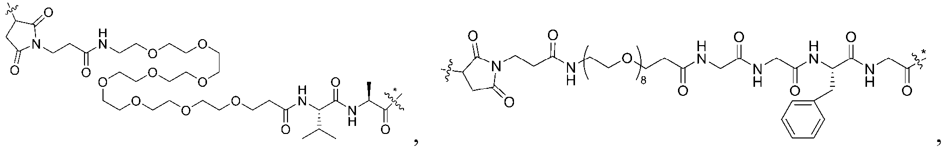 or 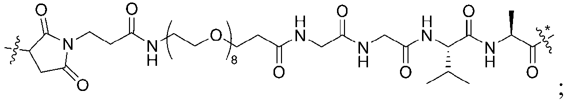 wherein "*" indicates the position where the linker connects to the payload drug molecule, and the other end indicates the position where the linker connects to the Ab. In any one embodiment of the compound of Formula I of the present disclosure, 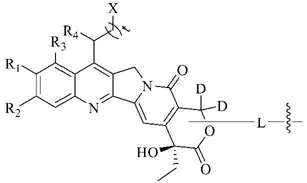 is the following structural moiety: 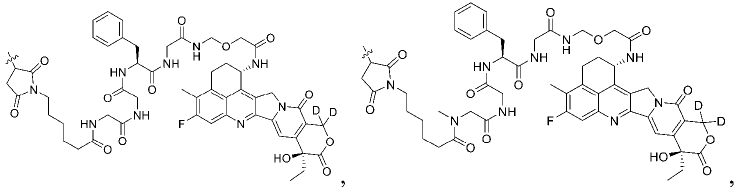 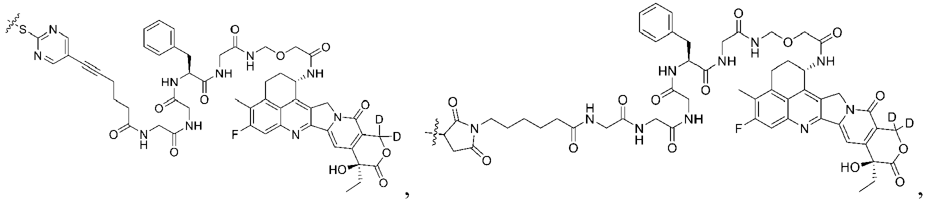 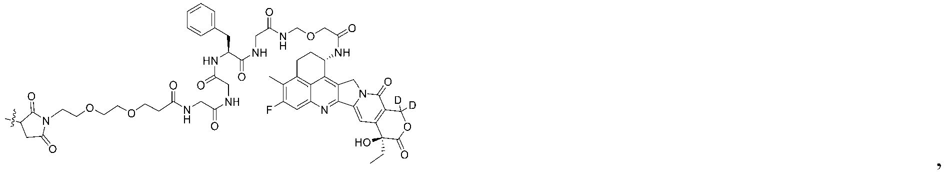 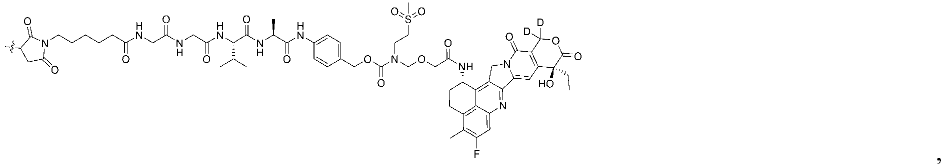 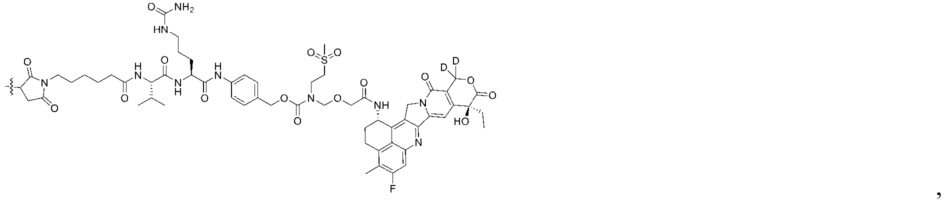 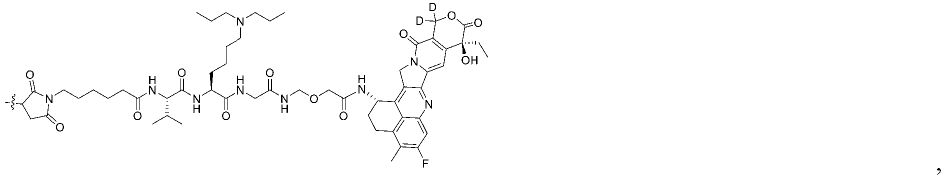 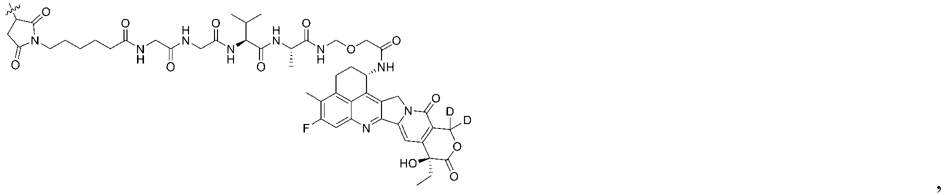 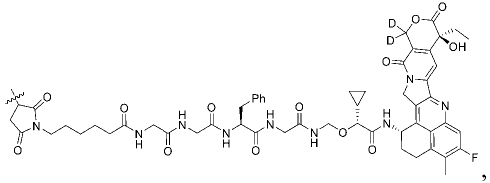 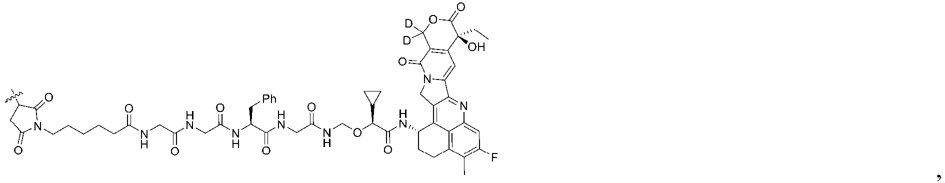 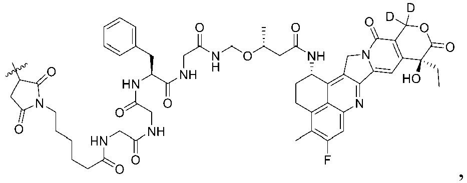 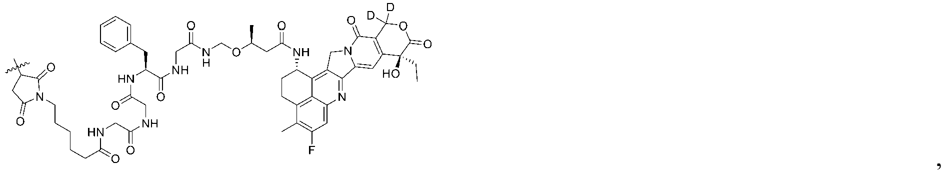 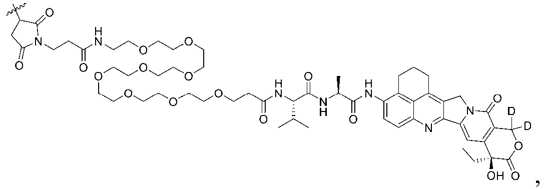 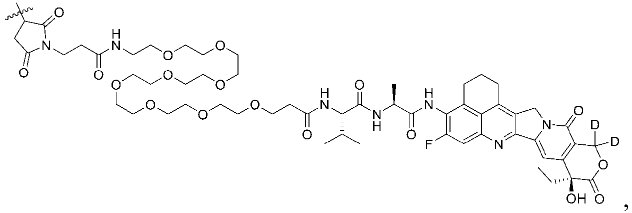 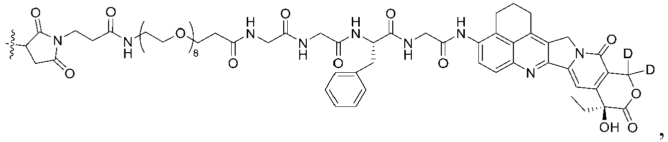 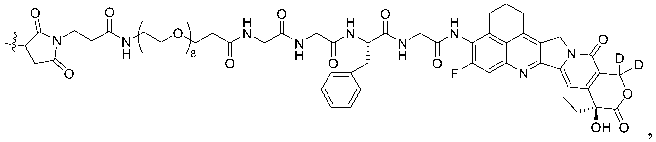 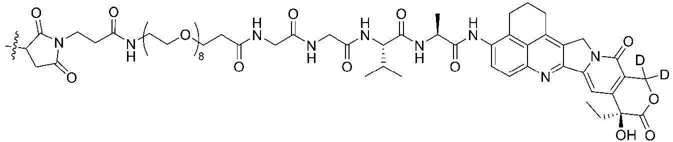 or

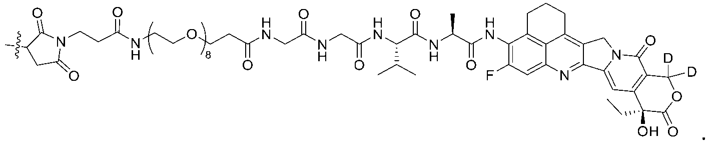

In one embodiment of the compound of Formula I of the present disclosure, the compound of Formula I is the following compound, a pharmaceutically acceptable salt thereof, or a stereoisomer thereof: 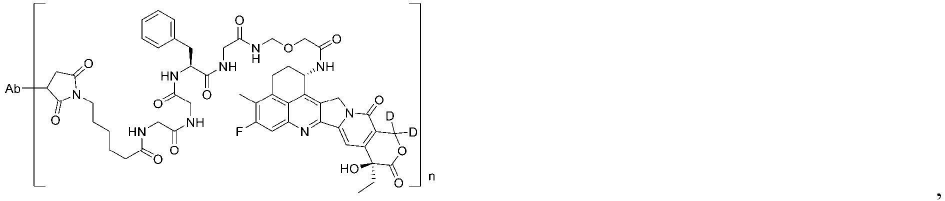 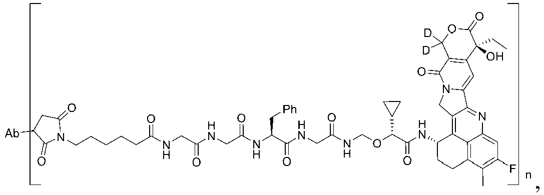 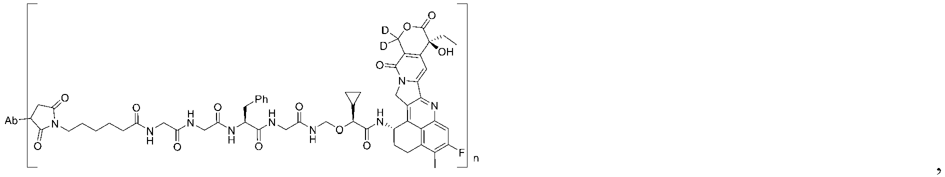 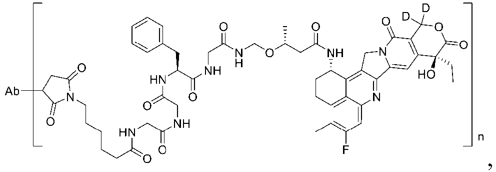 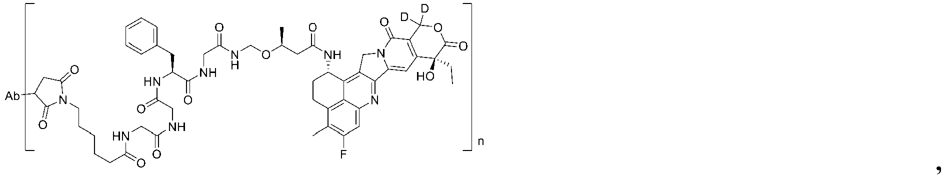 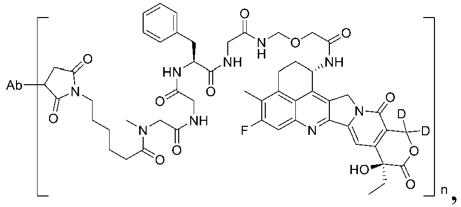 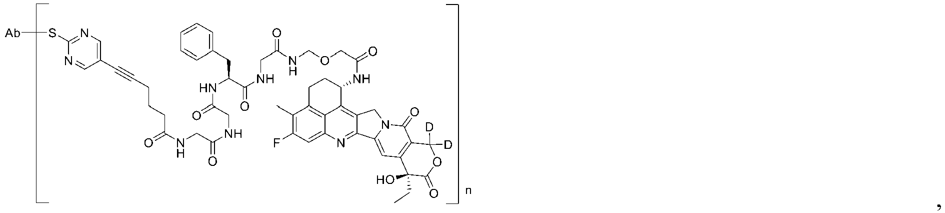 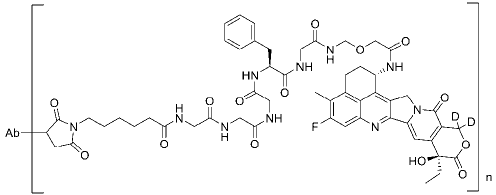 or 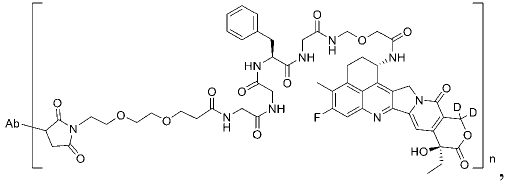 wherein n has the same meaning as in Formula I; for example, n is 7.0-8.0, or n is 7.4-7.8.

In a second aspect, the present disclosure provides the following compound, a pharmaceutically acceptable salt thereof, or a stereoisomer thereof: 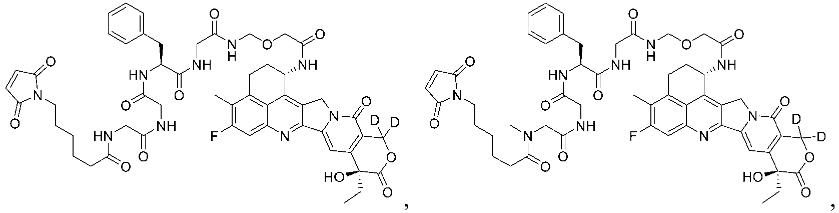 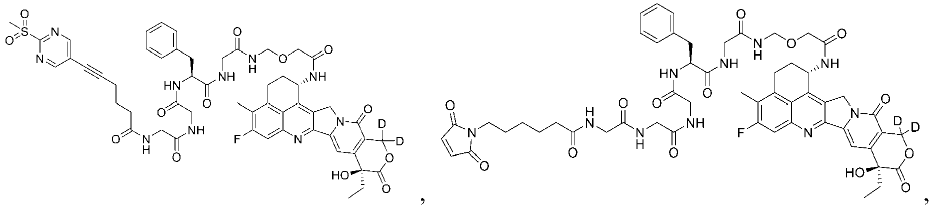 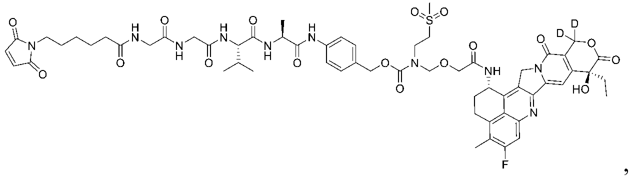 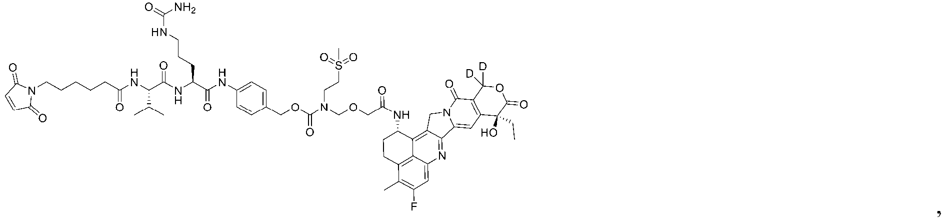 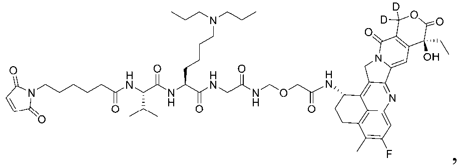 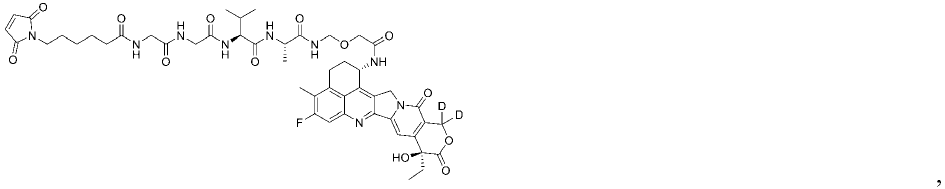 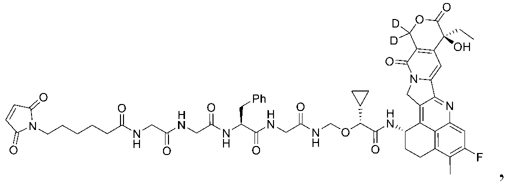 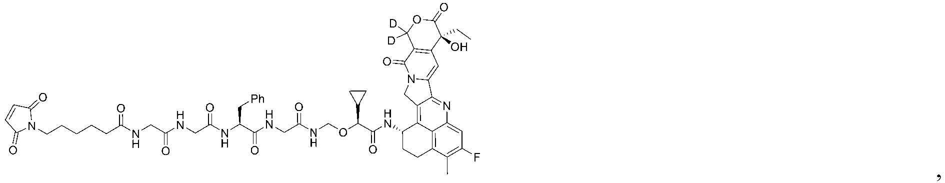 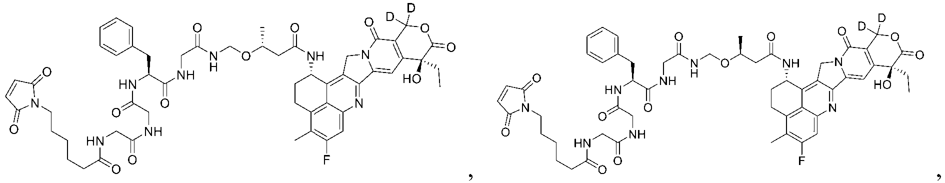 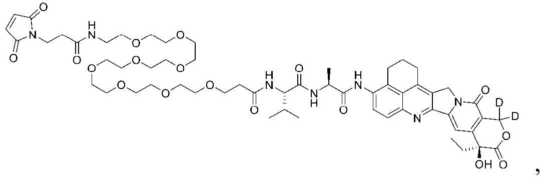 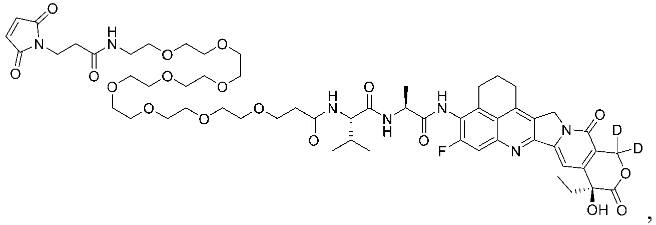 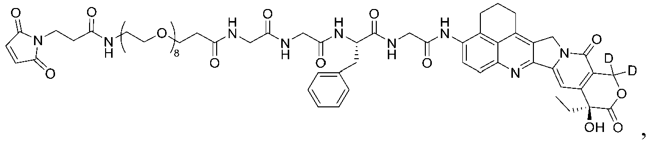 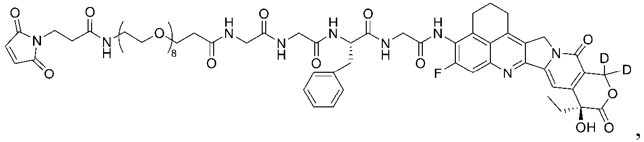 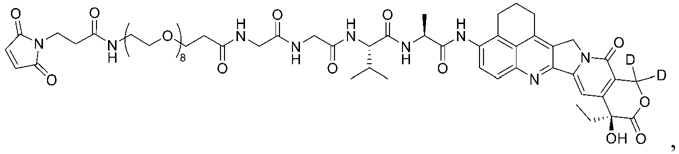 or

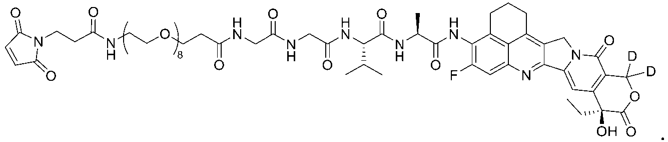

In a third aspect, the present disclosure provides the following structural moiety: 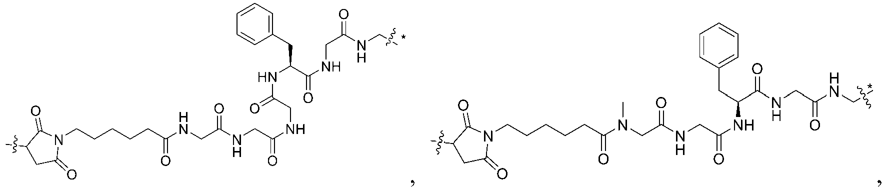 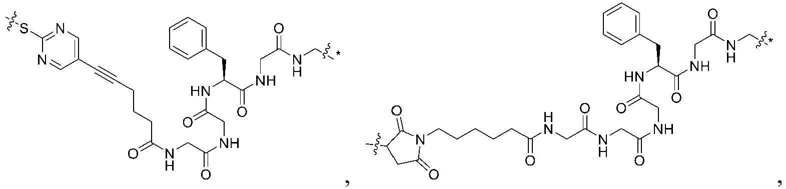 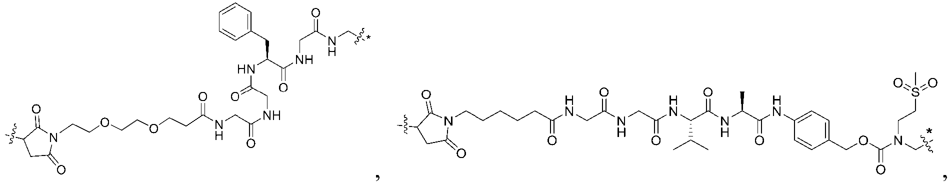 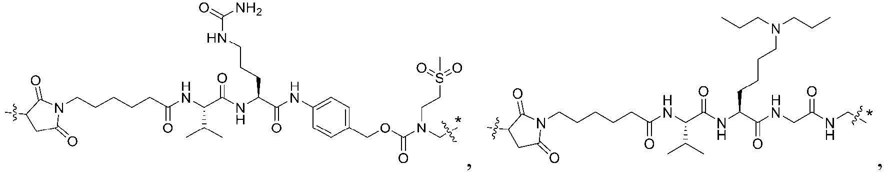 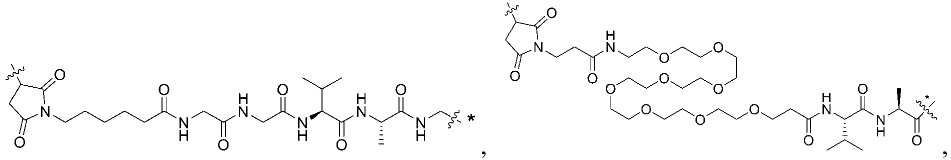 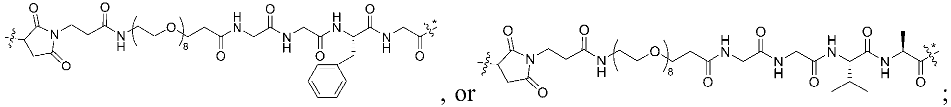 wherein the asterisk * end indicates the position where the linker connects to the payload drug molecule, and the other end indicates the position where the linker connects to the Ab.

In another aspect, the present disclosure provides a pharmaceutical composition comprising the compound of Formula I, Formula Ia, Formula Ib, Formula Ic, Formula Id, Formula Ia-1, Formula Ia-2, or Formula Ib-1, the stereoisomer thereof, or the pharmaceutically acceptable salt thereof, and a pharmaceutically acceptable excipient.

In yet another aspect, the present disclosure provides a use of the compound of Formula I, Formula Ia, Formula Ib, Formula Ic, Formula Id, Formula Ia-1, Formula Ia-2, or Formula Ib-1, the stereoisomer thereof, or the pharmaceutically acceptable salt thereof, or the pharmaceutical composition in the manufacture of a medicament for treating tumors.

In still another aspect, the present disclosure provides a method of treating tumors, comprising administering to a patient in need thereof an effective amount of the compound of Formula I, Formula Ia, Formula Ib, Formula Ic, Formula Id, Formula Ia-1, Formula Ia-2, or Formula Ib-1, the stereoisomer thereof, or the pharmaceutically acceptable salt thereof, or the pharmaceutical composition.

In still another aspect, the present disclosure provides the compound of Formula I, Formula Ia, Formula Ib, Formula Ic, Formula Id, Formula Ia-1, Formula Ia-2, or Formula Ib-1, the stereoisomer thereof, or the pharmaceutically acceptable salt thereof, or the pharmaceutical composition for use in treating tumors.

In still another aspect, the present disclosure provides a use of the compound of Formula I, Formula Ia, Formula Ib, Formula Ic, Formula Id, Formula Ia-1, Formula Ia-2, or Formula Ib-1, the stereoisomer thereof, or the pharmaceutically acceptable salt thereof, or the pharmaceutical composition in the treatment of tumors.

The payload compounds (*i.e.,* the payload drug molecule) in the compounds of Formula I, Formula Ia, Formula Ib, Formula Ic, Formula Id, Formula Ia-1, Formula Ia-2, or Formula Ib-1 of the present disclosure exhibit better stability in plasma, shorter half-life *in vivo,* and lower systemic exposure. Therefore, the payloads of the compounds of the present disclosure, upon detachment *in vivo,* are more readily eliminated and exhibit lower toxicity. Additionally, toxicity assays of the present disclosure have demonstrated that the compounds of the present disclosure exhibit lower *in vivo* toxicity and hematological toxicity, exhibiting improved safety.

### Terms and descriptions

Unless otherwise specified, the terms used herein have the general meanings in the technical field. A specific term or phrase without a particular definition should not be considered indefinite or unclear, but should be understood according to its ordinary meaning. When a trade name appears herein, it is intended to refer to the corresponding trade or its active ingredient.

In the present disclosure, the antibody Trastuzumab has the sequence shown in Table S1 below.

**Table S1. Sequence of the antibody Trastuzumab**

| | |
|---|---|
| Heavy chain variable region |  (SEQ ID NO: 4) |
| HCDR1 | DTYIH (SEQ ID NO: 1) |
| HCDR2 | RIYPTNGYTRYADSVKG (SEQ ID NO: 2) |
| HCDR3 | WGGDGFYAMDY (SEQ ID NO: 3) |
| Light chain variable region |  (SEQ ID NO: 9) |
| LCDR1 | RASQDVNTAVA (SEQ ID NO: 6) |
| LCDR2 | SASFLYS (SEQ ID NO: 7) |
| LCDR3 | QQHYTTPPT (SEQ ID NO: 8) |

More specifically, the antibody Trastuzumab has a heavy chain as set forth in SEQ ID NO: 5 and a light chain as set forth in SEQ ID NO: 10:

EVQLVESGGGLVQPGGSLRLSCAASGFNIKDTYIHWVRQAPGKGLEWVARIYPTNGY
TRYADSVKGRFTISADTSKNTAYLQMNSLRAEDTAVYYCSRWGGDGFYAMDYWGQ
GTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID
NO: 5);

DIQMTQSPSSLSASVGDRVTITCRASQDVNTAVAWYQQKPGKAPKLLIYSASFLYSGV
PSRFSGSRSGTDFTLTISSLQPEDFATYYCQQHYTTPPTFGQGTKVEIKRTVAAPSVFIFP
PSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 10).

In the present disclosure, the HCDR1, HCDR2, HCDR3, LCDR1, LCDR2, and LCDR3 are all encoded according to the Kabat scheme; the Kabat encoding scheme refers to an encoding scheme based on sequence variability.

In any one embodiment of the compound of Formula I of the present disclosure, when R₁ and R₂ are cyclized to form -O-(CH₂)ₘ-O-, the compound of Formula I is the following compound of Formula I-1. In the compound of Formula I of the present disclosure or any one embodiment thereof, when R₃ and R₄ are cyclized to form -(CH₂)ₖ-, -(CH₂)ₖ- is presented in the form of the following Formula I-2.

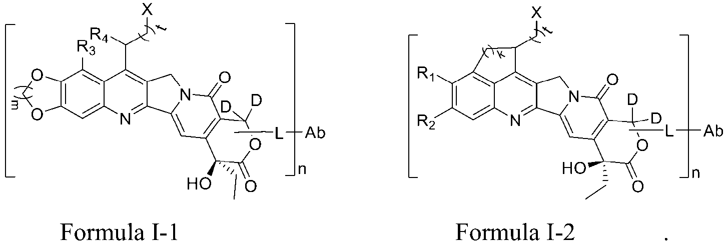

In the compound of Formula I of the present disclosure, 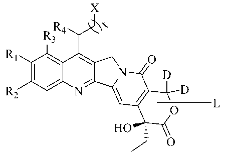 indicates that the linker L can form a chemical bond with any connectable site of the payload drug molecule D in Formula I, provided it is chemically feasible and can yield a robust drug.

It should be well-known to those skilled in the art that in the compounds of Formula I, Formula Ia, Formula Ib, Formula Ic, Formula Id, Formula Ia-1, Formula Ia-2, Formula Ib-1, or specific compounds of the present disclosure, the linker L or its corresponding specific structure is connected to Ab via a sulfur atom derived from Ab. This sulfur atom S may be reflected in the structural formula or chemical formula of L or L1, or may be omitted, hidden, or not reflected. For example, 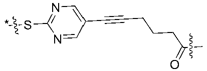 can also be written as 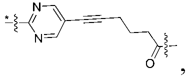 both representing the same structure and meaning; 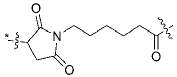 can also be written as 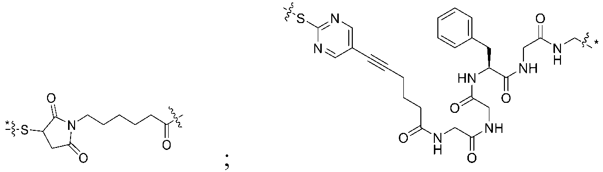 can be written as 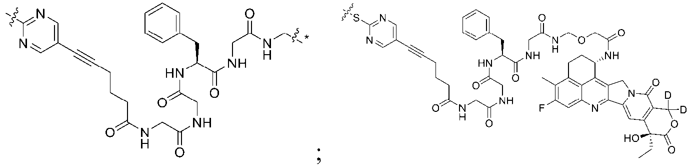 can also be written as 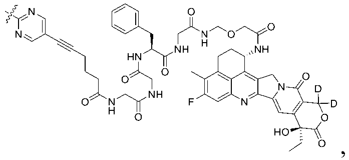 and other L1 moieties can be understood in the same way.

The term "payload drug molecule" is also referred to as "payload compound", "payload drug", or "payload", referring to a substance with preventive or therapeutic efficacy against diseases. In antibody-drug conjugates, the drugs typically refer to cytotoxic agents, which are chemical molecules capable of strongly disrupting the normal growth of tumor cells.

The term "linker" refers to a chemical structural moiety or bond that connects the ligand at one end and the drug at the other, or it may connect to other linker heads before attaching to the drug.

The term "drug-linker conjugate" is also referred to as payload-linker conjugate or linker-payload conjugate, which has the same meaning in the present disclosure.

The term "optional" or "optionally" means that the subsequently described event or circumstance may or may not occur, and the description includes instances where the event or circumstance occurs and instances where it does not occur. For example, the term "optionally" substituted means that a group may be unsubstituted or substituted with the designated substituent; whether substituted or not, both are encompassed within the scope of "optionally".

The term "independently" or "each independently" means that each of the aforementioned substituents of the term may select different optional variables from the specified range of choices, and is not influenced by the selection results of other substituents. When a certain substituent that may be "independent" has a plurality of identical substituents in the general chemical formula, it may still choose the same or different optional variables.

The term "halogen" or "halogen atom" refers to fluorine, chlorine, bromine, or iodine.

The term "halo" refers to a group formed by replacing one or more hydrogen atoms in a substituent with halogen atoms.

The term "alkyl" refers to a straight or branched hydrocarbon group where carbon atoms are connected by single bonds. The alkyl is preferably C₁₋₄ or C₁₋₆ alkyl. "C₁₋₄ alkyl" refers to a straight or branched alkyl group having 1, 2, 3, or 4 carbon atoms. Examples of C₁₋₄ alkyl include, but are not limited to, methyl, ethyl, *n*-propyl, isopropyl, *n*-butyl, *sec*-butyl, isobutyl, and *tert-*butyl. "C₁₋₆ alkyl" refers to a straight or branched alkyl group having 1, 2, 3, 4, 5, or 6 carbon atoms. Examples of C₁₋₆ alkyl include, but are not limited to: methyl, ethyl, *n*-propyl, isopropyl, *n*-butyl, *sec-*butyl, isobutyl, *tert-*butyl*, n*-pentyl, and *n*-hexyl.

The term "haloalkyl" refers to an alkyl group in which one or more hydrogen atoms are substituted by halogen atoms. Examples of C₁₋₄ haloalkyl include, but are not limited to, trifluoromethyl, trichloromethyl, 2,2,2-trifluoroethyl, and 2,2,2-trichloroethyl.

The term "alkoxy" refers to alkyl-O-, wherein alkyl is as defined above, including C₁₋₄ alkoxy or C₁₋₆ alkoxy. C₁₋₄ alkoxy can be understood as "C₁₋₄ alkyl-O-", examples of which include, but are not limited to, methoxy, ethoxy, *n*-propoxy, isopropoxy, *n*-butoxy, *sec*-butoxy, and *tert-*butoxy*.* C₁₋₆ alkoxy can be understood as "C₁₋₆ alkyl-O-", examples of which, in addition to the specific examples of C₁₋₄ alkoxy mentioned above, include but are not limited to *n*-pentyloxy, neopentyloxy, *n*-hexyloxy, *etc.*

The term "haloalkoxy" refers to an alkoxy group in which one or more hydrogen atoms are substituted by halogen atoms. Examples of C₁₋₄ haloalkoxy include, but are not limited to, trifluoromethoxy, trichloromethoxy, 2,2,2-trifluoroethoxy, and 2,2,2-trichloroethoxy.

The term "cycloalkyl" refers to a cyclic saturated monocyclic or polycyclic hydrocarbon group in which carbon atoms are connected by single bonds. The hydrogen atoms on the ring carbon atoms may optionally be substituted by oxo, *i.e.,* the "-CH₂-" in the ring may optionally be substituted by oxo to form "-C(O)-". 3- to 6-membered cycloalkyl refers to 3-, 4-, 5-, or 6-membered cycloalkyl, specific examples of which include, but are not limited to, cyclopropyl, cyclobutyl, cyclopentyl, and cyclohexyl.

The term "heterocycloalkyl" refers to a cycloalkyl group in which one or more (*e.g., 2,* 3, or 4) ring carbon atoms are replaced by a heteroatom or a heteroatom group (*i.e.,* a group containing a heteroatom). The ring carbon atoms refer to the carbon atoms constituting the cyclic skeleton structure; the heteroatom refers to an atom other than C and H in organic compounds, such as nitrogen atoms (N), oxygen atoms (O), sulfur atoms (S), phosphorus atoms (P), or boron atoms (B), wherein the heteroatoms such as nitrogen and sulfur atoms may be oxidized, and the nitrogen atoms may be quaternized; examples of the heteroatom group include but are not limited to -S(=O)₂-, -S(=O)-, and optionally substituted -NH-, - S(=O)(=NH)-, -C(=O)NH-, -C(=NH)-, -S(=O)₂NH-, S(=O)NH-, or -NHC(=O)NH-, *etc.* The term "3- to 6-membered heterocycloalkyl" refers to 3-, 4-, 5-, or 6-membered heterocycloalkyl, with specific examples including but not limited to aziridinyl, oxiranyl, thiiranyl, azetidinyl, oxetanyl, thietanyl, pyrrolidinyl, tetrahydrofuranyl, thiolanyl, piperazinyl, piperidinyl, tetrahydropyranyl, morpholinyl, and 1,4-dioxanyl.

In the present disclosure, the letters used to represent amino acids or amino acid residues are as follows: G represents glycine (Gly), F represents phenylalanine (Phe), V represents valine (Val), A represents alanine (Ala), and Cit represents citrulline.

The term "composition" is meant to include a product containing a specified amount of each of the specified ingredients, as well as any product derived directly or indirectly from a combination of the specified amount of each of the specified ingredients. Those skilled in the art can vary the actual dosage levels of the active ingredients in the pharmaceutical compositions of the present disclosure so that the amount of active compound obtained is effective to achieve the desired therapeutic response for a particular patient, composition, and mode of administration.

The term "pharmaceutically acceptable" refers to that for those compounds, materials, compositions and/or dosage forms, they are suitable for use in contact with the tissues of human and animal without excess toxicity, irritation, allergic reactions, or other problems or complications within the scope of reliable medical judgment, and are commensurate with a reasonable benefit/risk ratio.

The term "pharmaceutically acceptable salt" refers to a salt of a pharmaceutically acceptable acid or base, including salts formed by the compound with inorganic or organic acids, as well as salts formed by the compound with inorganic or organic bases.

The term "excipient" generally refers to carriers, diluents, and/or media required for formulating an effective pharmaceutical composition.

The term "effective amount" refers to an amount of the compound of the present disclosure or a pharmaceutically acceptable salt thereof that is suitable for treating a disease or condition with a reasonable benefit/risk ratio applicable to any medical treatment. The amount of the compound of the present disclosure that constitutes an "effective amount" varies depending on the compound, the disease condition and its severity, the route of administration, and the age of the mammal to be treated, but can be routinely determined by a person skilled in the art based on their own knowledge and the content of the present disclosure.

In the present disclosure, the " 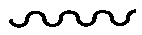 " in a substituent indicates the attachment position of the substituent to the parent structure or other moiety. The appearance of a dash "-" in a substituent structure is used to indicate the attachment point of the substituent. For example, -CH₃ indicates that the group is connected to the parent structure or other fragment through the C atom. " 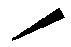 " and " 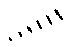 " denote the absolute configuration of a stereocenter, *i.e., R* or *S* configuration.

In the present disclosure, the chemical bonds depicted by solid and dashed lines " - - - - - -" represent single or double bonds, which can be determined within the scope commonly known to those skilled in the art based on the valences of the atoms at both ends of the chemical bond.

In the present disclosure, the term "isomer" includes geometric isomers and stereoisomers, such as atropisomers, *cis-trans* isomers, enantiomers, diastereomers, tautomers, and their racemic mixtures and other mixtures, all of which fall within the scope of the present disclosure. The term "enantiomer" refers to stereoisomers that are mirror images of each other. The term "tautomer" refers to a class of functional group isomers having different points of hydrogen attachment via displacement of one or more double bonds, for example, a ketone and its enol form are keto-enol tautomers. The term "diastereomer" refers to a stereoisomer where the molecule has two or more chiral centers and the molecules are not mirror images of each other. The term *"cis-trans* isomer" refers to different spatial configurations in which double bonds or single bonds of ring carbon atoms in a molecule cannot rotate freely. The term "atropisomer" refers to stereoisomers that can be separated due to hindered or extremely slow rotation of single bonds.

The stereoisomers of the compounds of the present disclosure may be prepared by chiral synthesis, chiral reagents, or other conventional techniques. For example, one enantiomer of a certain compound of the present disclosure may be prepared by asymmetric catalytic techniques or chiral auxiliary derivatization techniques. Alternatively, a single stereoisomeric compound can be obtained from a mixture via chiral resolution techniques. Alternatively, a single stereoisomeric compound can be prepared directly using chiral starting materials. The separation of optically pure compounds in the present disclosure is typically accomplished by preparative chromatography using a chiral chromatographic column to achieve the separation of chiral compounds.

In the present disclosure, the solution concentration unit M represents mol/L, mM represents mmol/L, and nM represents nmol/L. The solution concentration unit N represents equivalent concentration, which is expressed as the number of gram equivalents of solute contained in 1 liter of solution, denoted by the symbol N. For example, if 1 liter of concentrated hydrochloric acid contains 12.0 gram equivalents of hydrochloric acid (HCl), its concentration is 12.0 N. Equivalent concentration = number of gram equivalents of solute / volume of solution (L).

The trastuzumab used in the present disclosure was purchased from Sanyou Biopharmaceuticals (Shanghai) Co., Ltd. Those skilled in the art can also prepare it by referring to published literature.

The chemical abbreviations used in the present disclosure and their corresponding chemical names are as follows:

| **Chemical abbreviation** | **Chemical name** |
|---|---|
| DBU | 1,8-Diazabicyclo[5.4.0]undec-7-ene |
| HOBT | 1-Hydroxybenzotriazole |
| EDCI | Carbodiimide |
| DIPEA | Diisopropylethylamine |
| TCEP | Tris(2-carboxyethyl)phosphine hydrochloride |
| DME | Ethylene glycol dimethyl ether |
| DMTMM | 4-(4,6-Dimethoxy-1,3,5-triazin-2-yl)-4-methylmorpholinium chloride |
| MTBE | Methyl *tert-*butyl ether |

### Example

The present disclosure is further illustrated in detail below through specific preparation examples and biological experiments. However, it should be understood that these examples and biological experiments are provided solely for illustrative purposes and should not be construed as limiting the present disclosure in any form. It is clear to those skilled in the art that, unless otherwise specified, the materials used herein are well-known in the field, can be purchased commercially, or obtained by those skilled in the art based on published literature or conventional methods. Unless otherwise stated, all reactions in the present disclosure are carried out under continuous magnetic stirring in a dry nitrogen or argon atmosphere, using dry solvents, wherein: (i) temperatures are given in degrees Celsius (°C), and operations are performed at room temperature, which generally refers to 15-35°C, preferably 20-30°C, and more preferably 20-25°C; (ii) solvent removal is achieved by reduced-pressure evaporation using a rotary evaporator, with a bath temperature not exceeding 60°C; (iii) reaction progress is monitored by thin-layer chromatography (TLC); (iv) final products exhibit satisfactory hydrogen nuclear magnetic resonance spectroscopy (¹H-NMR) and/or mass spectrometry (MS) data.

### Example 1 Preparation of Compound 1

### Step 1:

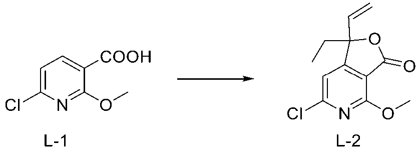

86.4 mL of 2,2,6,6-tetramethylpiperidine was added to a 3 L three-necked flask, followed by the addition of 1 L of anhydrous tetrahydrofuran. The system was cooled to - 78°C, and the reaction was carried out for 15 minutes. 272 mL of a 2.5 M *n*-butyllithium solution in petroleum ether was added, and the reaction was carried out for 30 minutes. 32 g of compound L-1 was dissolved in 200 mL of anhydrous tetrahydrofuran, then added to the system, and the reaction was carried out for 1 hour. 50 mL of 1-penten-3-one was dissolved in 200 mL of anhydrous tetrahydrofuran and then added to the system. The reaction was carried out for 1 hour. After completion of the reaction (monitored by TLC), 640 mL of 4 N hydrochloric acid was added, and the mixture was extracted with ethyl acetate. The organic phases were combined, washed with saturated aqueous sodium bicarbonate solution, washed with saturated brine, and dried over anhydrous sodium sulfate. After the system was concentrated under vacuum, 21 g of compound L-2 was obtained by column chromatography purification, with a yield of 49%. ESI-MS m/z: 253.68, [M+H]⁺.

¹H NMR (400 MHz, CDCl₃) δ 6.99 (s, 1H), 6.13 (dd, J = 17.3, 10.6 Hz, 1H), 5.29 (d, J = 7.0 Hz, 1H), 5.26 (s, 1H), 3.99 (s, 3H), 2.73 (s, 2H), 2.04 (dt, J = 14.8, 7.3 Hz, 1H), 1.98 - 1.87 (m, 1H), 0.91 (t, J = 7.3 Hz, 3H).

### Step 2:

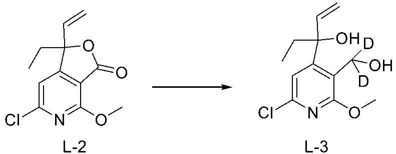

21 g of compound L-2 was added to a 1 L three-necked flask, followed by the addition of 400 mL of anhydrous ethanol. The system was cooled to 0°C, and then 14.21 g of sodium borodeuteride was added. The reaction mixture was stirred for 15 minutes and then warmed to room temperature to react for 12 hours. After completion of the reaction (monitored by TLC), 30 mL of 1 N hydrochloric acid was added, and the mixture was extracted with dichloromethane. The organic phases were combined, washed successively with saturated aqueous sodium bicarbonate solution and saturated brine, and dried over anhydrous sodium sulfate. After the system was concentrated under vacuum, 17 g of compound L-3 was obtained by column chromatography purification as a transparent viscous liquid, with a yield of 80%. ESI-MS m/z: 259.73, [M+H]⁺. ¹H NMR (400 MHz, CDCl₃) δ 6.99 (s, 1H), 6.13 (dd, J = 17.3, 10.6 Hz, 1H), 5.29 (d, J = 7.0 Hz, 1H), 5.26 (s, 1H), 3.99 (s, 3H), 2.73 (s, 2H), 2.04 (dt, J = 14.8, 7.3 Hz, 1H), 1.98 - 1.87 (m, 1H), 0.91 (t, J = 7.3 Hz, 3H).

### Step 3:

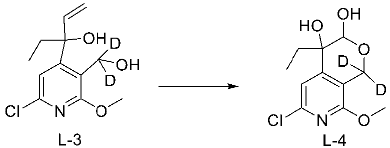

2 g of compound L-3 was added to a 250 mL round-bottom flask, followed by the addition of 100 mL of dichloromethane. The system was cooled to -78°C, and ozone was introduced under these conditions. After completion of the reaction (monitored by TLC), 0.5 mL of dimethyl sulfide was added, and the reaction mixture was warmed to room temperature and stirred for 30 minutes. After the system was concentrated under vacuum, 1.9 g of compound L-4 was obtained by column chromatography purification, with a yield of 95%. The product was directly used in the next step without further separation or purification. ESI-MS m/z: 261.70, [M+H]⁺.

¹H NMR (400 MHz, CDCl₃) δ 7.15 (s, 1H), 5.19 (d, J = 4.8 Hz, 1H), 3.96 (s, 3H), 3.21 (d, J = 4.8 Hz, 1H), 2.64 (s, 1H), 1.80 (q, J = 7.5 Hz, 2H), 0.92 (t, J = 7.5 Hz, 3H).

### Step 4:

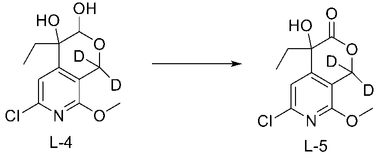

12 g of compound L-4 was added to a 1 L single-necked flask, followed by the addition of 240 mL of dichloromethane. The mixture was stirred for 30 minutes, and the system was cooled to 0°C. 286 mg of 2,2,6,6-tetramethylpiperidine oxide, 616 mg of sodium bicarbonate, 654 mg of potassium bromide, and 18 mL of water were added, and the reaction mixture was stirred for 15 minutes. 120 mL of aqueous sodium hypochlorite solution (>7.5wt%) was added, and the reaction was carried out for 30 minutes. After completion of the reaction (monitored by TLC), 12 g of sodium bisulfite was added, and the mixture was extracted with dichloromethane. The organic phases were combined, washed with water, washed with saturated brine, and dried over anhydrous sodium sulfate. After the system was concentrated under vacuum, 6.6 g of compound L-5 was obtained by column chromatography purification, with a yield of 55%. ESI-MS m/z: 259.68, [M+H]⁺.

¹H NMR (400 MHz, CDCl₃) δ 7.20 (s, 1H), 4.00 (s, 3H), 3.64 (s, 1H), 1.79 (q, J = 7.4 Hz, 2H), 0.97 (t, J = 7.4 Hz, 3H).

### Step 5:

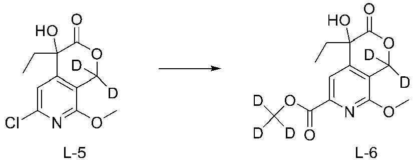

Under a carbon monoxide atmosphere, 3 g of compound L-5 was added to a 100 mL three-necked flask, followed by the addition of 286 mg of 1,3-bis(diphenylphosphino)propane, 2.4 g of potassium carbonate, 129 mg of palladium acetate, 15 mL of *N*,*N*-dimethylformamide, and 30 mL of deuterated methanol. The system was heated to 65°C, and the reaction was carried out for 12 hours. After completion of the reaction (monitored by TLC), 20 mL of 1 N hydrochloric acid was added, and the mixture was extracted with ethyl acetate. The organic phases were combined, washed successively with saturated sodium bicarbonate aqueous solution and saturated brine, and dried over anhydrous sodium sulfate. After the system was concentrated under vacuum, 1.33 g of compound L-6 was obtained by column chromatography purification, with a yield of 40%. ESI-MS m/z: 286.29, [M+H]⁺.

¹H NMR (400 MHz, CDCl₃) δ 7.98 (d, J = 2.0 Hz, 1H), 4.09 (d, J = 2.0 Hz, 3H), 3.71 (d, J = 2.6 Hz, 1H), 1.88 - 1.75 (m, 2H), 0.96 (td, J = 7.3, 1.9 Hz, 3H).

### Step 6:

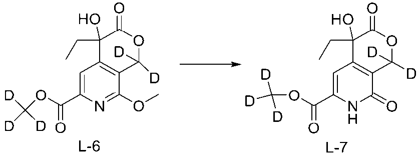

1 g of compound L-6 was added to a 50 mL two-necked flask, followed by the addition of 1.05 g of sodium iodide and 10 mL of acetonitrile. The system was cooled to 0°C and stirred for 30 minutes. 0.89 mL of trimethylsilyl chloride was added, and the system was warmed to room temperature to react for 12 hours. After completion of the reaction (monitored by TLC), 40 mL of water and 1 mL of saturated aqueous sodium bisulfite solution were added, and the mixture was stirred for 1 hour. The mixture was extracted with dichloromethane and dried over anhydrous sodium sulfate. After the system was concentrated under vacuum, 800 mg of compound L-7 was obtained by column chromatography purification, with a yield of 84%. ESI-MS m/z: 272.27, [M+H]⁺.

¹H NMR (400 MHz, CDCl₃) δ 10.03 (s, 1H), 7.32 (s, 1H), 3.90 (s, 1H), 1.81 (dd, J = 14.5, 7.2 Hz, 2H), 0.99 (t, J = 7.4 Hz, 3H).

### Step 7:

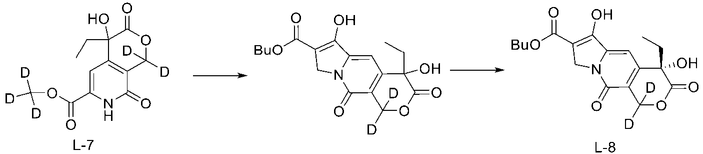

620 mg of compound L-7 was added to a 25 mL two-necked flask, followed by the addition of 1.48 g of cesium carbonate, 8 mL of dimethyl sulfoxide, and 1.67 mL of *tert-*butyl acrylate. The system was heated to 50°C, and the reaction was carried out for 12 hours. After completion of the reaction (monitored by TLC), 1 mL of concentrated hydrochloric acid was added, followed by the addition of 40 mL of water. The mixture was extracted with dichloromethane and dried over anhydrous sodium sulfate. After the system was concentrated under vacuum, 468 mg of compound L-8 was obtained by preparative SFC chiral separation (instrument model: SFC-350 (Waters), chromatographic column: AS 25 * 250 mm, 10 µm, mobile phase: CO₂/MeOH = 65/35, flow rate: 200 mL/min), with a yield of 55%. ESI-MS m/z: 365.38, [M+H]⁺.

### Step 8:

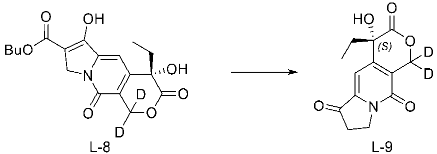

200 mg of compound L-8 was added to a 10 mL single-necked flask, followed by the addition of 5 mL of toluene and 0.5 mL of trifluoroacetic acid. The system was heated to 110°C, and the reaction was carried out for 2 hours. After completion of the reaction (monitored by TLC), the system was concentrated under vacuum, and 140 mg of compound L-9 was obtained by column chromatography purification, with a yield of 98%. ESI-MS m/z: 265.26, [M+H]⁺.

### Step 9:

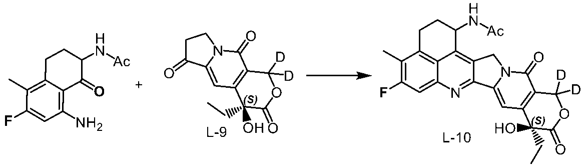

300 mg of compound L-9 was added to a 25 mL single-necked flask, followed by the addition of 266 mg of *N*-(5-methyl-6-fluoro-8-amino-1-oxo-1,2,3,4-tetrahydronaphthalen-2-yl)acetamide, 9 mL of toluene, 770 mL of *o*-cresol, and 133 mg of pyridinium *p-*toluenesulfonate. The system was heated to 110°C, and the reaction was carried out for 18 hours. After completion of the reaction (monitored by TLC), the system was concentrated under vacuum, and 443 mg of compound L-10 was obtained by column chromatography purification (dichloromethane: methanol = 10:1), with a yield of 81%.

### Step 10:

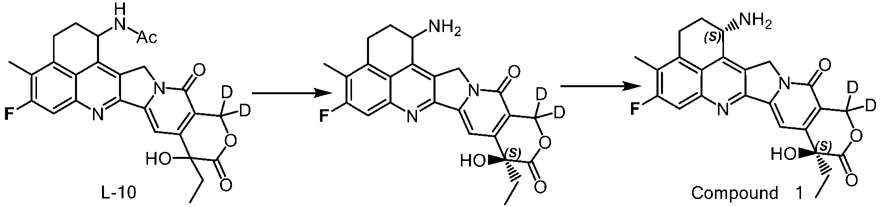

443 mg of intermediate compound L-10 was added to a 25 mL single-necked flask, followed by the addition of 7 mL of purified water and 2.2 mL of methanesulfonic acid. The system was purged with nitrogen. The system was heated to 85°C and reacted for 10 hours. After completion of the reaction (monitored by TLC), the system was concentrated under vacuum, and 141 mg of compound 1 or its methanesulfonate salt was obtained by column chromatography purification (dichloromethane: methanol), with a yield of 35%. ESI-MS m/z: 437.47, [M+H]⁺.

¹H NMR (400 MHz, D₂O) δ 7.20 - 7.06 (m, 2H), 5.42 - 5.14 (m, 5H), 3.30 (dd, J = 18.1, 4.0 Hz, 1H), 3.06 - 2.91 (m, 1H), 2.69 (s, 2H), 2.62 - 2.47 (m, 1H), 2.18 (s, 3H), 1.80 (q, J = 7.3 Hz, 2H), 0.79 (t,J = 7.4 Hz, 3H).

### Example 2 Preparation of Compound 2

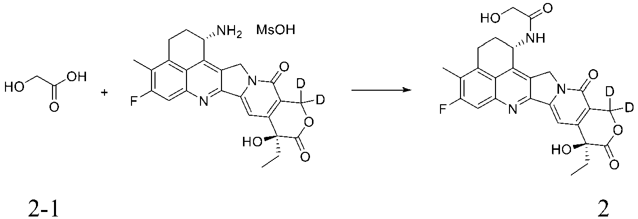

2.4 mL of water and 3.0 mL of tetrahydrofuran were added to a mixture of anhydrous sodium sulfate (2.25 eq), ethyl 2-cyano-2-hydroxyiminoacetate (2.25 eq), 2-hydroxyacetic acid (1.45 eq), and the methanesulfonate salt of compound 1 (100 mg). After stirring at room temperature for 15 minutes, *N*-methylmorpholine (1.10 eq) was added, and the mixture was stirred at room temperature for another 15 minutes. Subsequently, EDCI (2 eq) was added, and the reaction mixture was stirred at room temperature for 3 hours. After the complete reaction of compound 1 was monitored by HPLC, the reaction mixture was filtered, and the filter cake was dried. The title compound was obtained by silica gel column chromatography purification (DCM: MeOH).

¹H NMR (400 MHz, DMSO) δ 8.41 (d, *J =* 8.9 Hz, 1H), 7.77 (d, *J =* 11.0 Hz, 1H), 7.30 (s, 1H), 6.52 (s, 1H), 5.58 (d, *J =* 5.4 Hz, 1H), 5.49 (t, *J =* 5.7 Hz, 1H), 5.20 (t, *J =* 13.9 Hz, 2H), 3.96 (d, *J =* 5.5 Hz, 2H), 3.28 - 3.05 (m, 2H), 2.39 (s, 3H), 2.29 - 2.11 (m, 2H), 1.96 - 1.78 (m, 2H), 0.87 (t, *J* = 7.2 Hz, 3H). MS (ESI) m/z [M+H]⁺: 496.1.

### Example 3 Preparation of Compound 3

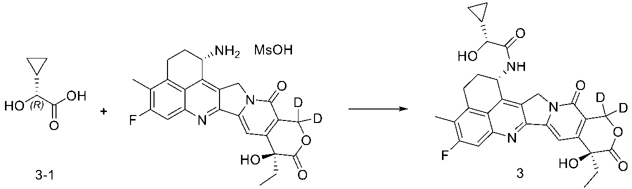

Referring to the preparation method of Example 2, 52 mg of compound 3 was prepared using (R)-2-cyclopropyl-2-hydroxyacetic acid as the starting material, with a yield of 52%.

¹H NMR (400 MHz, DMSO-d6) δ 8.36 (d, J = 8.4 Hz, 1H), 7.78 (d, J = 10.9 Hz, 1H), 7.31 (s, 1H), 6.52 (s, 0H), 5.58 - 5.48 (m, 1H), 5.38 (d, J = 5.3 Hz, 1H), 5.21 (q, J = 19.0 Hz, 2H), 3.62 (d, J = 5.2 Hz, 1H), 3.16 (dd, J = 11.2, 5.7 Hz, 3H), 2.39 (s, 4H), 2.22 - 2.12 (m, 3H), 1.86 (dt, J = 14.3, 7.1 Hz, 2H), 1.14 (q, J = 7.3 Hz, 2H), 0.87 (t, J = 7.2 Hz, 3H), 0.38 (dd, J = 20.9, 5.6 Hz, 4H). MS (ESI) m/z [M+H]⁺: 536.2.

### Example 4 Preparation of Compound 4

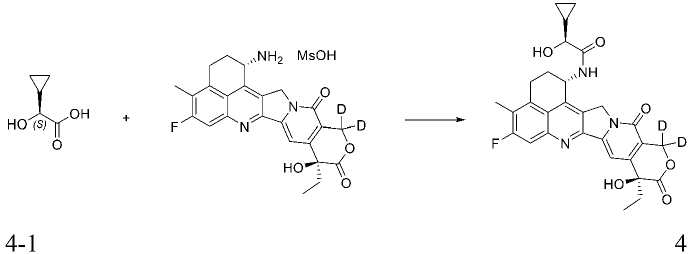

Referring to the preparation method of Example 2, 54 mg of compound 4 was prepared using (*S*)-2-cyclopropyl-2-hydroxyacetic acid as the starting material, with a yield of 54%.

¹H NMR (400 MHz, DMSO-*d*₆) δ 8.37 (d, *J =* 9.0 Hz, 1H), 7.77 (d, *J =* 11.0 Hz, 1H), 7.30 (s, 1H), 6.51 (s, 1H), 5.58 (q, *J =* 6.7 Hz, 1H), 5.49 (d, *J =* 5.2 Hz, 1H), 5.35 - 5.06 (m, 2H), 3.60 (t, *J =* 5.7 Hz, 1H), 3.17 (q, *J =* 17.2 Hz, 2H), 2.39 (s, 3H), 2.16 (d, *J =* 6.1 Hz, 2H), 1.85 (dq, *J =* 14.2, 7.1 Hz, 2H), 1.24 (q, *J =* 8.1 Hz, 1H), 0.87 (t, *J =* 7.3 Hz, 3H), 0.59 - 0.32 (m, 4H). MS (ESI) m/z [M+H]⁺: 536.2.

### Example 5 Preparation of Compound 5

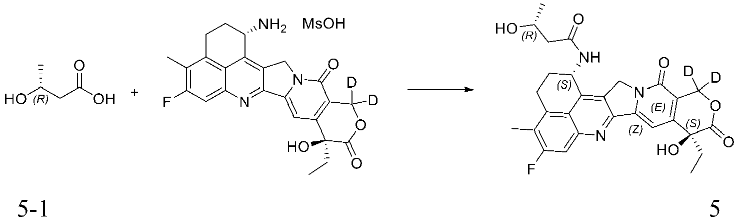

Referring to the preparation method of Example 2, 55 mg of compound 5 was prepared using (*R*)-3-hydroxybutyric acid as the starting material, with a yield of 56%.

¹H NMR (400 MHz, DMSO-d6) δ 8.42 (d, J = 8.7 Hz, 1H), 7.78 (d, J = 11.0 Hz, 1H), 7.30 (s, 1H), 6.52 (s, 1H), 5.55 (dt, J = 9.3, 5.0 Hz, 1H), 5.29 - 5.12 (m, 2H), 4.66 (d, J = 4.7 Hz, 1H), 4.05 (dt, J = 12.1, 6.0 Hz, 1H), 3.25 - 3.09 (m, 2H), 2.39 (s, 3H), 2.33 - 2.17 (m, 2H), 2.13 (dd, J = 10.7, 5.4 Hz, 2H), 1.86 (hept, J = 7.1 Hz, 2H), 1.09 (d, J = 6.2 Hz, 3H), 0.87 (t, J = 7.3 Hz, 3H). MS (ESI) m/z [M+H]⁺: 524.2.

### Example 6 Preparation of Compound 6

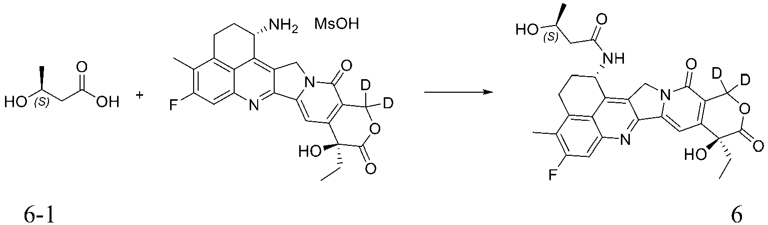

Referring to the preparation method of Example 2, 51 mg of compound 6 was prepared using (*S*)-3-hydroxybutyric acid as the starting material, with a yield of 52%.

¹H NMR (400 MHz, DMSO-*d*₆) δ 8.41 (d, *J =* 8.7 Hz, 1H), 7.78 (d, *J =* 11.0 Hz, 1H), 7.30 (s, 1H), 6.53 (s, 1H), 5.57 (dt, *J =* 8.8, 4.5 Hz, 1H), 5.22 (d, *J =* 2.7 Hz, 2H), 4.64 (d, *J =* 4.5 Hz, 1H), 4.05 (dt, *J =* 11.9, 6.1 Hz, 1H), 3.16 (t, *J =* 5.7 Hz, 2H), 2.39 (s, 3H), 2.32 - 2.17 (m, 2H), 2.17 - 2.04 (m, 2H), 1.86 (hept, *J=* 7.1 Hz, 2H), 1.08 (d, *J =* 6.1 Hz, 3H), 0.87 (t, *J =* 7.3 Hz, 3H). MS (ESI) m/z [M+H]⁺: 524.2.

### Example 7 Preparation of Compound 7

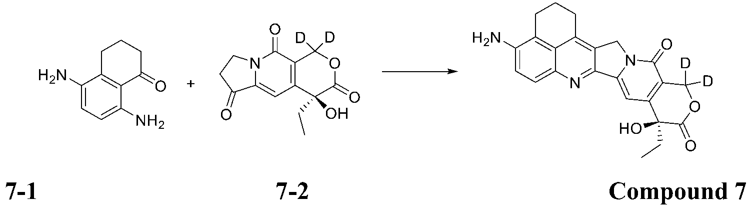

Compound 7-1 (87 mg, 1 eq), compound 7-2 (144 mg, 1.1 eq), pyridinium *p-*toluenesulfonate (50 mg, 0.4 eq), acetic acid (2.6 mL, 30 V), and toluene (2.6 mL, 30 V) were added to a reaction flask. The reaction was carried out at 110°C for 18 hours. After completion of the reaction (monitored by TLC), the solvent was removed by rotary evaporation. 123 mg of compound 7 was obtained by silica gel column chromatography purification (dichloromethane: methanol), with a yield of 61%.

¹H NMR (400 MHz, DMSO-d6) δ 7.72 (d, J = 9.0 Hz, 1H), 7.29 (d, J = 9.0 Hz, 1H), 7.17 (s, 1H), 6.45 (s, 1H), 5.65 (s, 2H), 5.16 (s, 2H), 3.04 (t, J = 6.1 Hz, 2H), 2.75 (t, J = 6.1 Hz, 2H), 2.08 - 1.95 (m, 2H), 1.85 (hept, J = 7.1 Hz, 2H), 0.88 (t, J = 7.2 Hz, 3H). MS (ESI) m/z [M+H]⁺: 406.1.

### Example 8 Preparation of Compound 8

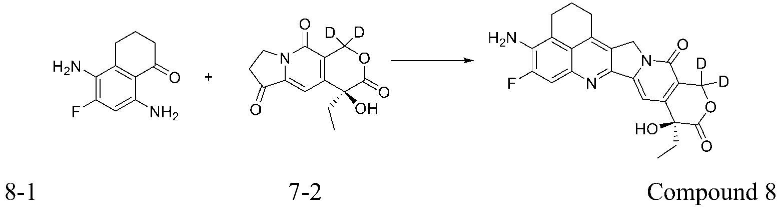

Referring to the preparation method of Example 7, 127 mg of compound 8 was prepared using compound 8-1 and 7-2 as the starting materials, with a yield of 60%.

¹H NMR (400 MHz, DMSO-*d*₆) δ 8.41 (d, *J =* 8.7 Hz, 1H), 7.78 (d, *J =* 11.0 Hz, 1H), 7.30 (s, 1H), 6.53 (s, 1H), 5.57 (dt, *J =* 8.8, 4.5 Hz, 1H), 5.22 (d, *J =* 2.7 Hz, 2H), 4.64 (d, *J =* 4.5 Hz, 1H), 4.05 (dt, *J=* 11.9, 6.1 Hz, 1H), 3.16 (t, *J =* 5.7 Hz, 2H), 2.39 (s, 3H), 2.32 - 2.17 (m, 2H), 2.17 - 2.04 (m, 2H), 1.86 (hept, *J =* 7.1 Hz, 2H), 1.08 (d, *J =* 6.1 Hz, 3H), 0.87 (t, *J =* 7.3 Hz, 3H). MS (ESI) m/z [M+H]⁺: 424.1.

### Example 9 Preparation of Compound 9

### Step 1:

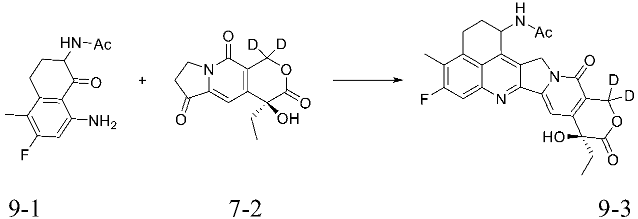

250 mg of compound 9-1, 300 mg of compound 7-2, 7.5 mL of acetic acid, 7.5 mL of toluene, and 68 mg of pyridinium *p*-toluenesulfonate were added to a 50 mL single-necked flask. The reaction was carried out at 110°C for 18 hours. After completion of the reaction (monitored by TLC), the solvent was evaporated under reduced pressure, and 386 mg of compound 9-3 was obtained by silica gel column chromatography purification (DCM: MeOH), with a yield of 81%. MS (ESI) m/z [M+H]⁺:480.2.

### Step 2:

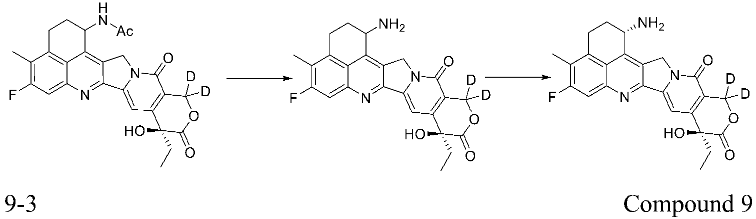

380 mg of compound 9-3 was added to a 100 mL single-necked flask, followed by the addition of 7.6 mL of purified water and 3.8 mL of methanesulfonic acid. The reaction was carried out at 85°C for 10 hours under a nitrogen atmosphere. After completion of the reaction (monitored by TLC), the reaction mixture was cooled to room temperature, and 22.8 mL of methanol was added. The mixture was stirred at room temperature for 2 hours, filtered, and the crude product was obtained. The crude product was purified by silica gel column chromatography (dichloromethane: methanol) to obtain 148 mg of compound 9, with a yield of 35%.

¹H NMR (400 MHz, D₂O) δ 7.20-7.06 (m, 2H), 5.42-5.14 (m, 5H), 3.30 (dd, J = 18.1, 4.0 Hz, 1H), 3.06-2.91 (m, 1H), 2.69 (s, 2H), 2.62-2.47 (m, 1H), 2.18 (s, 3H), 1.80 (q, J = 7.3 Hz, 2H), 0.79 (t, J = 7.4 Hz, 3H). MS (ESI) m/z [M+H]⁺: 438.2.

### Example 10 Preparation of Compound 10

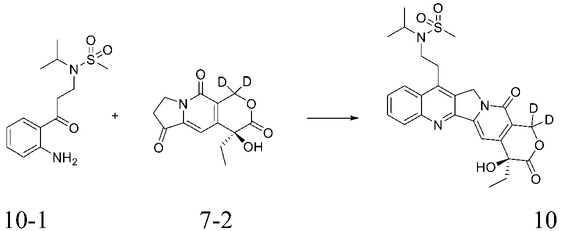

Referring to the preparation method of Example 9, 70 mg of compound 10 was prepared using compound 10-1 and compound 7-2 as the starting materials, with a yield of 34%.

¹H NMR (400 MHz, DMSO-d6) δ 8.32 (d, J = 8.4 Hz, 1H), 8.20 (dd, J = 8.4, 1.2 Hz, 1H), 7.93-7.84 (m, 1H), 7.79 (t, J = 7.6 Hz, 1H), 7.35 (s, 1H), 6.56 (s, 1H), 5.42 (s, 2H), 3.98 (p, J = 6.7 Hz, 1H), 3.50 (t, J = 8.0 Hz, 2H), 3.42-3.35 (m, 2H), 3.00 (s, 3H), 1.88 (hept, J = 7.3 Hz, 2H), 1.15 (d, J = 6.7 Hz, 6H), 0.88 (t, J = 7.3 Hz, 3H). MS (ESI) m/z [M+H]⁺: 514.2.

### Example 11 Preparation of Compound 11

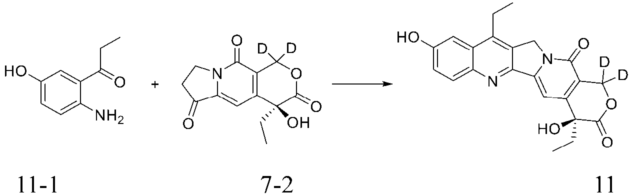

Referring to the preparation method of Example 9, 80 mg of compound 11 was prepared using compound 11-1 and compound 7-2 as the starting materials, with a yield of 37%.

¹H NMR (500 MHz, DMSO-d6) δ 0.88 (t, J = 7.25 Hz, 3H),1.32 (t, J = 7.5 Hz, 3H), 1.85 (m, 2H), 3.11 (q, J = 7.5 Hz, 2H), 5.26(s, 2H), 6.48(s, 1H), 7.23 (s, 1H), 7.41 (d, J = 10.0 Hz,2H), 8.01 (d, J *=* 10.0 Hz, 1H), 10.3 (s, 1H). MS (ESI) m/z [M+H]⁺: 395.1.

### Example 12 Preparation of Compound 12

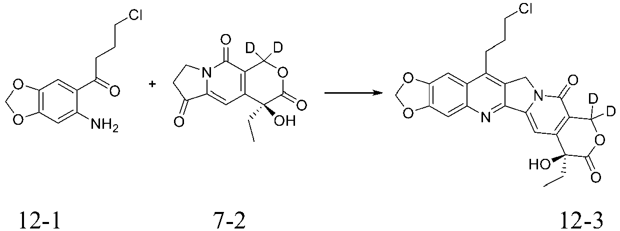

255 mg of compound 12-1, 300 mg of compound 7-2, 7.5 mL of acetic acid, 7.5 mL of toluene, and 68 mg of pyridinium *p*-toluenesulfonate were added to a 50 mL single-necked flask. The reaction was carried out at 110°C for 18 hours. After completion of the reaction (monitored by TLC), the solvent was evaporated under reduced pressure, and 330 mg of compound 12-3 was obtained by silica gel column chromatography purification (DCM: MeOH), with a yield of 70%.

¹H NMR (400 MHz, DMSO-d6) δ 7.63 (s, 1H), 7.51(s, 1H), 7.24 (s, 1H), 6.50 (s, 1H), 6.30 (s, 2H), 5.26 (s, 2H), 3.81 (d, J = 5.9 Hz, 2H), 3.22 (s, 2H), 1.98 (d, J = 6.7 Hz, 4H), 0.88 (t, J = 7.2 Hz, 3H). MS (ESI) m/z [M+H]⁺:471.1.

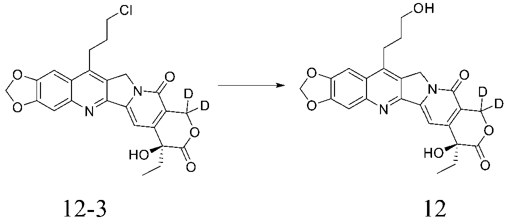

300 mg of intermediate compound 12-3 was dissolved in 15 mL of 10% sulfuric acid, and the reaction was carried out at 110°C for 48 hours. After completion of the reaction (monitored by TLC), the mixture was neutralized by adding a saturated aqueous sodium carbonate solution, extracted with dichloromethane, dried over anhydrous sodium sulfate, filtered, and concentrated under reduced pressure to obtain a crude product. The crude product was purified by silica gel column chromatography (DCM: MeOH) to obtain 89 mg of compound 12, with a yield of 31%.

¹H NMR (400 MHz, DMSO-d6) δ 7.63 (s, 1H), 7.50(s, 1H), 7.24 (s, 1H), 6.48 (s, 1H), 6.28 (s, 2H), 5.32-5.19 (m, 2H), 3.51-3.46 (m, 2H), 3.17-3.13 (m, 2H), 1.92 - 1.76 (m, 4H), 0.88 (t, J = 7.2 Hz, 3H). MS (ESI) m/z [M+H]⁺: 453.2.

### Example 13 Preparation of Compound 13

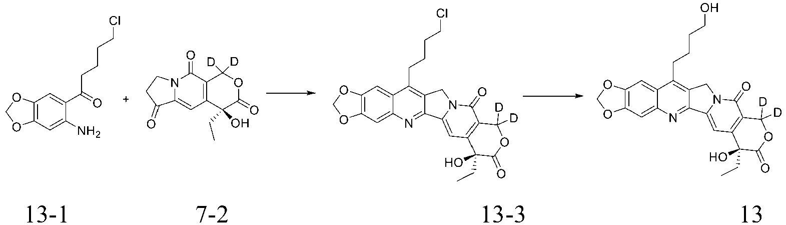

Referring to the preparation method of Example 12, 74 mg of compound 13 was prepared using compound 13-1 and compound 7-2 as the starting materials, with a two-step yield of 16%.

¹H NMR (400 MHz, DMSO-d6) δ 7.63 (s, 1H), 7.50(s, 1H), 7.24 (s, 1H), 6.48 (s, 1H), 6.28 (s, 2H), 5.32-5.19 (m, 2H), 3.51-3.46 (m, 2H), 3.17-3.13 (m, 2H), 1.94 - 1.76 (m, 6H), 0.88 (t, J = 7.2 Hz, 3H). MS (ESI) m/z [M+H]⁺: 467.2.

### Example 14 Preparation of Compound 14

### Step 1:

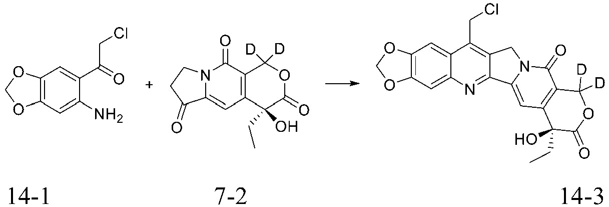

213 mg of compound 14-1, 300 mg of compound 7-2, 7.5 mL of acetic acid, 7.5 mL of toluene, and 68 mg of pyridinium p-toluenesulfonate were added to a 50 mL single-necked flask. The reaction was carried out at 110°C for 18 hours. After completion of the reaction (monitored by TLC), the solvent was removed by rotary evaporation, and 300 mg of compound 14-3 was obtained by silica gel column chromatography purification (DCM: MeOH), with a yield of 68%. MS (ESI) m/z [M+H]⁺: 443.1.

### Step 2:

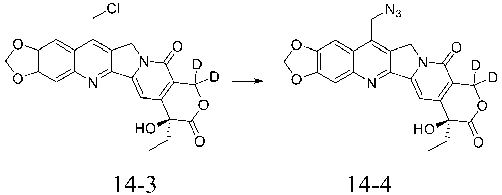

300 mg of compound 14-3 was dissolved in 3 mL of *N*,*N*-dimethylformamide, followed by the addition of sodium azide (48 mg, 1.1 eq). The reaction was carried out at 80°C for 16 hours. After completion of the reaction (monitored by TLC), the reaction mixture was cooled to room temperature, and 18 mL of water was added. The mixture was extracted with ethyl acetate, and the organic phases were combined. The organic phase was washed with water, dried over anhydrous sodium sulfate, filtered, and rotary evaporated to dryness to obtain 186 mg of crude compound 14-4, with a yield of 63%. MS (ESI) m/z [M+H]+: 436.1.

### Step 3:

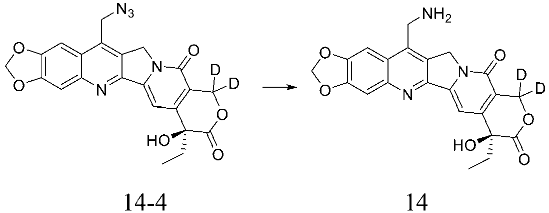

186 mg of compound 14-4 was dissolved in 4 mL of tetrahydrofuran, followed by the addition of triphenylphosphine (134 mg, 1.2 eq). The reaction mixture was stirred at room temperature for 4 hours. After completion of the reaction (monitored by TLC), hydrochloric acid (4 M, 1 mL) was added to the reaction system, and the mixture was reacted at 55°C for 16 hours. After concentration under reduced pressure, the residue was purified by a reverse-phase column (SEPA FLASH SW025, Spherical C18, 20-45 µm, 100 Å; mobile phase A: 0.05% formic acid/water, mobile phase B: acetonitrile; mobile phase A: mobile phase B = 40:60, flow rate 20 mL/min) to obtain 63 mg of compound 14, with a yield of 35%.

¹H NMR (400 MHz, DMSO-d6) δ 8.62 (s, 2H), 8.45 (s, 1H), 7.50 (s, 1H), 7.25 (s, 1H), 6.48 (s, 1H), 6.28 (s, 2H), 5.41 (s, 2H), 5.20 (s, 2H), 4.70 (d, J = 6.0 Hz, 2H), 1.86 (tt, J = 14.2, 6.1 Hz, 2H), 0.88 (t, J = 7.2 Hz, 3H). MS (ESI) m/z [M+H]⁺: 424.1.

### Example 15 Preparation of Compound 15

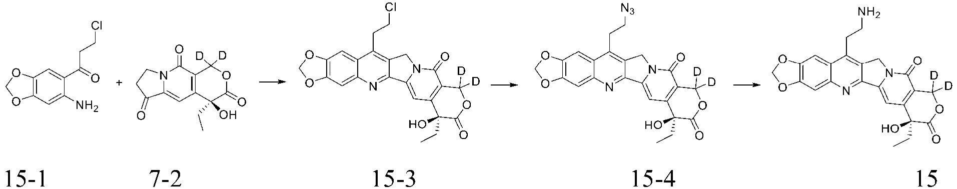

Referring to the preparation method of Example 14, 50 mg of compound 15 was prepared using compounds 15-1 and 7-2 as the starting materials, with a three-step yield of 11%.

¹H NMR (400 MHz, DMSO-d6) δ 8.62 (s, 2H), 8.45 (s, 1H), 7.50 (s, 1H), 7.25 (s, 1H), 6.48 (s, 1H), 6.28 (s, 2H), 5.41 (s, 2H), 5.20 (s, 2H), 3.51-3.46 (m, 2H), 3.17-3.13 (m, 2H), 1.86 (tt, J = 14.2, 6.1 Hz, 2H), 0.88 (t, J = 7.2 Hz, 3H). MS (ESI) m/z [M+H]⁺: 438.2.

### Example 16 Preparation of Compound 16

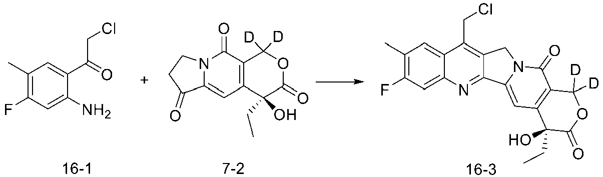

1 g of compound 16-1, 1.5 g of compound 7-2, 30 mL of acetic acid, 30 mL of toluene, and 340 mg of pyridinium *p*-toluenesulfonate were added to a 250 mL single-necked flask. The reaction was carried out at 110°C for 18 hours. After completion of the reaction (monitored by TLC), the solvent was removed by rotary evaporation, and the target intermediate compound 16-3 (1.6 g) was obtained by silica gel column chromatography purification (dichloromethane: methanol), with a yield of 75%. MS (ESI) m/z [M+H]+: 431.1.

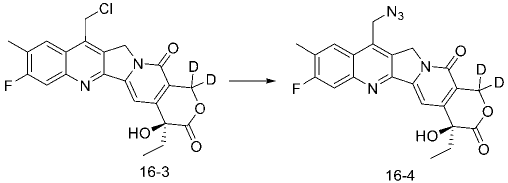

1.6 g of compound 16-3 was dissolved in 16 mL of *N,N-*dimethylformamide, followed by the addition of sodium azide (256 mg, 1.1 eq). The reaction was carried out at 80°C for 16 hours. After completion of the reaction (monitored by TLC), the reaction mixture was cooled to room temperature, and 96 mL of water was added. The mixture was extracted with ethyl acetate, and the organic phases were combined. The organic phase was washed with water, dried over anhydrous sodium sulfate, filtered, and concentrated under reduced pressure to obtain 1.1 g of crude compound 16-4, with a yield of 68%. MS (ESI) m/z [M+H]⁺: 438.2.

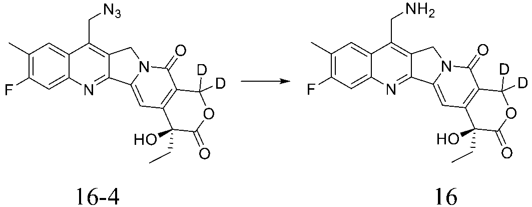

1.1 g of compound 16-4 was dissolved in 22 mL of tetrahydrofuran, followed by the addition of triphenylphosphine (0.79 g, 1.2 eq). The mixture was stirred at room temperature for 4 hours. After completion of the reaction (monitored by TLC), hydrochloric acid (4 M, 5 mL) was added to the reaction system, and the reaction was carried out at 55°C for 16 hours. The reaction mixture was concentrated under reduced pressure and purified by a reverse-phase column (instrument model: SEPA FLASH SW025, chromatographic column: Spherical C18, 20-45 µm, 100 Å, mobile phase A: 0.05% formic acid/water, mobile phase B: acetonitrile; mobile phase A: mobile phase B = 45:55, flow rate: 20 mL/min) to obtain 0.58 g of compound 16, with a yield of 56%.

¹H NMR (400 MHz, DMSO-d6) δ 8.62 (s, 3H), 8.43 (d, J = 8.0 Hz, 1H), 7.99 (d, J = 10.7 Hz, 1H), 7.35 (s, 1H), 5.59 (s, 2H), 4.70 (d, J = 6.0 Hz, 2H), 2.55 (s, 3H), 1.88 (hept, J = 7.1 Hz, 2H), 0.88 (t, J = 7.3 Hz, 3H). MS (ESI) m/z [M+H]⁺: 412.2.

### Example 17 Preparation of Compound 17

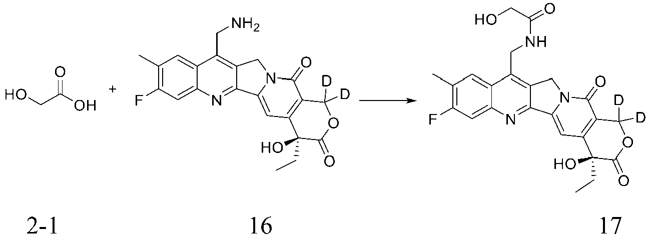

2.4 mL of water and 3.0 mL of tetrahydrofuran were added to a mixture of anhydrous sodium sulfate (2.25 eq), 2-hydroxyacetic acid (1.45 eq), and compound 16 (100 mg). After stirring at room temperature for 15 minutes, *N*-methylmorpholine (1.10 eq) was added, and the mixture was stirred at room temperature for 15 minutes. Subsequently, EDCI (2 eq) was added, and the reaction mixture was stirred at room temperature for 3 hours. After the complete reaction of compound 16 was monitored by HPLC, the mixture was extracted with dichloromethane, dried over anhydrous sodium sulfate, and filtered. The solvent was removed by rotary evaporation, and 82 mg of compound 17 was obtained by silica gel column chromatography purification (DCM: MeOH), with a yield of 72%.

¹H NMR (400 MHz, DMSO) δ 8.41 (d, J = 8.9 Hz, 1H), 7.77 (d, J = 11.0 Hz, 1H), 7.30 (s, 1H), 6.52 (s, 1H), 5.58 (d, J = 5.4 Hz, 1H), 5.49 (t, J = 5.7 Hz, 1H), 5.20 (t, J = 13.9 Hz, 2H), 3.96 (d, J = 5.5 Hz, 2H), 2.55 (s, 3H), 1.96 - 1.78 (m, 2H), 0.87 (t, J = 7.2 Hz, 3H). MS (ESI) m/z [M+H]⁺: 470.2.

### Example 18 Preparation of Compound 18

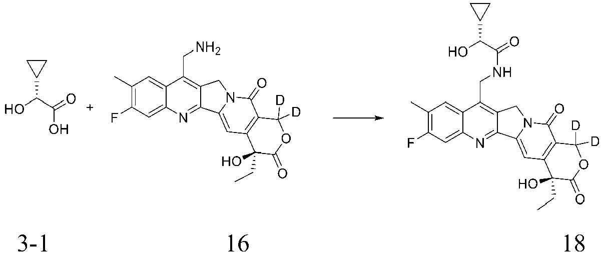

Referring to the preparation method of Example 17, 78 mg of compound 18 was prepared using compound 3-1 and compound 16 as the starting materials, with a yield of 63%.

¹H NMR (400 MHz, DMSO-d6) δ 8.36 (d, J = 8.4 Hz, 1H), 7.78 (d, J = 10.9 Hz, 1H), 7.31 (s, 1H), 6.52 (s, 0H), 5.58 - 5.48 (m, 1H), 5.38 (d, J = 5.3 Hz, 1H), 5.21 (q, J = 19.0 Hz, 2H), 3.62 (d, J = 5.2 Hz, 1H), 2.39 (s, 4H), 1.86 (dt, J = 14.3, 7.1 Hz, 2H), 1.14 (q, J = 7.3 Hz, 2H), 0.87 (t, J = 7.2 Hz, 3H), 0.38 (dd, J = 20.9, 5.6 Hz, 4H). MS (ESI) m/z [M+H]⁺: 510.2.

### Example 19 Preparation of Compound 19

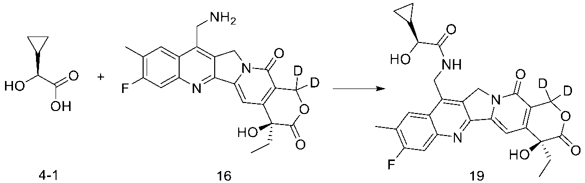

Referring to the preparation method of Example 17, 74 mg of compound 19 was prepared using compound 4-1 and compound 16 as the starting materials, with a yield of 60%.

¹H NMR (400 MHz, DMSO-d6) δ 8.37 (d, J = 9.0 Hz, 1H), 7.77 (d, J = 11.0 Hz, 1H), 7.30 (s, 1H), 6.51 (s, 1H), 5.58 (q, J = 6.7 Hz, 1H), 5.49 (d, J = 5.2 Hz, 1H), 5.35 - 5.06 (m, 2H), 3.60 (t, J = 5.7 Hz, 1H), 2.39 (s, 3H), 1.85 (dq, J = 14.2, 7.1 Hz, 2H), 1.24 (q, J = 8.1 Hz, 1H), 0.87 (t, J = 7.3 Hz, 3H), 0.59 - 0.32 (m, 4H). MS (ESI) m/z [M+H]⁺: 510.2.

### Example 20 Preparation of Compound 20

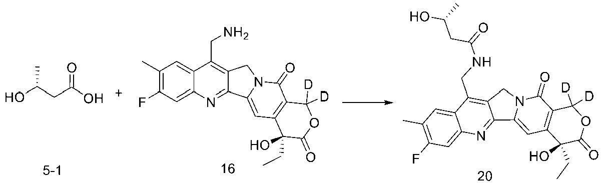

Referring to the preparation method of Example 17, 70 mg of compound 20 was prepared using compound 5-1 and compound 16 as the starting materials, with a yield of 58%.

¹H NMR (400 MHz, DMSO-d6) δ 8.42 (d, J = 8.7 Hz, 1H), 7.78 (d, J = 11.0 Hz, 1H), 7.30 (s, 1H), 6.52 (s, 1H), 5.55 (dt, J = 9.3, 5.0 Hz, 1H), 5.29 - 5.12 (m, 2H), 4.66 (d, J = 4.7 Hz, 1H), 4.05 (dt, J = 12.1, 6.0 Hz, 1H), 2.39 (s, 3H), 2.13 (dd, J = 10.7, 5.4 Hz, 2H), 1.86 (hept, J = 7.1 Hz, 2H), 1.09 (d, J = 6.2 Hz, 3H), 0.87 (t, J = 7.3 Hz, 3H). MS (ESI) m/z [M+H]⁺: 498.2.

### Example 21 Preparation of Compound 21

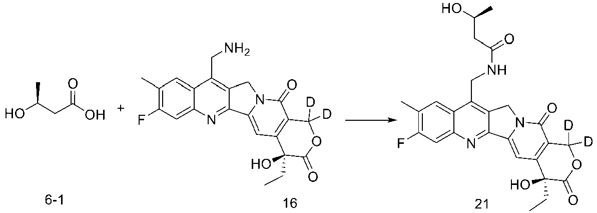

Referring to the preparation method of Example 17, 75 mg of compound 21 was prepared using compound 6-1 and compound 16 as the starting materials, with a yield of 62%.

¹H NMR (400 MHz, DMSO-d6) δ 8.41 (d, J = 8.7 Hz, 1H), 7.78 (d, J = 11.0 Hz, 1H), 7.30 (s, 1H), 6.53 (s, 1H), 5.57 (dt, J = 8.8, 4.5 Hz, 1H), 5.22 (d, J = 2.7 Hz, 2H), 4.64 (d, J = 4.5 Hz, 1H), 4.05 (dt, J = 11.9, 6.1 Hz, 1H), 2.39 (s, 3H), 2.17 - 2.04 (m, 2H), 1.86 (hept, J = 7.1 Hz, 2H), 1.08 (d, J = 6.1 Hz, 3H), 0.87 (t, J = 7.3 Hz, 3H). MS (ESI) m/z [M+H]⁺: 498.2.

### Example 22 Preparation of Compound LK1-1

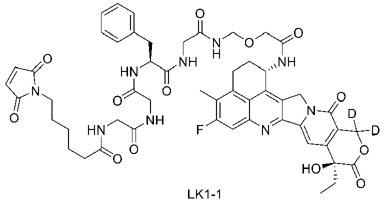

### Step 1:

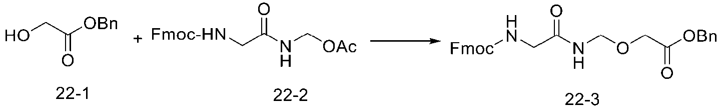

Compound 22-2 (160 g, 1.0 eq) was added to a 10 L four-necked flask, followed by the addition of 2.4 L of ethylene glycol dimethyl ether. The mixture was mechanically stirred, and compound 22-1 (123.37 mL, 2.0 eq) was added. The reaction was stirred at 0°C, and 10 mol/L sodium hydroxide (43.43 mL, 1.0 eq) was added dropwise via a constant-pressure dropping funnel. The mixture was stirred for 1 hour. The reaction was monitored by TLC. After the reaction was completed, acetic acid (12.42 mL) was added, and the mixture was stirred for 1 hour. Water (660 mL) was added, followed by stirring for 1 hour. Purified water (1.54 L) was then added, and the mixture was stirred for another 1 hour. The mixture was filtered, and the filter cake was washed with 50% (v/v) ethylene glycol dimethyl ether aqueous solution (640 mL) and dried in an oven at 40°C to obtain 204.3 g of compound 22-3, with a yield of 99%. MS (ESI) m/z: 475.2[M+H]⁺.

### Step 2:

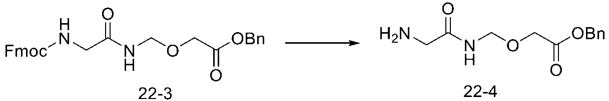

Compound 22-3 (180 g, 90%, 1.0 eq) was added to a 10 L four-necked flask, followed by the addition of 5.4 L of acetonitrile. The mixture was mechanically stirred, and DBU (25.5 mL, 0.5 eq) was added. The reaction was stirred at room temperature for 4 hours, after which HOBT (101.5 g, 2.2 eq) was added and stirred for 0.5 hours at room temperature. The temperature was then lowered to 0°C, and the mixture was stirred overnight and filtered. The filter cake was washed with acetonitrile (800 mL) and dried in an oven at 40°C to obtain 119.0 g of compound 22-4, with a yield of 81%. ¹H NMR (400 MHz, MeOD) δ 7.69 (dd, *J* = 12.9, 8.6 Hz, 2H), 7.40 - 7.24 (m, 7H), 5.19 (s, 2H), 4.80 (s, 2H), 4.21 (s, 2H), 3.66 (s, 2H).

### Step 3:

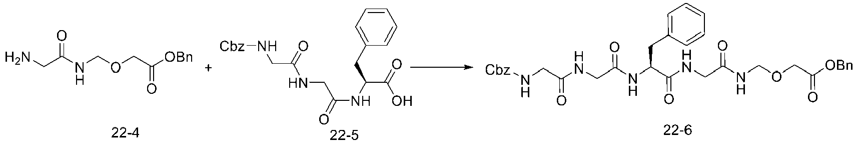

Compound 22-5 (55 g, 1.0 eq) was added to a 5 L three-necked flask, followed by the addition of 600 mL of acetonitrile and 100 mL of water to dissolve. Compound 22-4 (50 g, 1.0 eq) was then added. Under stirring at 0°C, EDCI (24 g, 1.0 eq) was added, and the mixture was stirred for 4 hours. After completion of the reaction (monitored by TLC), 500 mL of ethanol and 750 mL of water were added, and the mixture was stirred at room temperature overnight. Subsequently, an additional 1450 mL of water was added, and the mixture was stirred for 2 hours. The mixture was filtered, and the filter cake was dried in an oven at 40°C to obtain 80.0 g of compound 22-6, with a yield of 96%. MS (ESI) m/z: 648.3 [M+H]⁺.

### Step 4:

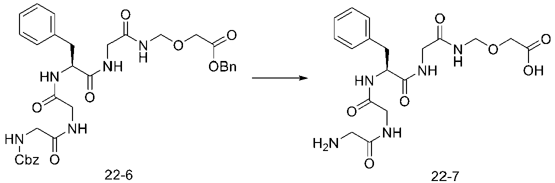

Under a nitrogen atmosphere, 840 mL of tetrahydrofuran and 540 mL of water were added to compound 22-6 (40.0 g, 1.0 eq), followed by the addition of Pd(5%)/C (8.8 g, 0.07 eq). The mixture was purged with hydrogen and stirred overnight at room temperature. After completion of the reaction (monitored by HPLC), the Pd/C was filtered off using diatomite. The Pd/C was washed with 300 mL of water, and the filtrates were combined. The filtrate was concentrated under reduced pressure, and 400 mL of ethanol was added for further concentration under reduced pressure. This process was repeated three times. Subsequently, 800 mL of ethanol was added, and the mixture was mechanically stirred, filtered, and the filter cake was dried in an oven at 40°C to obtain 19.6 g of compound 22-7, with a yield of 75%. MS (ESI) m/z: 424.2 [M+H]⁺.

¹H NMR (400 MHz, D₂O) δ 7.31 (ddd, *J* = 22.4, 14.6, 7.3 Hz, 5H), 4.72 - 4.56 (m, 3H), 3.94 (s, 2H), 3.93 - 3.73 (m, 6H), 3.08 (ddd, *J =* 22.1, 13.7, 7.7 Hz, 2H).

### Step 5:

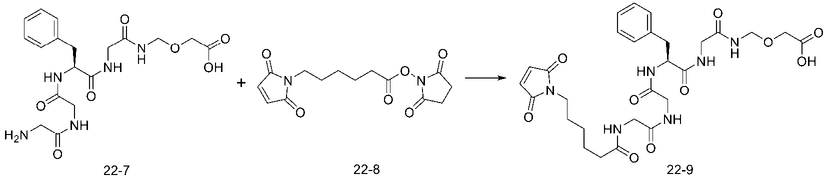

N-Succinimidyl 6-maleimidohexanoate (5.4 g, 1.5 eq) was added to a 250 mL single-necked flask, followed by dissolution in 45 mL of acetonitrile. Compound 22-7 (5.0 g, 1.0 eq), 105 mL of water, and DIPEA (1.56 mL, 0.8 eq) were added, and the mixture was stirred at room temperature overnight. After completion of the reaction (monitored by HPLC), 50 mL of isopropyl acetate, 10 g of anhydrous sodium dihydrogen phosphate, and 0.65 g of disodium hydrogen phosphate were added to the reaction mixture, followed by stirring for 0.5 hours. The organic phase was separated and removed. Then, 50 mL of isopropyl acetate was added, and the organic phase was again separated and removed. 25 mL of ethylene glycol dimethyl ether, 25 mL of ethyl acetate, 2.5 mL of acetonitrile, and 40 g of anhydrous sodium dihydrogen phosphate were added to the aqueous phase, stirred for 1 hour, and the aqueous phase was separated. The organic phase was treated twice with the addition of 75 mL of acetonitrile, 11.3 mL of water, 3 g of sodium chloride, and 750 mg of anhydrous sodium dihydrogen phosphate, followed by stirring and liquid separation to remove the aqueous phase. The organic phase was concentrated under reduced pressure to 50 mL, then 75 mL of ethylene glycol dimethyl ether was added and concentrated to 50 mL. Subsequently, 1 mL of water and 100 mL of ethylene glycol dimethyl ether were added, and the mixture was stirred overnight at room temperature. Subsequently, the mixture was filtered, and the filter cake was added with 200 mL of ethylene glycol dimethyl ether and 6.5 mL of water, stirred at 45°C for 0.5 hours, rinsed with 15 mL of a mixed solution of ethylene glycol dimethyl ether and water (v/v: 97/3), and the filtrates were combined and concentrated to 100 mL. Then, 25 mL of ethylene glycol dimethyl ether was added, and the mixture was stirred at room temperature overnight. The precipitated solid was rinsed with 25 mL of ethylene glycol dimethyl ether and dried under reduced pressure at 25°C to obtain compound 22-9, with a yield of 58%. MS (ESI) m/z: 617.3 [M+H]⁺.

¹H NMR (400 MHz, DMSO) δ 12.59 (s, 1H), 8.56 (t, *J =* 6.5 Hz, 1H), 8.31 (t, *J =* 5.5 Hz, 1H), 8.13 (d, *J =* 8.0 Hz, 1H), 8.08 (t, *J =* 5.2 Hz, 1H), 8.01 (t, *J =* 5.4 Hz, 1H), 7.21 (dt, *J* = 23.3, 6.0 Hz, 5H), 7.00 (s, 2H), 4.61 (d, *J =* 6.6 Hz, 2H), 4.49 (dd, *J =* 12.4, 8.7 Hz, 1H), 3.98 (s, 2H), 3.78 - 3.69 (m, 3H), 3.66 (d, *J =* 5.4 Hz, 2H), 3.59 (dd, *J =* 16.8, 5.3 Hz, 1H), 3.42 (s, 4H), 3.36 (t, *J* = 7.2 Hz, 3H), 3.23 (s, 6H), 3.08 - 3.02 (m, 1H), 2.84 - 2.76 (m, 1H), 2.10 (t, *J* = 7.4 Hz, 2H), 1.51 - 1.42 (m, 4H), 1.20 (dd, *J =* 15.1, 7.7 Hz, 2H).

### Step 6:

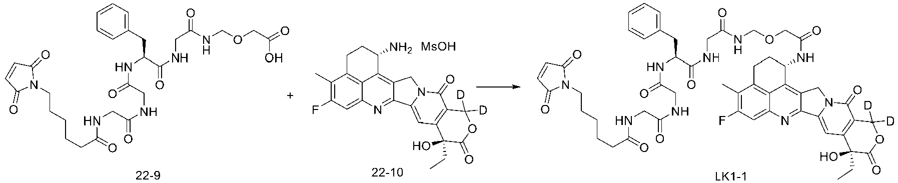

Anhydrous sodium sulfate (60 mg, 2.25 eq), ethyl cyanoglyoxylate-2-oxime (60 mg, 2.25 eq), compound 22-9 (20 mg, 1.45 eq), 0.8 mL of water, and 0.6 mL of tetrahydrofuran were added to a 10 mL single-necked flask, and the mixture was stirred at room temperature for 30 min. Compound 22-10 (100 mg, 1.0 eq), 0.3 mL of water, and 0.5 mL of tetrahydrofuran were added, and the mixture was stirred for 15 min. *N*-Methylmorpholine (23 µL, 1.1 eq) and 0.3 mL of tetrahydrofuran were added, and the mixture was stirred for another 15 min. EDCI (72 mg, 2.0 eq), 0.5 mL of water, and 0.5 mL of tetrahydrofuran were then added. The mixture was stirred at room temperature for 3 hours, and the reaction was monitored by HPLC until completion. The mixture was extracted with dichloromethane, dried, and the combined organic phases were rotary evaporated to dryness. The residue was purified by reverse-phase preparative chromatography (Santai C18 column (20 g), 35-40% acetonitrile/water) to obtain 99 mg of compound LK1-1, with a yield of 51%. MS (ESI) m/z: 1036.4[M+H]⁺.

¹H NMR (400 MHz, DMSO-d6) δ 8.62 (t, *J =* 6.5 Hz, 1H), 8.50 (d, *J =* 8.8 Hz, 1H), 8.29 (t, *J=* 5.7 Hz, 1H), 8.12 (d, *J = 7.9* Hz, 1H), 8.06 (t, *J =* 5.6 Hz, 1H), 8.00 (t, *J =* 5.5 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, *J =* 6.5 Hz, 2H), 4.53 - 4.42 (m, 1H), 4.02 (s, 2H), 3.79 - 3.53 (m, 6H), 3.35 (q, *J =* 6.3 Hz, 2H), 3.17 (q, *J =* 17.5 Hz, 2H), 3.02 (dd, *J =* 13.7, 4.3 Hz, 1H), 2.77 (dd, *J =* 13.7, 9.7 Hz, 1H), 2.37 (s, 3H), 2.18 (dt, *J =* 12.0, 6.9 Hz, 2H), 2.13 - 2.02 (m, 2H), 1.85 (hept, *J =* 7.1 Hz, 2H), 1.42 (tt, *J =* 14.3, 7.4 Hz, 4H), 1.17 (m, 2H), 0.85 (dt, *J =* 14.7, 7.3 Hz, 3H).

### Example 23 Preparation of Compound LK1-2

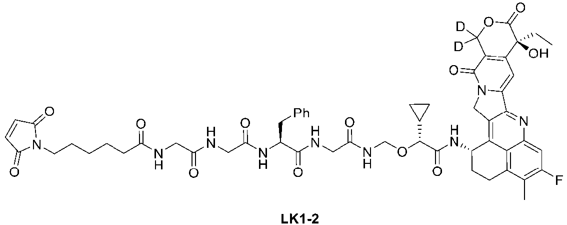

Referring to the preparation method of Example 22, compound LK1-2 was prepared by replacing compound 22-1 (*i.e.,* benzyl glycolate) with benzyl (*R*)-2-cyclopropyl-2-hydroxyacetate. MS (ESI) m/z: 1076.4 [M+H]⁺.

¹H NMR (400 MHz, DMSO-d6) δ 8.62 (t, *J =* 6.5 Hz, 1H), 8.50 (d, *J =* 8.8 Hz, 1H), 8.29 (t, *J =* 5.7 Hz, 1H), 8.12 (d, *J =* 7.9 Hz, 1H), 8.06 (t, *J=* 5.6 Hz, 1H), 8.00 (t, *J =* 5.5 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, J = 6.5 Hz, 2H), 4.53 - 4.42 (m, 1H), 3.79 - 3.53 (m, 7H), 3.35 (q, *J =* 6.3 Hz, 2H), 3.17 (q, *J =* 17.5 Hz, 2H), 3.02 (dd, *J =* 13.7, 4.3 Hz, 1H), 2.77 (dd, *J =* 13.7, 9.7 Hz, 1H), 2.37 (s, 3H), 2.18 (dt, *J=* 12.0, 6.9 Hz, 2H), 2.13 - 2.02 (m, 2H), 1.85 (hept, *J =* 7.1 Hz, 2H), 1.42 (tt, *J =* 14.3, 7.4 Hz, 4H), 1.17 (m, 3H), 0.85 (dt, *J=* 14.7, 7.3 Hz, 3H), 0.38 (dd, *J =* 20.9, 5.6 Hz, 4H).

### Example 24 Preparation of Compound LK1-3

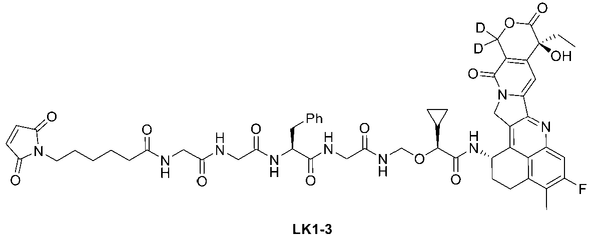

Referring to the preparation method of Example 22, compound LK1-3 was prepared by replacing compound 22-1 (*i.e.,* benzyl glycolate) with benzyl (S)-2-cyclopropyl-2-hydroxyacetate. MS (ESI) m/z: 1076.4 [M+H]⁺.

¹H NMR (400 MHz, DMSO-d6) δ 8.62 (t, *J =* 6.5 Hz, 1H), 8.50 (d, *J =* 8.8 Hz, 1H), 8.29 (t, *J* = 5.7 Hz, 1H), 8.12 (d, *J =* 7.9 Hz, 1H), 8.06 (t, *J =* 5.6 Hz, 1H), 8.00 (t, *J =* 5.5 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, *J =* 6.5 Hz, 2H), 4.53 - 4.42 (m, 1H), 3.79 - 3.53 (m, 7H), 3.35 (q, *J =* 6.3 Hz, 2H), 3.17 (q, *J =* 17.5 Hz, 2H), 3.02 (dd, *J =* 13.7, 4.3 Hz, 1H), 2.77 (dd, *J =* 13.7, 9.7 Hz, 1H), 2.37 (s, 3H), 2.18 (dt, *J =* 12.0, 6.9 Hz, 2H), 2.13 - 2.02 (m, 2H), 1.85 (hept, *J=* 7.1 Hz, 2H), 1.42 (tt, *J=* 14.3, 7.4 Hz, 4H), 1.24 (q, *J= 8.1* Hz, 1H), 1.17 (m, 2H), 0.85 (dt, *J=* 14.7, 7.3 Hz, 3H), 0.59 - 0.32 (m, 4H).

### Example 25 Preparation of Compound LK1-4

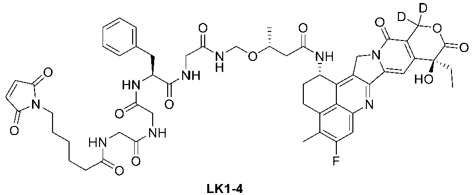

Referring to the preparation method of Example 22, compound LK1-4 was prepared by replacing compound 22-1 (*i.e.,* benzyl glycolate) with benzyl (R)-3-hydroxybutanoate. MS (ESI) m/z: 1064.4 [M+H]⁺.

¹H NMR (400 MHz, DMSO-d6) δ 8.62 (t, *J =* 6.5 Hz, 1H), 8.50 (d, *J =* 8.8 Hz, 1H), 8.29 (t, *J* = 5.7 Hz, 1H), 8.12 (d, *J* = 7.9 Hz, 1H), 8.06 (t, *J =* 5.6 Hz, 1H), 8.00 (t, *J =* 5.5 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, *J =* 6.5 Hz, 2H), 4.53 - 4.42 (m, 1H), 4.05 (dt, *J =* 12.1, 6.0 Hz, 1H), 3.79 - 3.53 (m, 6H), 3.35 (q, *J =* 6.3 Hz, 2H), 3.17 (q, *J =* 17.5 Hz, 2H), 3.02 (dd, *J =* 13.7, 4.3 Hz, 1H), 2.77 (dd, *J =* 13.7, 9.7 Hz, 1H), 2.37 (s, 3H), 2.18 (dt, *J =* 12.0, 6.9 Hz, 2H), 2.13 - 2.02 (m, 4H), 1.85 (hept, *J =* 7.1 Hz, 2H), 1.42 (tt, *J =* 14.3, 7.4 Hz, 4H), 1.17 (m, 2H), 1.09 (d, *J =* 6.2 Hz, 3H), 0.85 (dt, *J =* 14.7, 7.3 Hz, 3H).

### Example 26 Preparation of Compound LK1-5

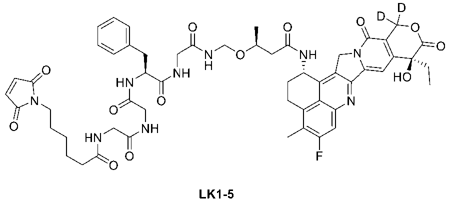

Referring to the preparation method of Example 22, compound LK1-5 was prepared by replacing compound 22-1 (*i.e.,* benzyl glycolate) with benzyl (*S*)-3-hydroxybutanoate. MS (ESI) m/z: 1064.4 [M+H]⁺.

¹H NMR (400 MHz, DMSO-d6) δ 8.62 (t, *J =* 6.5 Hz, 1H), 8.50 (d, *J =* 8.8 Hz, 1H), 8.29 (t, *J =* 5.7 Hz, 1H), 8.12 (d, *J =* 7.9 Hz, 1H), 8.06 (t, *J =* 5.6 Hz, 1H), 8.00 (t, *J =* 5.5 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, *J =* 6.5 Hz, 2H), 4.53 - 4.42 (m, 1H), 4.05 (dt, *J* = 11.9, 6.0 Hz, 1H), 3.79 - 3.53 (m, 6H), 3.35 (q, *J =* 6.3 Hz, 2H), 3.17 (q, *J =* 17.5 Hz, 2H), 3.02 (dd, *J =* 13.7, 4.3 Hz, 1H), 2.77 (dd, *J =* 13.7, 9.7 Hz, 1H), 2.37 (s, 3H), 2.18 (dt, *J =* 12.0, 6.9 Hz, 2H), 2.13 - 2.02 (m, 4H), 1.85 (hept, *J =* 7.1 Hz, 2H), 1.42 (tt, *J =* 14.3, 7.4 Hz, 4H), 1.17 (m, 2H), 1.08 (d, *J =* 6.1 Hz, 3H), 0.85 (dt, *J =* 14.7, 7.3 Hz, 3H).

### Example 27 Preparation of Compound LK1a-1

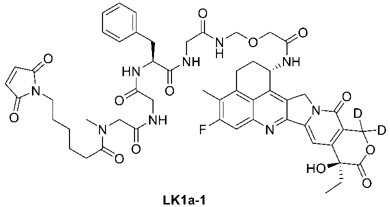

### Step 1:

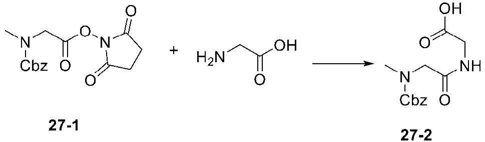

Glycine (3 g, 1.00 eq) was added to a 250 mL four-necked flask, followed by the addition of 90 mL of tetrahydrofuran and 30 mL of purified water. Sodium bicarbonate (3.70 g, 1.10 eq) was then added, and the mixture was mechanically stirred until the solution became clear. Compound 27-1 (12.8 g, 1.00 eq) was dissolved in DME (30 mL) and slowly added via a constant-pressure dropping funnel, after which the mixture was stirred at room temperature overnight. 100 mL of saturated sodium bicarbonate and 100 mL of purified water were added, washed three times with 100 mL of ethyl acetate, and 12 M hydrochloric acid was slowly added dropwise to adjust the pH to 4. The mixture was filtered, and the filter cake was rinsed with water and dried to obtain 10.1 g of compound 27-2, with a yield of 98%. MS (ESI) m/z: 281.1 [M+H]⁺.

### Step 2:

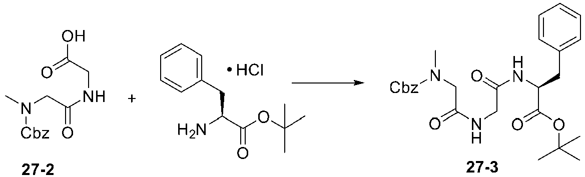

500 mL of *tert*-butyl *L*-phenylalaninate hydrochloride (6.5 g, 1.00 eq) and compound 27-2 (7.8 g, 1.1 eq) were added to a four-necked flask, followed by the addition of 200 mL of N,N-dimethylformamide. Triethylamine (3.5 mL, 1.00 eq) and DMTMM (8.9 g, 1.20 eq) were then added, and the mixture was stirred at room temperature for 2 hours. The reaction mixture was added to 1 L of purified water, filtered, and the filter cake was rinsed with water to obtain 11 g of compound 27-3, with a yield of 90%. MS (ESI) m/z: 484.2 [M+H]⁺.

### Step 3:

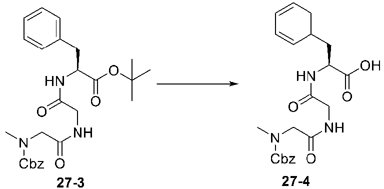

Compound 27-3 (10 g, 1.0 eq), 100 mL of dichloromethane, and trifluoroacetic acid (50 mL, 30.00 eq) were added to a 250 mL four-necked flask and stirred at room temperature for 2 hours. The reaction mixture was concentrated, triturated with MTBE, filtered, and the filter cake was rinsed with water to obtain 8.5 g of compound 27-4, with a yield of 96%. MS (ESI) m/z: 428.2[M+H]⁺.

### Step 4:

Referring to the preparation method of Example 22, compound LK1a-1 was prepared by replacing N-(benzyloxy)carbonylglycylglycyl-*L*-phenylalanine (*i.e.,* compound 22-5) with *N*-((benzyloxy)carbonyl)-*N*-methylglycylglycyl-*L*-phenylalanine (*i.e.,* compound 27-4). MS (ESI) m/z: 1050.4[M+H]⁺.

¹H NMR (400 MHz, DMSO-d6) δ 8.62 (t, *J =* 6.5 Hz, 1H), 8.50 (d, *J =* 8.8 Hz, 1H), 8.29 (t, *J =* 5.7 Hz, 1H), 8.12 (d, *J = 7.9* Hz, 1H), 8.06 (t, *J =* 5.6 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, *J =* 6.5 Hz, 2H), 4.53 - 4.42 (m, 1H), 4.02 (s, 2H), 3.79 - 3.53 (m, 6H), 3.35 (q, *J =* 6.3 Hz, 2H), 3.17 (q, *J =* 17.5 Hz, 2H), 3.02 (dd, *J =* 13.7, 4.3 Hz, 1H), 2.97 (s, 3H), 2.77 (dd, *J =* 13.7, 9.7 Hz, 1H), 2.37 (s, 3H), 2.18 (dt, *J =* 12.0, 6.9 Hz, 2H), 2.13 - 2.02 (m, 2H), 1.85 (hept, *J =* 7.1 Hz, 2H), 1.42 (tt, *J =* 14.3, 7.4 Hz, 4H), 1.17 (m, 2H), 0.85 (dt, *J=* 14.7, 7.3 Hz, 3H).

### Example 28 Preparation of Compound LK1b-1

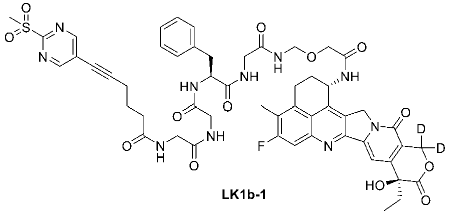

Referring to the preparation method of Example 22, compound LK1b-1 was prepared by replacing compound 22-8 with 2,5-dioxopyrrolidin-1-yl 6-(2-(methylsulfonyl)pyrimidin-5-yl)hex-5-ynoate (CAS No.: 2813270-39-0). MS (ESI) m/z: 1093.4[M+H]⁺.

¹H NMR (400 MHz, DMSO-d6) δ 9.10 (s, 2H), 8.64 (t, *J =* 6.6 Hz, 1H), 8.51 (d, *J* = 8.8 Hz, 1H), 8.31 (t, *J =* 5.7 Hz, 1H), 8.26 - 8.00 (m, 3H), 7.77 (d, *J =* 10.9 Hz, 1H), 7.36 - 7.10 (m, 6H), 6.52 (s, 1H), 5.67 - 5.52 (m, 1H), 5.19 (s, 2H), 4.64 (d, *J =* 6.5 Hz, 2H), 4.47 (dt, *J =* 12.9, 6.7 Hz, 1H), 4.02 (s, 2H), 3.77 - 3.68 (m, 4H), 3.63 - 3.57 (m, 1H), 3.40 (s, 3H), 3.17 (td, *J =* 12.8, 11.5, 6.7 Hz, 2H), 3.02 (dd, *J =* 13.7, 4.2 Hz, 1H), 2.77 (dd, *J =* 13.7, 9.7 Hz, 1H), 2.38 (s, 3H), 2.32 (t, *J* = 7.3 Hz, 2H), 2.17 (dd, *J =* 13.4, 6.8 Hz, 2H), 2.08 (s, 1H), 1.84 (dq, *J* = 18.9, 7.0 Hz, 4H), 1.28 - 1.22 (m, 2H), 0.87 (t, *J =* 7.2 Hz, 3H).

### Example 29 Preparation of Compound LK1c-1

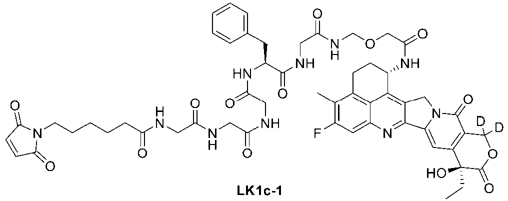

Referring to the preparation method of Example 22, compound LK1c-1 was prepared by replacing compound 22-8 with 2,5-dioxopyrrolidin-1-yl (6-(2,5-dioxo-2,5-dihydro-1*H-*pyrrol-1-yl)hexanoyl)glycinate (CAS No.: 1956326-21-8). MS (ESI) m/z: 1093.4 [M+H]⁺.

¹H NMR (400 MHz, DMSO-d6) δ 8.62 (t, *J =* 6.5 Hz, 1H), 8.50 (d, *J =* 8.8 Hz, 1H), 8.29 (t, *J =* 5.7 Hz, 1H), 8.12 (d, *J = 7.9* Hz, 1H), 8.06 (t, *J =* 5.6 Hz, 1H), 8.00 (t, *J =* 5.5 Hz, 1H), 7.96 (t, *J =* 5.5 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, *J =* 6.5 Hz, 2H), 4.53 - 4.42 (m, 1H), 4.02 (s, 2H), 3.80 - 3.53 (m, 8H), 3.35 (q, *J* = 6.3 Hz, 2H), 3.17 (q, *J= 17.5* Hz, 2H), 3.02 (dd, *J =* 13.7, 4.3 Hz, 1H), 2.77 (dd, *J* = 13.7, 9.7 Hz, 1H), 2.37 (s, 3H), 2.18 (dt, *J =* 12.0, 6.9 Hz, 2H), 2.13 - 2.02 (m, 2H), 1.85 (hept, *J =* 7.1 Hz, 2H), 1.42 (tt, *J =* 14.3, 7.4 Hz, 4H), 1.17 (m, 2H), 0.85 (dt, *J =* 14.7, 7.3 Hz, 3H).

### Example 30 Preparation of Compound LK1d-1

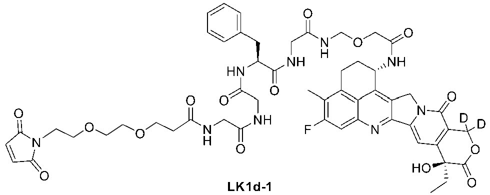

### Step 1:

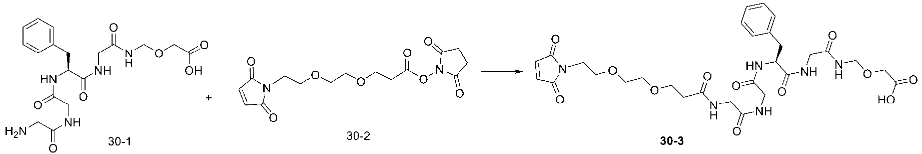

### Step 2:

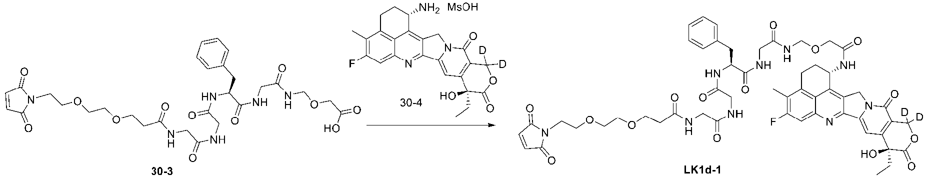

Referring to the preparation method of Example 22, LK1d-1 was prepared by replacing compound 22-8 with 2,5-dioxo-1-pyrrolidinyl 3-[2-[2-(2,5-dihydro-2,5-dioxo-1*H*-pyrrol-1-yl)ethoxy]ethoxy]propanoate (CAS: 1433997-01-3). MS (ESI) m/z: 1082.4 [M+H]⁺.

¹H NMR (400 MHz, DMSO-d6) δ 8.62 (t, *J* = 6.5 Hz, 1H), 8.50 (d, *J =* 8.8 Hz, 1H), 8.29 (t, *J* = 5.7 Hz, 1H), 8.12 (d, *J* = 7.9 Hz, 1H), 8.06 (t, *J* = 5.6 Hz, 1H), 8.00 (t, *J =* 5.5 Hz, 1H), 7.75 (d, *J =* 10.9 Hz, 1H), 7.30 (s, 1H), 7.20 (dp, *J =* 20.5, 7.2, 6.7 Hz, 5H), 6.99 (s, 2H), 6.51 (s, 1H), 5.59 (q, *J =* 6.6, 6.1 Hz, 1H), 5.27 - 5.08 (m, 2H), 4.64 (d, *J =* 6.5 Hz, 2H), 4.53 - 4.42 (m, 1H), 4.02 (s, 2H), 3.79 - 3.53 (m, 16H), 3.17 (q, *J =* 17.5 Hz, 2H), 3.02 (dd, *J =* 13.7, 4.3 Hz, 1H), 2.77 (dd, *J =* 13.7, 9.7 Hz, 1H), 2.37 (s, 3H), 2.28 (dt, *J* = 12.0, 6.9 Hz, 2H), 2.13 - 2.02 (m, 2H), 1.85 (hept, *J =* 7.1 Hz, 2H), 0.85 (dt, *J* = 14.7, 7.3 Hz, 3H).

### Example 31 Preparation of Compound LK1b-1

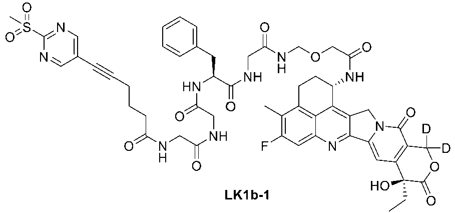

### Step 1:

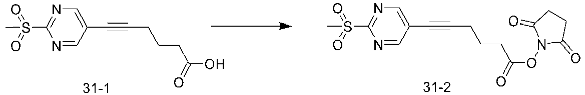

6-(2-(Methylsulfonyl)pyrimidin-5-yl)hex-5-ynoic acid (10.0 g, 1.0 eq) and *N-*hydroxysuccinimide (5.15 g, 1.2 eq) were added to a 250 mL single-necked flask and dissolved in 100 mL of dichloromethane, followed by the addition of *N*,*N*'-dicyclohexylcarbodiimide (10.0 g, 1.3 eq). The mixture was stirred at room temperature overnight. After completion of the reaction (monitored by TLC), the mixture was filtered, and the filtrate was concentrated under reduced pressure to obtain 14.0 g of compound 31-2. The product was directly used in the next step without further separation or purification. MS(ESI) m/z: 366.36 [M+H]⁺.

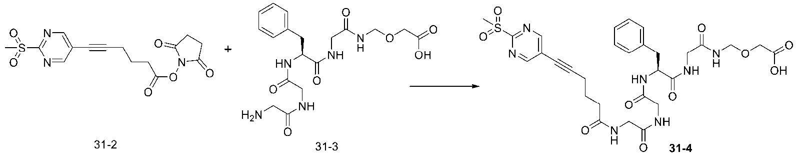

31-2 (13.6 g, 1.0 eq) was added to a 250 mL single-necked flask, dissolved in 135 mL of acetonitrile, followed by the addition of compound 31-3 (18.9 g, 1.2 eq), 270 mL of water, and DIPEA (5.3 mL, 0.8 eq). The mixture was stirred at room temperature overnight. After completion of the reaction (monitored by HPLC), the reaction mixture was extracted three times with 300 mL of ethyl acetate. 10 g of anhydrous sodium dihydrogen phosphate was added to the aqueous phase, and the aqueous phase was extracted three times with 300 mL of ethylene glycol dimethyl ether. The ethylene glycol dimethyl ether layers were combined, dried over anhydrous sodium sulfate, filtered, and concentrated under reduced pressure to obtain the crude product. The crude product was purified by reverse-phase preparative chromatography (acetonitrile/water) to obtain 18.6 g of compound 31-4, with a yield of 74%.

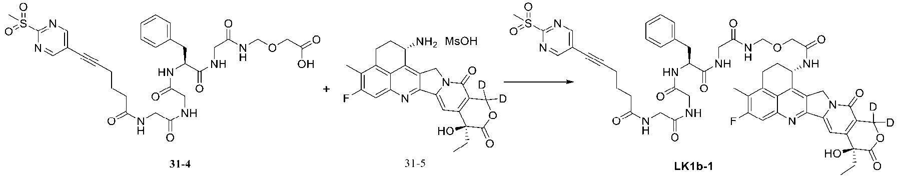

Compound 31-4 (8.3 g, 1.4 eq), compound 31-5 (4.9 g, 1.0 eq), and 175 mL of DMF were added to a 250 mL single-necked flask, followed by stirring at room temperature. Triethylamine (1.9 mL, 1.6 eq) and DMTMM (3.87 g, 1.5 eq) were then added. The mixture was stirred at room temperature for 1 hour, and the reaction was monitored by HPLC until completion. The reaction mixture was added to 500 mL of water, filtered, and dried to obtain 12 g of crude product. The crude product was purified by column chromatography to obtain 8.9 g of compound LK1b-1, with a yield of 85%.

MS (ESI) m/z: 1093.4 [M+H]⁺.

¹H NMR (400 MHz, DMSO-d6) δ 9.10 (s, 2H), 8.64 (t, *J =* 6.6 Hz, 1H), 8.51 (d, *J* = 8.8 Hz, 1H), 8.31 (t, *J =* 5.7 Hz, 1H), 8.26 - 8.00 (m, 3H), 7.77 (d, *J =* 10.9 Hz, 1H), 7.36 - 7.10 (m, 6H), 6.52 (s, 1H), 5.67 - 5.52 (m, 1H), 5.19 (s, 2H), 4.64 (d, *J* = 6.5 Hz, 2H), 4.47 (dt, *J* = 12.9, 6.7 Hz, 1H), 4.02 (s, 2H), 3.77 - 3.68 (m, 4H), 3.63 - 3.57 (m, 1H), 3.40 (s, 3H), 3.17 (td, *J* = 12.8, 11.5, 6.7 Hz, 2H), 3.02 (dd, *J* = 13.7, 4.2 Hz, 1H), 2.77 (dd, *J* = 13.7, 9.7 Hz, 1H), 2.38 (s, 3H), 2.32 (t, *J* = 7.3 Hz, 2H), 2.17 (dd, *J =* 13.4, 6.8 Hz, 2H), 2.08 (s, 1H), 1.84 (dq, *J* = 18.9, 7.0 Hz, 4H), 1.28 - 1.22 (m, 2H), 0.87 (t, *J =* 7.2 Hz, 3H).

### Example 32 General Preparation Method of Antibody-Drug Conjugates

The antibody was exchanged into a 10 mM phosphate buffered saline at pH 7.0 using desalting chromatography or ultrafiltration buffer exchange, and the antibody concentration was determined by the UV method. A 10 mM TCEP solution (6-10 equivalents) was added to the antibody solution, mixed well, and reduced at 37°C for 3 hours. Subsequently, the reaction mixture was cooled to 15-25°C using a water bath, and a 10 mM solution of the drug-linker conjugate in dimethyl sulfoxide (10-20 equivalents) was added. After mixing well, the reaction was allowed to proceed for 1.5-2 hours. After the coupling reaction was completed, a 100 mM N-acetyl-cysteine solution was added at a quenching ratio of 20:1 (quenching agent: antibody), shaken well, and quenched at room temperature for 20 min to terminate the coupling reaction. The ADC was exchanged into 20 mM His-HAc buffer at pH 5.5 using desalting chromatography or ultrafiltration buffer exchange, and then filtered through a 0.22 µm PES filter to obtain the ADC stock solution, which was aliquoted according to experimental requirements and stored frozen at -20°C or -40°C. Depending on the specific properties of the ADC, the antibody concentration was determined using UV or Lowry method. The absorbance values were measured respectively at 280 nm and 370 nm using the UV method, and the DAR value n was calculated. The prepared compounds and their DAR results are shown in the table below.

| **ADC molecule** | **Payload-linker** | **Antibody** | **DAR value n** |
|---|---|---|---|
| ADC1-LK1-Dxd | LK1-Dxd | Trastuzumab | 7.71 |
| ADC1-LK1-1 | LK1-1 | Trastuzumab | 7.55 |
| ADC1-LK1a-1 | LK1a-1 | Trastuzumab | 7.73 |
| ADC1-LK1b-1 | LK1b-1 | Trastuzumab | 7.54 |
| ADC1-LK1c-1 | LK1c-1 | Trastuzumab | 7.75 |
| ADC1-LK1d-1 | LK1d-1 | Trastuzumab | 7.78 |
| ADC1-LK1-2 | LK1-2 | Trastuzumab | 7.65 |
| ADC1-LK1-3 | LK1-3 | Trastuzumab | 7.43 |
| ADC1-LK1-4 | LK1-4 | Trastuzumab | 7.39 |
| ADC1-LK1-5 | LK1-5 | Trastuzumab | 7.75 |

The antibody-drug conjugates and payload-linker conjugates provided in the present disclosure could be prepared by referring to the relevant preparation methods documented in WO2014057687A, WO2020063676A, WO2022068878, and other literatures, based on the preparation methods of the related compounds described herein.

### Biological activity test

### 1. In vitro anti-tumor activity detection of the compounds of the present disclosure

### (1) In vitro anti-tumor activity detection of the payload compounds of the present disclosure

Experimental objective: To detect the *in vitro* inhibitory activity of the payload compounds of the present disclosure against NCI-N87 (human gastric cancer cells), Calu-3 (human lung adenocarcinoma cells), MDA-MB-453 (human breast cancer cells), KPL-4 (human breast cancer cells), or MDA-MB-468 (human breast cancer cells).

Tumor cells in the logarithmic growth phase, including NCI-N87 (source: ATCC, catalog number: CRL-5822), Calu-3 (source: ATCC, catalog number: HTB-55), MDA-MB-453 (source: ATCC, catalog number: HTB-131), KPL-4 (source: Nanjing Cobioer Biosciences, catalog number: CBP60379), or MDA-MB-468 (source: ATCC, catalog number: HTB-132), were seeded into cell plates at a density of 5,000 cells/well. The cell plates were then incubated in a 37°C, 5% CO₂ cell culture incubator for 12-16 hours. 100 µL of sample (starting at 10 µM, 4-fold dilution, 10 concentrations) was added to each well. After gentle shaking, the plates were placed in the incubator for further incubation. After 48 hours of incubation, 70 µL of CellTiter-Glo^{™} (Promega, catalog number: G7572) working solution was added, gently shaken to lyse the cells, and the plate was read on a microplate reader. The cell proliferation inhibition rate was calculated using the following formula: cell proliferation inhibition rate = (1 - sample well/control well) × 100%. GraphPad Prism 8.0 software was used to plot the Log value of sample concentration as the x-axis and Cytotoxicity% as the y-axis. The data were analyzed by Nonlinear regression (curve fit) to obtain the IC₅₀ values for each sample, with specific results shown in Tables 1-1 and 1-2.

**Table 1-1. In vitro activity test results of the payload compounds of the present disclosure**

| **Payload compound** | **NCI-N87** | **Calu-3** | **MDA-MB-453** | **MDA-MB-468** |
|---|---|---|---|---|
| | **IC₅₀ (nM)** | **IC₅₀ (nM)** | **IC₅₀ (nM)** | **IC₅₀ (nM)** |
| 2 | 7.17 | 27.15 | 114.5 | 0.98 |
| 3 | 4.37 | 17.13 | 40.27 | 0.97 |
| 4 | 4.06 | 19.32 | 55.34 | 0.88 |
| 5 | 9.18 | 32.9 | 94.6 | 1.3 |
| 6 | 13.35 | 46.63 | 121.1 | 3.11 |

**Table 1-2. In vitro activity test results of the payload compounds of the present disclosure**

| **Payload compound** | NCI-N87 | KPL-4 | MDA-MB-468 |
|---|---|---|---|
| | IC₅₀ (nM) | IC₅₀ (nM) | IC₅₀ (nM) |
| 12 | 7.29 | 7.05 | 1.7 |

The experimental results showed that the payload compounds of the present disclosure exhibited significant proliferation inhibitory activity against NCI-N87, Calu-3, MDA-MB-453, KPL-4, or MDA-MB-468.

### (2) In vitro anti-tumor activity detection of the ADC compounds of the present disclosure

Experimental objective: To detect the *in vitro* inhibitory activity of the ADC compounds of the present disclosure against NCI-N87 (human gastric cancer cells), KPL-4 (human breast cancer cells), or MDA-MB-468 (human breast cancer cells).

Tumor cells NCI-N87, KPL-4 (source: ATCC, catalog number: ATCC-477), or MDA-MB-468 in the logarithmic growth phase were taken and added to the cell plate at a density of 2000 cells/well. The cell plate was placed in a 37°C, 5% CO₂ cell culture incubator and incubated for 12-16 hours. 100 µL of sample (starting at 100 µg/mL, 5-fold dilution, 9 concentrations) was added to each well. After gentle shaking well, the plates were placed in the incubator for further incubation. After 144 hours of incubation, 70 µL of CellTiter-Glo^{™} (Promega, catalog number: G7572) working solution was added, gently shaken well to lyse the cells, and the plate was read on a microplate reader. The cell proliferation inhibition rate was calculated using the following formula: cell proliferation inhibition rate = (1 - sample well/control well) × 100%. GraphPad Prism 8.0 software was used to plot the Log value of sample concentration as the x-axis and Cytotoxicity% as the y-axis. The data were analyzed by Nonlinear regression (curve fit) to obtain the IC₅₀ values for each test sample. The specific results are shown in Table 1-3.

**Table 1-3. In vitro anti-tumor activity detection of the ADC compounds of the present disclosure**

| **ADC compound** | KPL-4 (HER2+++) | | NCI-N87 (HER2+++) | | MDA-MB-468 (HER2-) | |
|---|---|---|---|---|---|---|
| | IC₅₀ (µg/mL) | Max inhibition | IC₅₀ (µg/mL) | Max inhibition | IC₅₀ (µg/mL) | Max inhibition |
| ADC1-LK1-1 | 0.096 | 90.39% | 0.104 | 90.88% | 10.71 | 101% |
| ADC1-LK1a-1 | 0.0921 | 98.30% | 0.0282 | 91.70% | 2.78 | 95.90% |
| ADC1-LK1b-1 | 0.0794 | 95.90% | 0.0218 | 85.70% | 22 | 83.10% |
| ADC1-LK1-2 | 0.0951 | 99.40% | 0.04522 | 97.70% | 5.722 | 97.03% |
| ADC1-LK1-3 | 0.0898 | 98.40% | 0.1126 | 93.49% | 10.58 | 90.84% |
| ADC1-LK1-4 | 0.1487 | 96.06% | 0.0409 | 93.21% | 10.26 | 95.74% |
| ADC1--LK1-5 | 0.0788 | 96.52% | 0.02541 | 93.88% | 9.79 | 96.20% |

Conclusion: The ADC compounds of the present disclosure exhibited significant proliferation inhibitory activity against HER2-positive cells KPL-4 and NCI-N87; meanwhile, they exhibited weak proliferation inhibitory activity against HER2-negative cells MDA-MB-468, showing good selectivity.

### 2. Pharmacokinetics of the payload compounds of the present disclosure in mice

Experimental objective: To evaluate the pharmacokinetic characteristics of the payload compounds of the present disclosure in mice.

Balb/c mice were taken, with 12 mice per group (half male and half female), randomly grouped, and administered a single injection of the sample (Formulation A: 5% DMSO + 95% (15% sulfobutyl-β-cyclodextrin-normal saline), Formulation B: 5% DMSO-10% Solutol HS 15-85% normal saline, diluted according to the dose). Blood samples were collected at crossover time points, including before administration and at 5 min, 30 min, 1 h, 2 h, 4 h, 6 h, 8 h, 10 h, and 24 h after administration. Plasma was collected, and the sample concentration in plasma was detected by LC-MS. The pharmacokinetic parameters were calculated and shown in Table 2-1.

**Table 2-1. PK parameters of the payload compounds of the present disclosure in mice**

| Payload **compound** | **Mouse PK (IV, 3 mpk)** | | |
|---|---|---|---|
| | **T_{1/2} (h)** | **Cₘₐₓ (ng/mL)** | **Formulation** |
| Dxd | 1.17 | 521 | A |
| 2 | 0.455 | 449 | A |
| 3 | 0.607 | 4305 | B |
| 5 | 0.674 | 3515 | B |
| 6 | 0.679 | 2582.5 | B |

The experimental results showed that the payload compounds of the present disclosure exhibited a short *in vivo* half-life in mice. When these compounds were used as payloads for ADCs, the ADCs could be rapidly cleared upon payload detachment *in vivo,* thereby exhibiting superior *in vivo* safety.

### 3. Pharmacokinetic study - rats

### (1) Pharmacokinetics of the payload compounds of the present disclosure in rats

Experimental objective: To evaluate the pharmacokinetic characteristics of the payload compounds of the present disclosure in rats.

SD rats (purchased from Beijing Vital River Laboratory Animal Technology Co., Ltd.) were used, with 6 rats per group (half male and half female), randomly grouped. A single injection of the sample (Formulation A: 5% DMSO + 95% (15% sulfobutyl-β-cyclodextrin-normal saline), diluted according to the dose) was administered. Blood samples were collected at crossover time points: before administration and at 5 min, 30 min, 1 h, 2 h, 4 h, 6 h, 8 h, 10 h, and 24 h after administration. Plasma was collected, and the sample concentration in plasma was detected by LC-MS. Pharmacokinetic parameters were calculated, and the results are shown in Table 3-1.

**Table 3-1. PK parameters of the payload compounds of the present disclosure in rats**

| **Payload compound** | **Rat PK (IV, 3 mpk)** | | | | |
|---|---|---|---|---|---|
| | **T_{1/2} (h)** | **Cmax (ng/mL)** | **AUCinf (h*ng/mL)** | **Cl (mL/h/kg)** | **Formulation** |
| Dxd | 1.172 | 621.2 | 352.1 | 8629.5 | A |
| 2 | 0.661 | 406.2 | 217.7 | 14072.1 | A |

The experimental results showed that the payload compounds of the present disclosure also exhibited a short *in vivo* half-life and rapid *in vivo* clearance rate in rats.

### (2) Pharmacokinetic study of the ADC compounds of the present disclosure in rats

Experimental objective: To evaluate the pharmacokinetic characteristics of the ADC compounds of the present disclosure in SD rats.

SD rats (purchased from Beijing Vital River Laboratory Animal Technology Co., Ltd.) were used, with 4 rats per group (half male and half female), randomly grouped. A single injection of the sample (prepared with an appropriate volume of normal saline for administration) was administered. Blood samples were collected before administration and at 5 min, 1 h, 4 h, 8 h, 24 h, 48 h, 72 h, 120 h, 168 h, 336 h, 504 h, and 672 h after administration. The concentrations of total antibody and conjugated form in plasma samples were detected by ELISA; the concentration of free payload in plasma samples was detected by LC-MS/MS. Pharmacokinetic parameters for different detection forms were calculated using WinNonlin software. Specific results are shown in Table 3-2, Table 3-3, and Table 3-4.

**Table 3-2. Pharmacokinetic characteristics of the ADC compounds of the present disclosure in rats**

| **ADC compound** | **Rat PK (IV, 30 mpk)** | | | |
|---|---|---|---|---|
| | **T_{1/2} (h)** | **Cₘₐₓ (µg/mL)** | **AUCₗₐₛₜ (h*µg/mL)** | **Cl (mL/h/kg)** |
| ADC1-LK1-Dxd | 158.1 | 598.6 | 34615.7 | 0.789 |
| ADC1-LK1-1 | 146.8 | 698.6 | 40413.6 | 0.683 |

**Table 3-3. Pharmacokinetic characteristics of the ADC compounds of the present disclosure in rats**

| **ADC compound** | **Rat PK (IV, 20 mpk)** | | | |
|---|---|---|---|---|
| | **T_{1/2} (h)** | **Cₘₐₓ (µg/mL)** | **AUCₗₐₛₜ (h*µg/mL)** | **Cl (mL/h/kg)** |
| ADC1-LK1-Dxd | 224.4 | 401.4 | 25737.2 | 0.648 |
| ADC1-LK1-2 | 204.9 | 504.2 | 28633.5 | 0.608 |
| ADC1-LK1-4 | 234.1 | 595.2 | 36566.2 | 0.455 |
| ADC1-LK1-5 | 204 | 553.1 | 32529.6 | 0.528 |

**Table 3-4. Pharmacokinetic characteristics of the ADC compounds of the present disclosure in rats**

| **ADC compound** | **Rat PK (IV, 30 mpk)** | | | |
|---|---|---|---|---|
| | **T_{1/2} (h)** | **Cₘₐₓ (µg/mL)** | **AUCₗₐₛₜ (h*µg/mL)** | **Cl (mL/h/kg)** |
| ADC1-LK1-Dxd | 167.1 | 687.8 | 39066.6 | 0.693 |
| ADC1-LK1a-1 | 140.3 | 749.1 | 42117.7 | 0.661 |
| ADC1-LK1b-1 | 281.6 | 591.9 | 58148.5 | 0.382 |
| ADC1-LK1-2 | 173.8 | 722.8 | 43748.3 | 0.609 |
| ADC1-LK1-4 | 286.6 | 709.5 | 46148.8 | 0.495 |

The experimental results showed that the ADC compounds of the present disclosure exhibited high exposure levels and low *in vivo* clearance rates in rats.

### 4. In vitro plasma stability

### (1) In vitro plasma stability of the payload compounds of the present disclosure

398 µL of plasma was taken and preheated to 37°C, then co-incubated for 15 minutes. 2 µL of the sample solution was added to the plasma to prepare a final sample concentration of 5 µM. The above solution was co-incubated at 37°C, and 50 µL aliquots were taken at 0, 15, 30, 60, and 120 minutes, followed by the addition of 450 µL of ice-cold acetonitrile (containing the internal standard). The solution was vortexed for 10 minutes, centrifuged, and the remaining content of the parent substrate was detected by the LC-MS/MS method. For specific results, refer to Table 4-1.

**Table 4-1. Plasma stability of the payload compounds of the present disclosure**

| Payload compound | Residual content of parent payload at 120 minutes (relative to 0 minutes) | |
|---|---|---|
| | Human plasma | CD1 mouse plasma |
| Dxd | 87.57% | 95.42% |
| 2 | 111.04% | 102.54% |
| 3 | 105.54% | 104.97% |
| 5 | 107.73% | 96.02% |
| 6 | 94.89% | 105.93% |

The experimental results showed that the payload compounds of the present disclosure exhibited good plasma stability.

### (2) Human plasma stability of the ADC compounds of the present disclosure

Experimental objective: To evaluate the stability of the ADC compounds of the present disclosure in human plasma.

ADC samples, healthy human plasma, and 1% BSA (prepared in-house using BSA powder purchased from Sangon Biotech (Shanghai) Co., Ltd.) were separately filtered and sterilized using 0.22 µm filters. The ADC sample was added to the sterile plasma at a final concentration of 100 µg/mL and incubated in a 37°C cell culture incubator. The day of incubation was recorded as day 0, and the sample was retrieved on day 21 for free payload analysis.

Free payload detection method: 15 µL of the sample and 60 µL of acetonitrile were added to a centrifuge tube, vortex-mixed, and centrifuged at 13000 rpm for 15 minutes. In a new centrifuge tube, 30 µL of the supernatant and 30 µL of deionized water were added, vortex-mixed, and 2 µL was subjected to LC-MS/MS analysis (Shimadzu LC-MS 8050 triple quadrupole liquid chromatography-mass spectrometer). The specific experimental results are shown in Table 4-2.

**Table 4-2. Free payload release rate of the ADC compounds of the present disclosure in human plasma on day 21**

| **ADC compound** | **Free payload release rate (%)** |
|---|---|
| | **Human plasma** |
| ADC1-LK1-Dxd | 0.98% |
| ADC1-LK1-2 | 0.25% |
| ADC1-LK1-4 | 0.18% |
| ADC1-LK1-5 | 0.24% |
| ADC1-LK1b-1 | 0.18% |

The results showed that the ADC compounds of the present disclosure exhibited good stability in human plasma.

### 5. Efficacy evaluation of the ADC compounds of the present disclosure in NCI-N87 tumor-bearing mice

BALB/c Nude mice (purchased from Beijing Vital River) were used as test animals to evaluate the efficacy of anti-Her2-ADC administered via tail vein injection on human gastric cancer cell NCI-N87 xenograft nude mice.

NCI-N87 cells (source: ATCC, catalog number: CRL-5822) (5 × 10⁶/mouse, with 50% artificial basement membrane) were subcutaneously inoculated into the right flank of the mice. After 11 days of tumor growth, when the tumor volume reached 160.1 ± 24.7 mm³, the animals were randomly grouped (dl), with 5 mice per group.

A single dose was administered via tail vein injection. Tumor volume and body weight were measured twice a week, and the data were recorded. The tumor growth inhibition rate (%) = (Crtv - Trtv) / Crtv (%), wherein Crtv and Trtv represent the relative tumor volumes of the blank control group (Vehicle, PBS) and the experimental group, respectively, at the end of the experiment. The specific experimental results at the end of the experiment on day 28 are shown in 5-1.

**Table 5-1. In vivo efficacy evaluation of the ADC compounds of the present disclosure**

| ADC compound | Dosage | Tumor growth inhibition rate |
|---|---|---|
| ADC1-LK1-1 | 1 mpk | 56.2% |
| ADC1-LK1-2 | 1 mpk | 55.6% |
| ADC1-LK1b-1 | 1 mpk | 78.0% |
| ADC1-LK1-Dxd | 1 mpk | 37.3% |

### 6. Repeated dose toxicity experiments in rats

### (1) Repeated dose toxicity experiments of the payload compounds of the present disclosure in rats

Test sample preparation: The test sample was weighed according to the predetermined weight into a glass vial. First, 1 mL of DMSO was added and mixed until the test sample was dissolved, followed by the addition of 1 mL of Solutol HS-15. After mixing thoroughly, 8 mL of sodium chloride injection was added and vortex-mixed to obtain a clear and transparent solution.

SD rats (purchased from Beijing Vital River) were used as test animals. Compound 2 and the positive control Dxd were administered via tail vein injection for 7 consecutive days, with both compound 2 and the positive control Dxd set at two doses of 0.3 mg/kg and 1.0 mg/kg. During the acclimation period, one cage-side observation was conducted daily; during the dosing period, all animals were subjected to clinical observations twice daily (morning and afternoon). Reactions in various aspects, including the skin, fur, eyes (sclera), ears, nose, oral cavity, chest, abdomen, urogenital area, limbs, as well as respiration, movement, urination, defecation (including urine and feces color), and behavioral changes, were recorded. Daily weight data of the main test group animals were documented. For animals scheduled for dissection, terminal body weight (fasted) was measured prior to dissection. The food intake of the test animals was also recorded. The specific results are shown in Table 6-1.

**Table 6-1. Results of repeated dose toxicity experiments of the payload compounds of the present disclosure in rats**

| **Observation parameters** | **Test results** | |
|---|---|---|
| | **Control Dxd group** | **Compound 2 group** |
| **Clinical observations** | Main experimental group + TK group: | Main experimental group + TK group: |
| | Dxd: 0.3 mpk dose group: 31% (5/16) of animals showed immediate ataxia after administration, wherein 40% (2/5) of rats died during recovery, while others gradually recovered; | compound 2: 0.3 mpk dose group: 13% (2/16) of animals showed immediate ataxia after administration, followed by gradual recovery; |
| | | compound 2: 1 mpk dose group: 25% (4/16) of animals showed immediate ataxia after administration, followed by gradual recovery; |
| | Dxd: 1 mpk dose group: 31% (5/16) of animals showed immediate ataxia after administration, wherein 40% (2/5) of rats died during recovery, while others gradually recovered; | |
| **Body weight and food consumption** | Slow/decreased body weight gain; decreased food consumption; | At the same dose, the effects of compound 2 and Dxd on rat body weight and food consumption were essentially comparable; |
| | | for ♀ rats, at the same dose, the average food consumption of the compound 2 group was higher than that of the control group; |

wherein ♂ represents male, and ♀ represents female.

At the end of the administration, gross dissection was performed on D8 after the administration. Focal/punctate dark red discoloration was observed in the lungs of some deceased rats in the Dxd control group, while no such pathological changes were found in any dose group of payload compound 2.

The above experimental data showed that payload compound 2 of the present disclosure exhibited lower *in vivo* toxicity and superior *in vivo* safety compared to the positive control Dxd.

### (2) Repeated dose toxicity experiments of the ADC compounds of the present disclosure in rats

SD rats were used as test animals to study the toxic reactions of ADC compounds of the present disclosure after repeated administration to SD rats (5 females and 5 males per group). The dosing regimen was as follows: intravenous injection at a dose of 200 mg/kg, administered once every three weeks for a total of three doses. On the dosing day, the animals' conditions were observed, including whether they were dead, moribund, as well as their feces, appearance, respiration, fur, activity status, *etc.* Body weight and food intake were measured twice weekly after administration. Blood samples of approximately 0.2 mL were collected from the jugular venous sinus on days 7, 16, 28, and 50 after administration into anticoagulant tubes containing anticoagulant (EDTA-K₂). All animals scheduled for necropsy were fasted for approximately 12 hours on the night before necropsy on day 50. Moribund animals were euthanized, and approximately 2 mL of blood was collected from the abdominal aorta during necropsy into anticoagulant tubes containing anticoagulant (EDTA-K₂). Hematological parameters were detected using a Sysmex XN-1000 hematology analyzer. Changes in some hematological parameters measured on day 7 are shown in Table 6-2.

**Table 6-2. Changes in hematological parameters on day 7**

| Hematological parameters | Animal groups | ADC1-LK1b-1 | ADC1-LK1-Dxd |
|---|---|---|---|
| WBC | Male | 43%↓ | 59%↓ |
| WBC | Female | 31%↓ | 45%↓ |
| #LYMPH | Male | 47%↓ | 63%↓ |
| #LYMPH | Female | 32%↓ | 48%↓ |
| #MONO | Male | 24%↓ | 37%↓ |
| #MONO | Female | / | 33%↓ |
| #NEUT | Male | / | 37%↓ |
| #EO | Male | 46%↓ | 60%↓ |

Note: ↓ indicates the degree of reduction relative to the control group rats; / indicates no significant change relative to the control group rats.

The experimental results showed that, at the same dose, the ADC1-LK1b-1 dose group exhibited lower reductions in WBC (white blood cell count), #LYMPH (lymphocyte percentage), #MONO (monocyte count), #NEUT (neutrophil count), and #EO (eosinophil percentage) compared to ADC1-LK1-Dxd, indicating that the ADC compounds of the present disclosure had lower hematological toxicity and superior safety.

In the present disclosure, the comparative compound LK1-Dxd refers to the following compound, which can be prepared according to the published literature WO2014057687A:

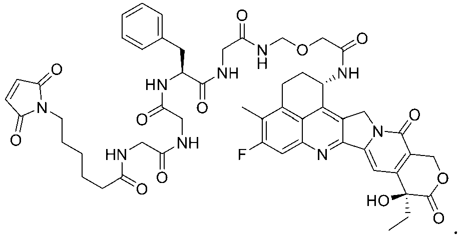

## Claims

1. A compound of Formula I, a pharmaceutically acceptable salt thereof, or a stereoisomer thereof: 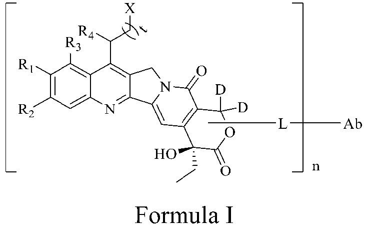
wherein Ab represents an antibody or an antigen-binding fragment; L represents a linker connecting Ab and a payload drug molecule; n is selected from the group consisting of 1-12;
R₁ is H, halogen, OH, SH, NH₂, C₁₋₄ alkyl, C₁₋₄ haloalkyl, or C₁₋₄ alkoxy;
R₂ is H, halogen, C₁₋₄ alkyl, or C₁₋₄ alkoxy;
or R₁ and R₂ are cyclized to form -O-(CH₂)ₘ-O-, wherein m is 1, 2, or 3;
R₃ and R₄ are each independently H, C₁₋₄ alkyl, C₁₋₄ alkoxy, C₁₋₄ haloalkyl, or C₁₋₄ haloalkoxy;
or R₃ and R₄ are cyclized to form -(CH₂)ₖ-, wherein k is 1, 2, 3, or 4;
X is H, OH, HO-CH(R₅)-(CH₂)ₚ-CO-NH-, or -N(R₆)(R₇), wherein p is 0, 1, or 2;
R₅ is H, C₁₋₄ alkyl, C₁₋₄ haloalkyl, 3- to 6-membered cycloalkyl, or 3- to 6-membered heterocycloalkyl;
R₆ is H, C₁₋₄ alkyl, or C₁₋₄ haloalkyl;
R₇ is H or R₈-S(O)₂-;
R₈ is C₁₋₄ alkyl; and
t is 0, 1, 2, 3, 4, or 5.

2. The compound, the pharmaceutically acceptable salt thereof, or the stereoisomer thereof according to claim 1, wherein R₁ is C₁₋₄ alkyl, and R₂ is halogen; or R₁ is methyl, and R₂ is F; or R₁ is H, and R₂ is H; or R₁ and R₂ are cyclized to form -O-CH₂-O-; or R₁ is NH₂, and R₂ is H or halogen; or R₁ is NH₂, and R₂ is H or F.

3. The compound, the pharmaceutically acceptable salt thereof, or the stereoisomer thereof according to claim 1 or 2, wherein R₃ is H, and R₄ is H; or R₃ is H, and R₄ is C₁₋₄ alkyl; or R₃ is H, and R₄ is methyl; or R₃ and R₄ are cyclized to form -CH₂-CH₂-.

4. The compound, the pharmaceutically acceptable salt thereof, or the stereoisomer thereof according to any one of claims 1-3, wherein X is HO-CH(R₅)-(CH₂)ₚ-CO-NH-, wherein p is 0, 1, or 2; or X is OH or NH₂; or X is H, and t is 0; or X is -N(R₆)(R₇).

5. The compound, the pharmaceutically acceptable salt thereof, or the stereoisomer thereof according to any one of claims 1-4, wherein n is 4-9; or n is 6-8, 7-8, 7.4-8.0, or 7.5-8.5; or n is 6, 7, or 8; or n is 7-8, 7.3-7.9, 7.4-7.8, 7.5-7.7, 7.5-7.6, 7.5-7.8, or 7.4-7.7.

6. The compound, the pharmaceutically acceptable salt thereof, or the stereoisomer thereof according to any one of claims 1-5, wherein the compound, the pharmaceutically acceptable salt thereof, or the stereoisomer thereof is a compound of Formula Ia, a pharmaceutically acceptable salt thereof, or a stereoisomer thereof: 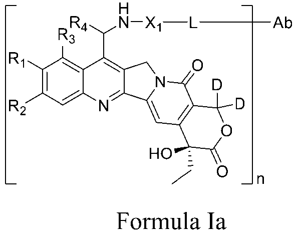 wherein R₁, R₂, R₃, and R₄ are as defined in any one of claims 1-3; X₁ is a chemical bond or -O-CH(R₅)-(CH₂)ₚ-CO-, wherein the -CO- end is connected to -NH-; p is 0, 1, or 2; R₅ is H, C₁₋₄ alkyl, 3- to 6-membered cycloalkyl, or 3- to 6-membered heterocycloalkyl.

7. The compound, the pharmaceutically acceptable salt thereof, or the stereoisomer thereof according to claim 6, wherein R₁ is methyl or methoxy; or R₂ is F or Cl; or R₁ is methyl, and R₂ is F; or R₃ and R₄ are cyclized to form -(CH₂)ₖ-, wherein k is 2; or R₃ is H; and R₄ is H.

8. The compound, the pharmaceutically acceptable salt thereof, or the stereoisomer thereof according to any one of claims 6-7, wherein X₁ is -O-CH(R₅)-(CH₂)ₚ-CO-, p is 0 or 1, R₅ is H, C₁₋₄ alkyl, or 3- to 6-membered cycloalkyl; or X₁ is -O-CH(R₅)-CO-, R₅ is H or 3- to 6-membered cycloalkyl; or X₁ is -O-CH(R₅)-CO-, and R₅ is H or cyclopropyl; or X₁ is -O-CH(R₅)-CH₂-CO-; R₅ is C₁₋₄ alkyl; or X₁ is -O-CH(R₅)-CH₂-CO-, R₅ is methyl; or X₁ is the following group: 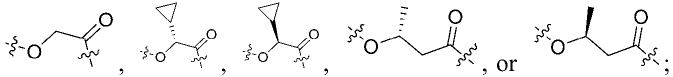 wherein the -CO- end of X₁ is connected to NH.

9. The compound, the pharmaceutically acceptable salt thereof, or the stereoisomer thereof according to any one of claims 1-8, wherein the compound, the pharmaceutically acceptable salt thereof, or the stereoisomer thereof is a compound of Formula Ia-1 or Formula Ia-2, a pharmaceutically acceptable salt thereof, or a stereoisomer thereof; 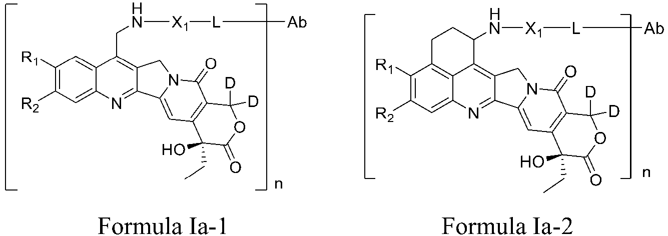 wherein Ab, L, R₁, R₂, X₁, and n are as defined in any one of claims 1-8.

10. The compound, the pharmaceutically acceptable salt thereof, or the stereoisomer thereof according to claim 1, wherein 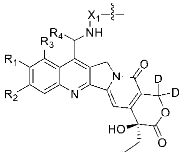 is the following compound moiety: 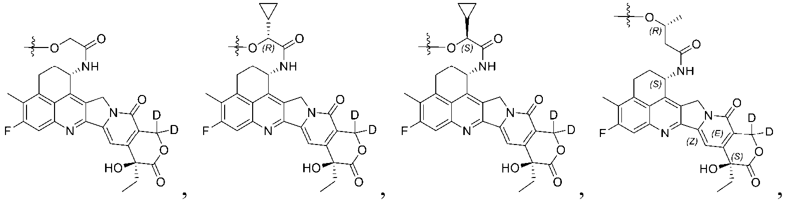 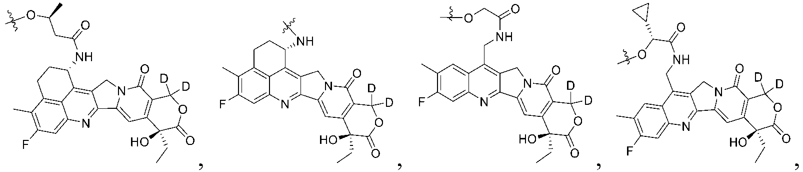 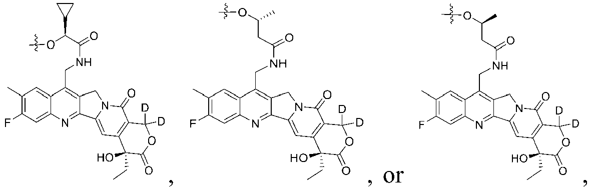 wherein " 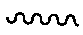 " indicates that this position is connected to the linker L via a chemical bond.

11. The compound, the pharmaceutically acceptable salt thereof, or the stereoisomer thereof according to any one of claims 1-5, wherein the compound, the pharmaceutically acceptable salt thereof, or the stereoisomer thereof is a compound of Formula Ib, a pharmaceutically acceptable salt thereof, or a stereoisomer thereof: 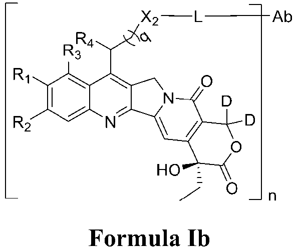
wherein Ab, L, and n are as defined in claim 1;
R₁ is H, OH, halogen, NH₂, C₁₋₄ alkyl, C₁₋₄ haloalkyl, or C₁₋₄ alkoxy;
R₂ is H, halogen, C₁₋₄ alkyl, or C₁₋₄ alkoxy;
or R₁ and R₂ are cyclized to form -O-(CH₂)ₘ-O-, wherein m is 1, 2, or 3;
R₃ and R₄ are each independently H, C₁₋₄ alkyl, or C₁₋₄ alkoxy;
or R₃ and R₄ are cyclized to form -(CH₂)ₖ-, wherein k is 1, 2, or 3;
X₂ is O or -N(R₆)-;
R₆ is H or C₁₋₄ alkyl;
q is 0, 1, 2, 3, or 4.

12. The compound, the pharmaceutically acceptable salt thereof, or the stereoisomer thereof according to claim 11, wherein R₁ is methyl, and R₂ is H, Cl, or F; or R₁ and R₂ are cyclized to form -O-(CH₂)ₘ-O-, wherein m is 1 or 2.

13. The compound, the pharmaceutically acceptable salt thereof, or the stereoisomer thereof according to claim 11 or 12, wherein R₃ and R₄ are both H; or R₃ is H, and R₄ is methyl.

14. The compound, the pharmaceutically acceptable salt thereof, or the stereoisomer thereof according to any one of claims 11-13, wherein X₂ is -O- or -NH-; or X₂ is -O- or -NH-, and q is 0, 1, 2, 3, or 4; or X₂ is -O- or -NH-, and q is 1, 2, 3, or 4.

15. The compound, the pharmaceutically acceptable salt thereof, or the stereoisomer thereof according to any one of claims 11-14, wherein the compound of formula Ib is a compound of formula Ib-1: 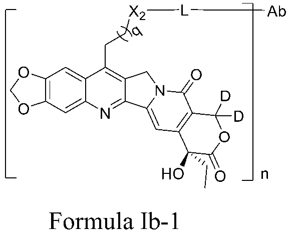 wherein Ab, L, and n are as defined in claim 1, and X₂ and q are as defined in any one of claims 12-15.

16. The compound, the pharmaceutically acceptable salt thereof, or the stereoisomer thereof according to any one of claims 11-15, wherein 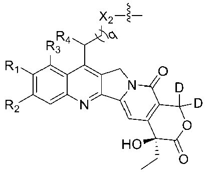 is the following compound moiety; 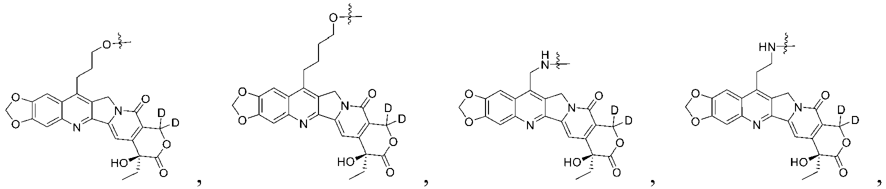 or 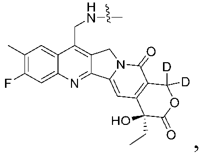 wherein " 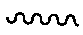 " indicates that this position is connected to the linker L via a chemical bond.

17. The compound, the pharmaceutically acceptable salt thereof, or the stereoisomer thereof according to any one of claims 1-5, wherein the compound, the pharmaceutically acceptable salt thereof, or the stereoisomer thereof is a compound of Formula Ic, a pharmaceutically acceptable salt thereof, or a stereoisomer thereof: 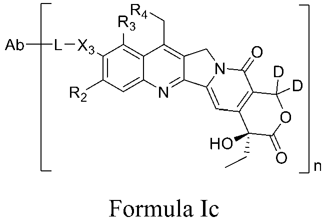 wherein X₃ is NH, O, or S, or X₃ is NH or O; R₂, R₃, and R₄ are as defined in any one of claims 1-3; and Ab, L, and n are as defined in claim 1.

18. The compound, the pharmaceutically acceptable salt thereof, or the stereoisomer thereof according to claim 17, wherein R₂ is H or F; or R₃ is H, and R₄ is methyl; or R₃ and R₄ are cyclized to form -(CH₂)ₖ-, wherein k is 1, 2, or 3; or R₃ and R₄ are cyclized to form -(CH₂)₂-; or X₃ is NH; or X₃ is O.

19. The compound, the pharmaceutically acceptable salt thereof, or the stereoisomer thereof according to claim 17, wherein 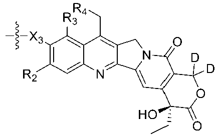 is the following compound moiety:

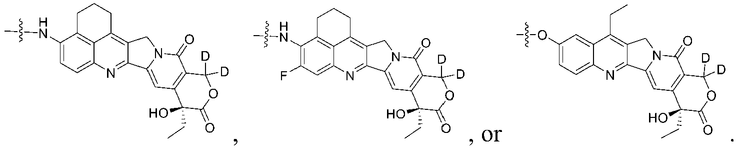

20. The compound, the pharmaceutically acceptable salt thereof, or the stereoisomer thereof according to any one of claims 1-5, wherein the compound, the pharmaceutically acceptable salt thereof, or the stereoisomer thereof is a compound of Formula Id or a pharmaceutically acceptable salt thereof: 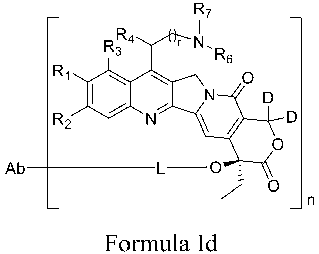 wherein Ab, L, n, R₆, and R₇ are as defined in claim 1; R₁, R₂, R₃, and R₄ are as defined in any one of claims 1-3, and r is 0, 1, 2, or 3.

21. The compound, the pharmaceutically acceptable salt thereof, or the stereoisomer thereof according to claim 20, wherein R₁ and R₂ are both H; or R₃ and R₄ are both H; or r is 1; or R₆ is methyl, ethyl, or isopropyl; or R₆ is isopropyl; or R₇ is H; or R₇ is R₈-S(O)₂-, wherein R₈ is methyl or ethyl, or R₈ is methyl.

22. The compound, the pharmaceutically acceptable salt thereof, or the stereoisomer thereof according to claim 20, wherein 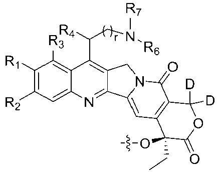 is the following compound moiety:

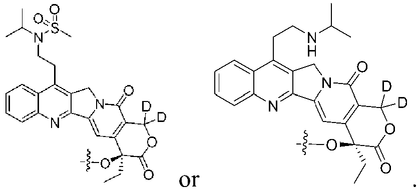

23. The compound, the pharmaceutically acceptable salt thereof, or the stereoisomer thereof according to any one of claims 1-22, wherein the Ab is an anti-Her2 antibody, an anti-Trop2 antibody, or an anti-Claudin18.2 antibody.

24. The compound, the pharmaceutically acceptable salt thereof, or the stereoisomer thereof according to any one of claims 1-22, wherein the Ab is an anti-Her2 antibody, having HCDR1, HCDR2, and HCDR3 as set forth in SEQ ID NO: 1, SEQ ID NO: 2, and SEQ ID NO: 3, respectively, and LCDR1, LCDR2, and LCDR3 as set forth in SEQ ID NO: 6, SEQ ID NO: 7, and SEQ ID NO: 8, respectively.

25. The compound, the pharmaceutically acceptable salt thereof, or the stereoisomer thereof according to any one of claims 1-24, wherein the Ab is an anti-Her2 antibody, having a heavy chain variable region as set forth in SEQ ID NO: 4 and a light chain variable region as set forth in SEQ ID NO: 9.

26. The compound, the pharmaceutically acceptable salt thereof, or the stereoisomer thereof according to any one of claims 1-25, wherein the Ab is an anti-Her2 antibody, having a heavy chain as set forth in SEQ ID NO: 5 and a light chain as set forth in SEQ ID NO: 10.

27. The compound, the pharmaceutically acceptable salt thereof, or the stereoisomer thereof according to any one of claims 1-22, wherein the Ab is Trastuzumab, Pertuzumab, Sacituzumab, or zolbetuximab.

28. The compound, the pharmaceutically acceptable salt thereof, or the stereoisomer thereof according to any one of claims 1-27, wherein the L is -L₁-L₂-L₃-L₄-, wherein L₁ is connected to the Ab at one end, and L₄ is connected to the payload drug molecule at the other end; L₁ is the following group, and the end of L₁ marked with an asterisk * is connected to the Ab: 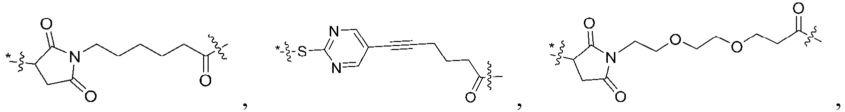 or 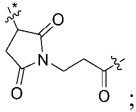 L₂ is a chemical bond, -N(R₁₀)-CH₂-CO-, or 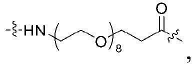 and the CO end of L₂ is connected to L₃, while the other end is connected to L₁; R₁₀ is C₁₋₄ alkyl or C₁₋₄ haloalkyl; L₃ is a polypeptide residue composed of 2-6 amino acids, and the C-terminus of the polypeptide residue is connected to L₄; and L₄ is a chemical bond, -NH-CH-, or 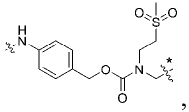 wherein the CH end is connected to the payload drug molecule, and the -NH- end is connected to L₃.

29. The compound, the pharmaceutically acceptable salt thereof, or the stereoisomer thereof according to claim 28, wherein L₂ is a chemical bond; or L₂ is -N(CH₃)-CH₂-CO-; or/and L₃ is the following polypeptide residue: GFG, GGFG, GGGFG, GGVA, V-Cit, 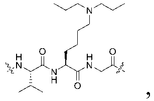 or VA; or L₁ is 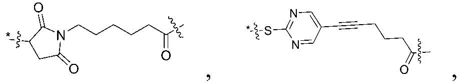 or 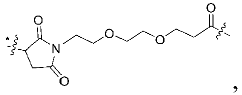 L₂ is a chemical bond or -N(CH₃)-CH₂-CO-, L₃ is GFG, GGFG, or GGGFG, and L₄ is -NH-CH-.

30. The compound, the pharmaceutically acceptable salt thereof, or the stereoisomer thereof according to any one of claims 1-27, wherein L is the following moiety: 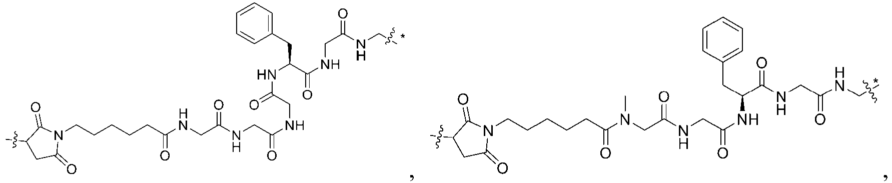 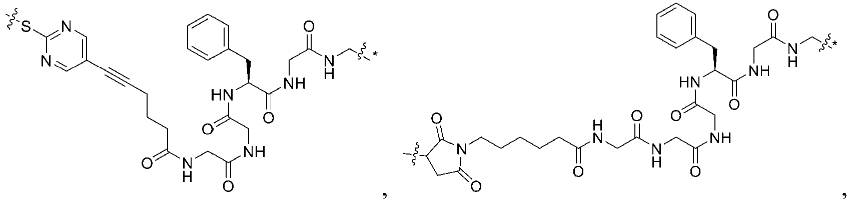 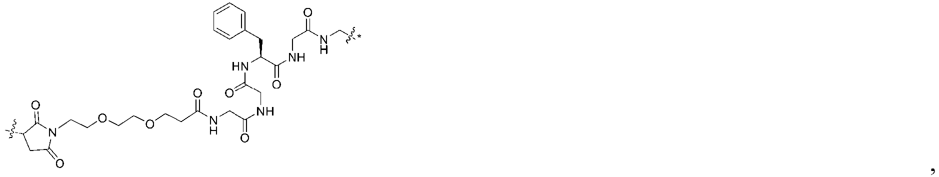 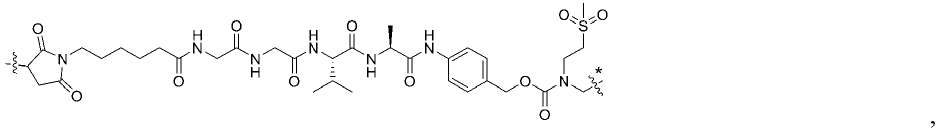 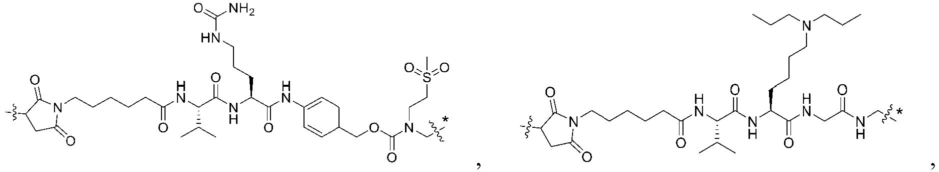 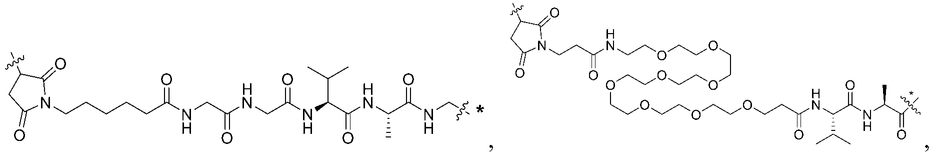 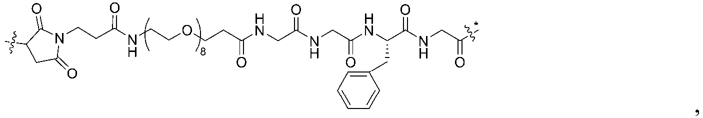 or 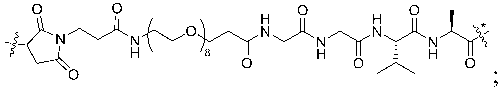 wherein "*" indicates the position where the linker connects to the payload drug molecule, and the other end indicates the position where the linker connects to the Ab.

31. The compound, the pharmaceutically acceptable salt thereof, or the stereoisomer thereof according to any one of claims 1 or 23-27, wherein 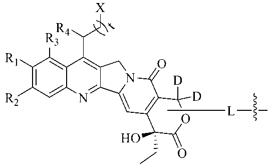 is the following structural moiety: 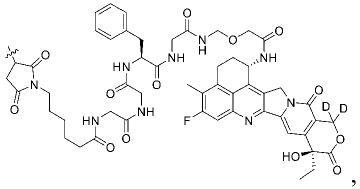 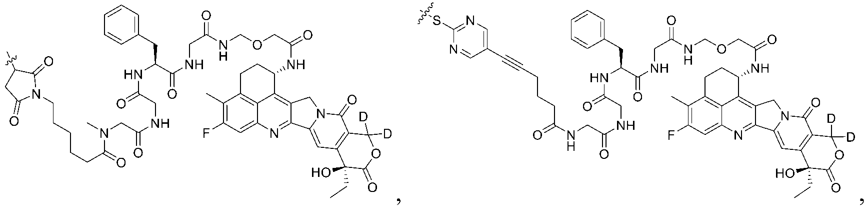 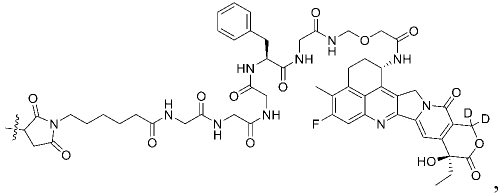 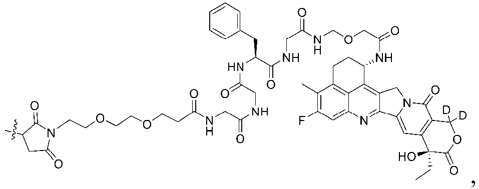 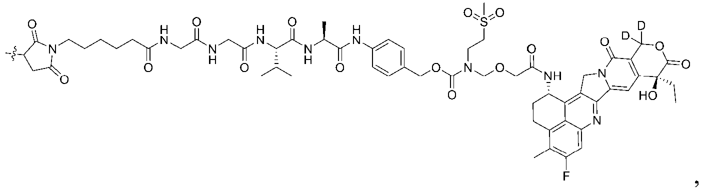 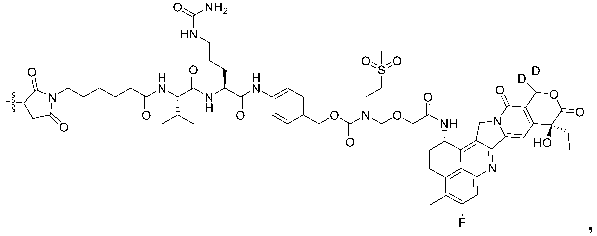 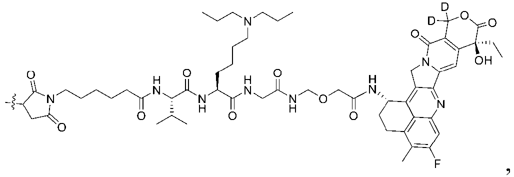 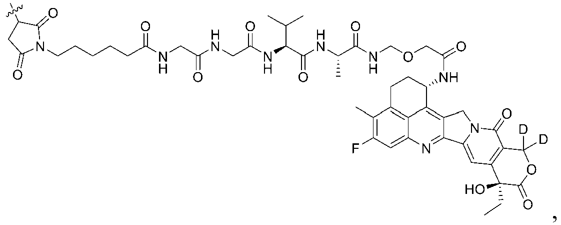 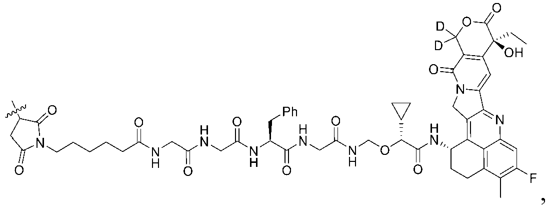 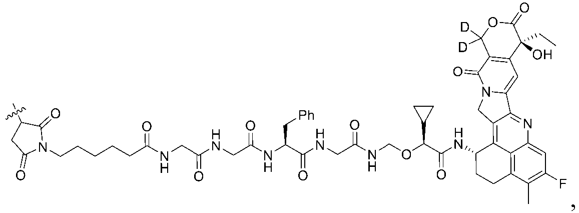 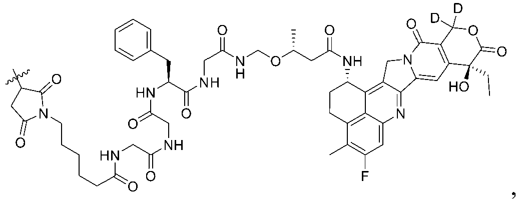 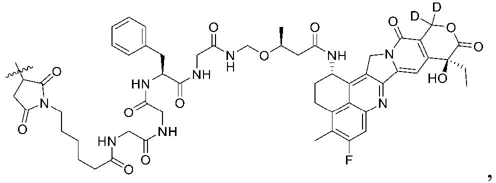 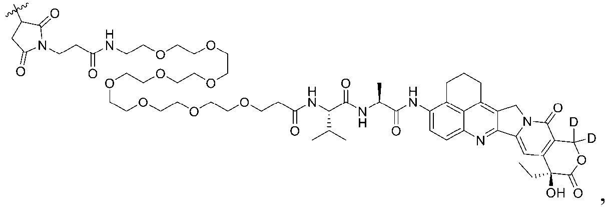 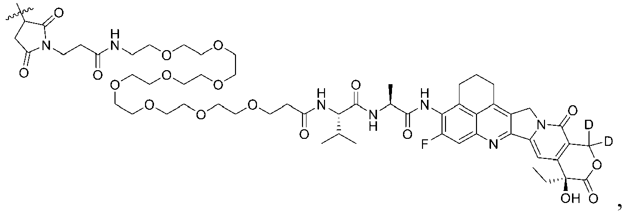 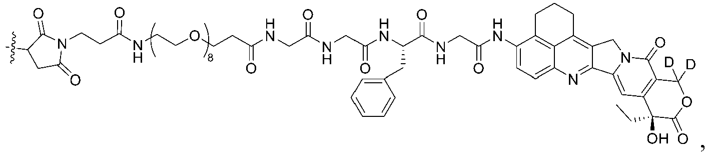 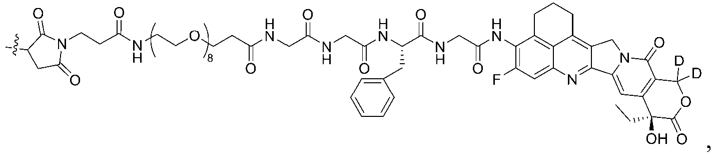 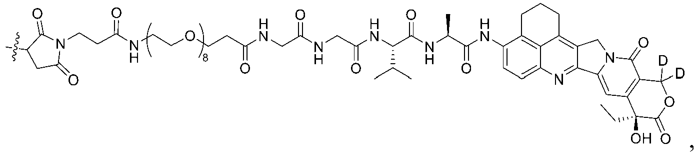 or

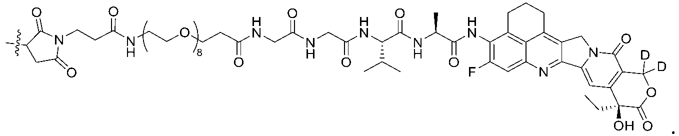

32. The compound, the pharmaceutically acceptable salt thereof, or the stereoisomer thereof according to any one of claims 1 or 23-27, wherein the compound, the pharmaceutically acceptable salt thereof, or the stereoisomer thereof is the following compound, a pharmaceutically acceptable salt thereof, or a stereoisomer thereof: 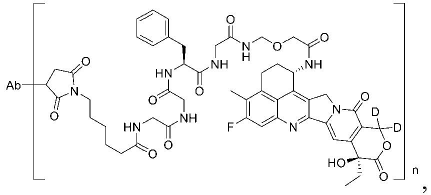 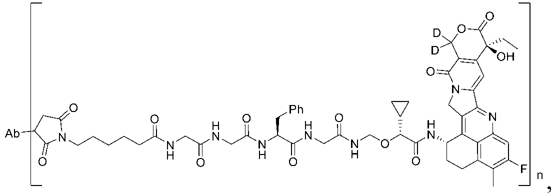 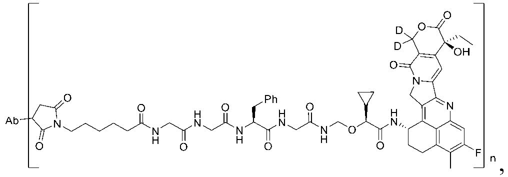 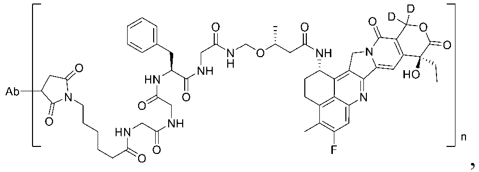 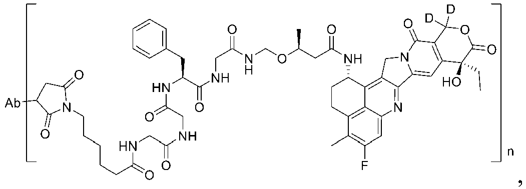 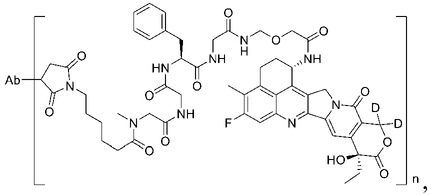 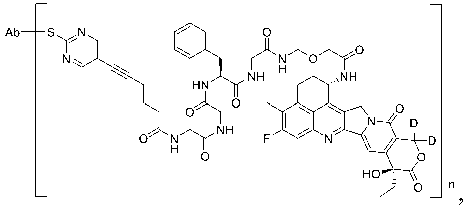 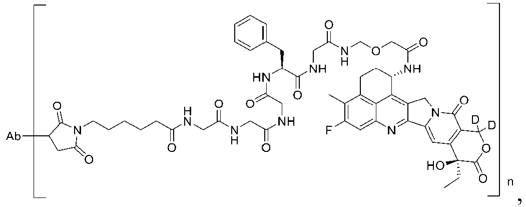 or 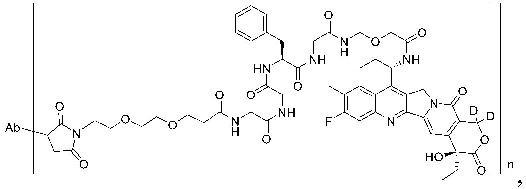 wherein n is 7.0-8.0, or n is 7.4-7.8, or n is 7.4-8.0, or n is 7.5-8.5.

33. A compound, wherein the compound is one of the following compounds, a pharmaceutically acceptable salt thereof, or a stereoisomer thereof: 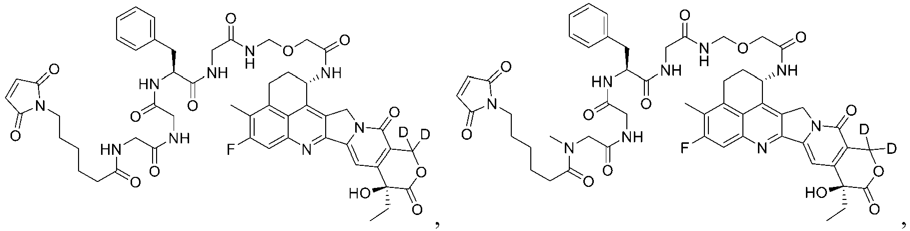 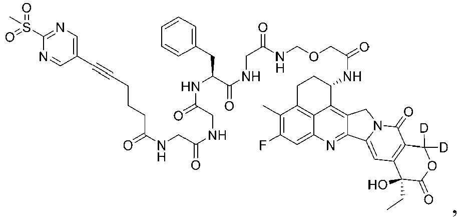 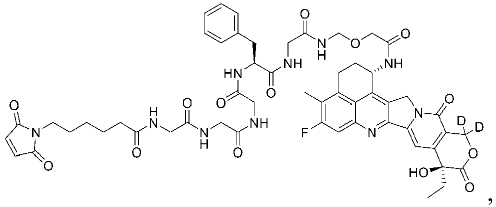 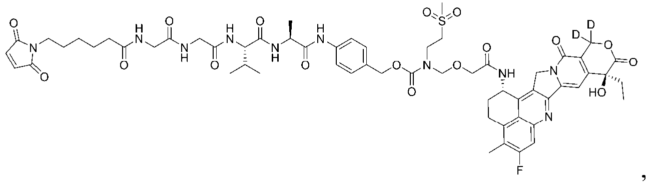 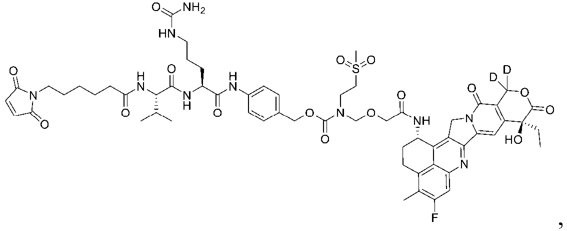 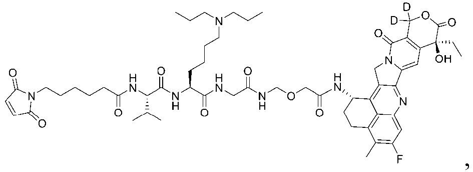 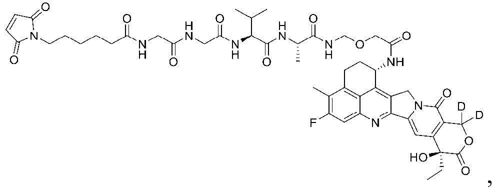 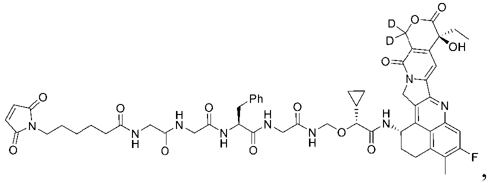 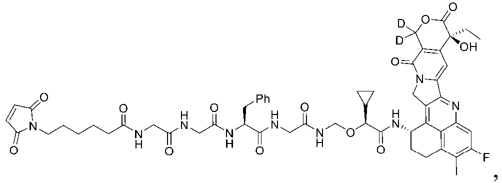 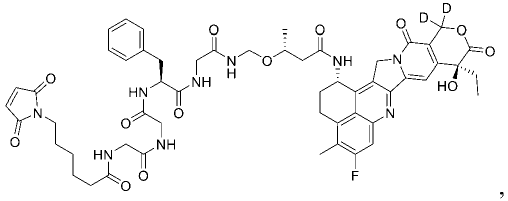 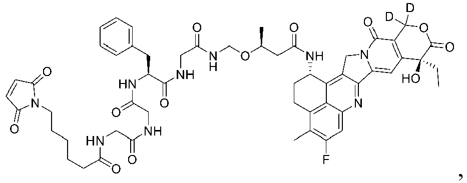 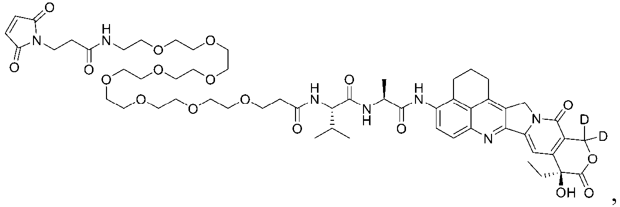 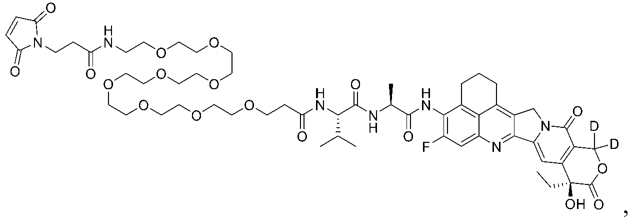 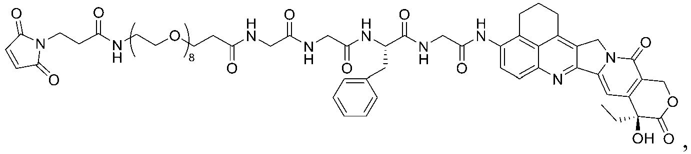 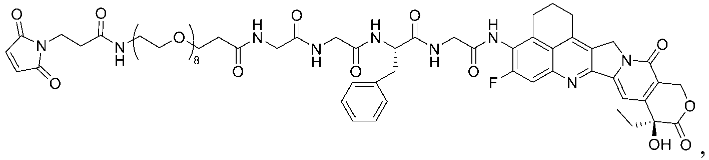 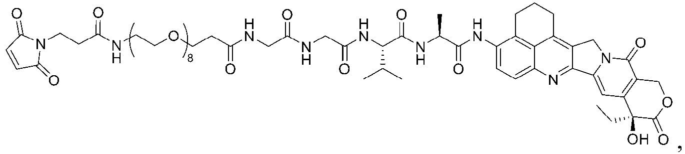 or

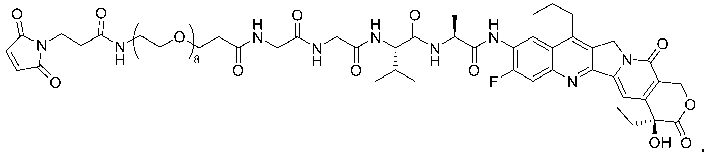

34. A structural moiety, wherein the structural moiety is: 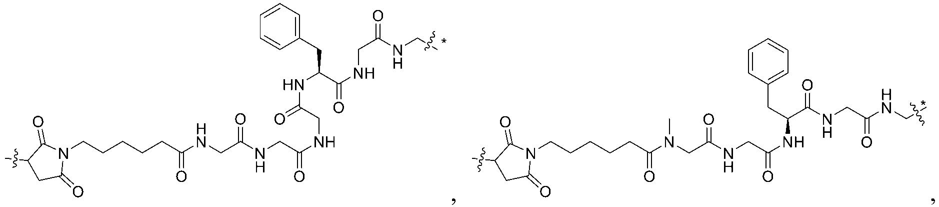 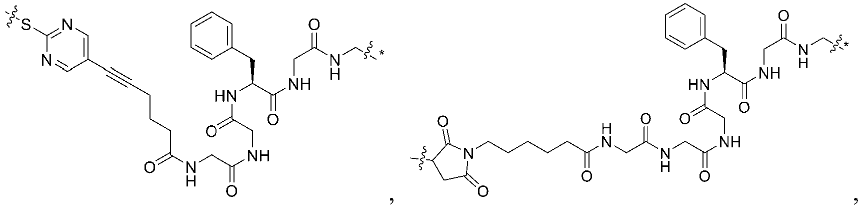 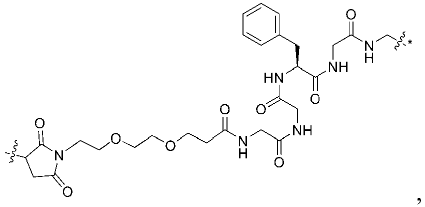 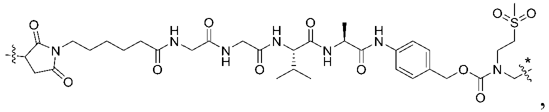 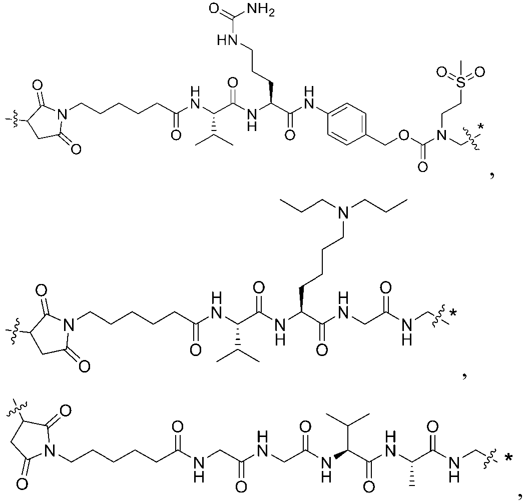 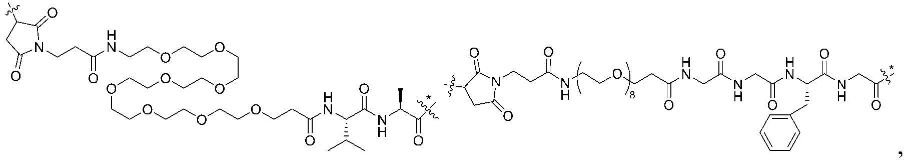 or 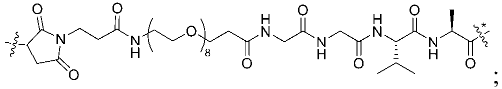 wherein the asterisk * end is connected to the payload drug molecule.

35. A pharmaceutical composition comprising the compound, the pharmaceutically acceptable salt thereof, or the stereoisomer thereof as defined in any one of claims 1-32, and a pharmaceutically acceptable excipient.

36. Use of the compound, the pharmaceutically acceptable salt thereof, or the stereoisomer thereof as defined in any one of claims 1-32, or the pharmaceutical composition as defined in claim 35, in the manufacture of a medicament for treating tumors.

37. A method of treating tumors, comprising administering to a patient in need thereof an effective amount of the compound, the pharmaceutically acceptable salt thereof, or the stereoisomer thereof as defined in any one of claims 1-32, or the pharmaceutical composition as defined in claim 35.

38. The compound, the pharmaceutically acceptable salt thereof, or the stereoisomer thereof as defined in any one of claims 1-32, or the pharmaceutical composition as defined in claim 35, for use in treating tumors.
